(19) **Europäisches Patentamt**
**European Patent Office**
**Office européen des brevets**

(11) **EP 4 538 385 A1**

(12) **EUROPEAN PATENT APPLICATION**
published in accordance with Art. 153(4) EPC

(43) Date of publication:
**16.04.2025 Bulletin 2025/16**

(21) Application number: **23849689.7**

(22) Date of filing: **23.03.2023**

(51) International Patent Classification (IPC):
**C12Q 1/04** (2006.01)      **G01N 21/45** (2006.01)
**C12M 1/34** (2006.01)

(52) Cooperative Patent Classification (CPC):
**C12M 1/34; C12Q 1/04; G01N 21/45**

(86) International application number:
**PCT/JP2023/011554**

(87) International publication number:
**WO 2024/029125 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.08.2022 JP 2022125335**

(71) Applicant: **Hamamatsu Photonics K.K.
Hamamatsu-shi, Shizuoka 435-8558 (JP)**

(72) Inventors:
- **TAKEUCHI, Kozo**
  **Hamamatsu-shi, Shizuoka 435-8558 (JP)**
- **YASUHIKO, Osamu**
  **Hamamatsu-shi, Shizuoka 435-8558 (JP)**

(74) Representative: **Hoffmann Eitle
Patent- und Rechtsanwälte PartmbB
Arabellastraße 30
81925 München (DE)**

(54) **METHOD, DEVICE, AND INFORMATION PROCESSING PROGRAM FOR DETERMINING NECROSIS CELL REGION**

(57) Disclosed are a method, device, and information processing program for determining a necrosis cell region in an object to be observed, using refractive-index distribution data pertaining to the object to be observed.

**EP 4 538 385 A1**

## Description

## Technical Field

The present disclosure relates to a method, an apparatus, and an information processing program for determining a region of cells that have undergone necrosis.

## Background Art

Among cells deaths, accidental necrotic death of cells is referred to as necrosis. Necrosis occurs when cells are exposed to external stimuli, for example, hypoxia, high temperature, poisons, nutrient deficiency, and cell membrane damage. Necrosis is typically a cell death that occurs in cells exposed to these stimuli in the living body, and even when cells are three-dimensionally cultured to create cell clusters, since a region with a high cell density inside a cell cluster is likely to be in a hypoxic condition and/or a nutrient-deficient condition, cells inside the cell cluster, particularly cells in the vicinity of the center, often undergo necrosis.

Generally, under conditions that enable observation of individual cells, it is relatively easy to judge dead cells from the appearance. For example, when two-dimensionally cultured adherent cells undergo cell death, the adherent cells lose adherence to the culture vessel and float. Furthermore, floating cells often undergo morphological changes when the cells undergo cell death. Therefore, dead cells can be judged by these changes in appearance.

On the other hand, in an environment in which cells are densely packed three-dimensionally as is the case of cell clusters, floating or morphological change of dead cells is less likely to occur, and it is not easy to observe in detail any changes in appearance that have occurred, at the single-cell level. Judgment of cell death inside a cell cluster is expected to be applied as a method for analyzing the effect of a drug on cell death and a method for evaluating a cell cluster itself or the like in the field of regenerative medicine, and particularly, it is desirable to develop a non-invasive method for determining a region of cell death.

With regard to non-invasive determination of a region of cell death, for example, Patent Literature 1 discloses a cell analysis method of analyzing the condition of cell death inside spheroids by correcting a plurality of spheroid cross-sectional images at different distances from the position of light irradiation, based on the three-dimensional structure of spheroids and the average luminance information for each pixel when the spheroids are irradiated with light, by taking into account the signal intensity attenuation based on the distance from the position of light irradiation to the position of measurement. Furthermore, Patent Literature 2 discloses a spheroid evaluation method of evaluating the degree of spheroid disintegration from an image capturing spheroids, based on the information on the contour, optical density, degree of circularity, and visibility of a spheroid region.

## Citation List

## Patent Literature

Patent Literature 1: Japanese Patent No. 6792616
Patent Literature 2: Japanese Patent No. 6122817

## Summary of Invention

## Technical Problem

In existing non-invasive methods for determining a region of cell death, it is difficult to determine a region of cell death with high resolution at the single cell level, let alone judge the type of a cell death that has occurred in a cell. Fluorescent staining is usually used to judge a region of cell death with such high resolution while specifying the type of cell death; however, this requires labeling, and observation of deep parts is difficult unless complicated treatments such as a transparentization treatment are applied.

Therefore, an object of the present invention is to provide a method for non-invasively determining a region of dead cells, particularly a region of cells that have undergone necrosis, in an observation objectobservation object.

## Solution to Problem

The inventors have found that when refractive index distribution data of an observation objectobservation object is used, it is possible to determine a region of cells that have undergone necrosis in an observation objectobservation object.

That is, an aspect of the present disclosure is a method for determining a region of cells that have undergone necrosis in an observation object, on the basis of refractive index distribution data of the observation object.

Another aspect of the present disclosure is a method for determining a region of cells that have undergone necrosis in an observation object, the method including: a step of acquiring refractive index distribution data of an observation object; and a step of determining a region of cells that have undergone necrosis in the observation object, on the basis of the refractive index distribution data of the observation object.

Another aspect of the present disclosure is a method for evaluating quality of a cell cluster, the method including: a step of acquiring refractive index distribution data of a cell cluster; and a step of determining a region of cells that have undergone necrosis inside the cell cluster, on the basis of refractive index distribution data of the cell cluster.

Another aspect of the present disclosure is an apparatus for determining a region of cells that have undergone necrosis, the apparatus including: a data acquisition unit for acquiring refractive index distribution data of an observation object; and a determination unit for determining a region of cells that have undergone necrosis in the observation object, on the basis of the refractive index distribution data of the observation object.

Another aspect of the present disclosure is an information processing program for causing a computer to execute a determination step of determining a region of cells that have undergone necrosis in an observation object, on the basis of refractive index distribution data of the observation object.

Another aspect of the present disclosure is an information processing program for causing a computer to execute: a data acquisition step of acquiring refractive index distribution data of an observation object; and a determination step of determining a region of cells that have undergone necrosis in the observation object, on the basis of the refractive index distribution data of the observation object.

More specifically, the present disclosure relates to the following [1] to [21].

[1] A method for determining a region of cells that have undergone necrosis in an observation object, on a basis of refractive index distribution data of the observation object.

[2] The method according to [1], wherein determination of the region of cells that have undergone necrosis is carried out based on a fact that a region of each cell included in the refractive index distribution data has features of necrotic cells, and the features of necrotic cells include features of having a region equivalent to a nucleus.

[3] The method according to [2], wherein the features of the necrotic cell further include one or more features selected from the group consisting of a feature in which a statistic value of refractive index of a whole cell is equal to or less than a threshold value, and a feature in which statistical dispersion of refractive index of a nucleus is equal to or greater than the threshold value.

[4] The method according to [2], wherein the features of the necrotic cell further include a feature in which a statistic value of refractive index of a whole cell is equal to or less than a threshold value, and a feature in which statistical dispersion of refractive index of a nucleus is equal to or greater than the threshold value.

[5] The method according to any one of [2] to [4], wherein the region equivalent to the nucleus is an approximately circular or approximately spherical region, and is a region in which a statistic value of refractive index is greater than a statistic value of refractive index of a whole cell.

[6] The method according to [1], wherein the determination of the region of cells that have undergone necrosis is carried out by inputting the refractive index distribution data of the observation object into a learning model that has learned using training data including necrotic region data of a reference observation object and refractive index distribution data of the reference observation object corresponding to the necrotic region data.

[7] The method according to [1], wherein the determination of the region of cells that have undergone necrosis is carried out by inputting refractive index distribution data of an observation object into a learning model that has learned using training data including necrotic region data of a reference observation object and refractive index distribution data of the reference observation object corresponding to the necrotic region data, and a feature quantity utilized by the learning model includes a feature quantity corresponding to features of having a region equivalent to a nucleus.

[8] The method according to [7], wherein the feature quantity utilized by the learning model further includes one or more feature quantities corresponding to one or more features selected from the group consisting of a feature in which a statistic value of refractive index of a whole cell is equal to or less than a threshold value, and a feature in which statistical dispersion of refractive index of a nucleus is equal to or greater than a threshold value.

[9] The method according to [7], wherein the feature quantity utilized by the learning model further includes one or more feature quantities corresponding to a feature in which a statistic value of refractive index of a whole cell is equal to or less than a threshold value, and a feature in which statistical dispersion of refractive index of a nucleus is equal to or greater than a threshold value.

[10] The method according to any one of [3] to [9], wherein the statistic value is an average value or a median value.

[11] The method according to any one of [1] to [10], wherein the refractive index distribution data is refractive index tomography data in a predetermined direction.

[12] A method for determining a region of cells that have undergone necrosis in an observation object, the method including:

a step of acquiring refractive index distribution data of an observation object; and
a step of determining a region of cells that have undergone necrosis by the method according to any one of [1] to [11].

[13] A method for evaluating quality of a cell cluster,

the observation object being a cell cluster,
the method including:

a step of acquiring refractive index distribution data of a cell cluster; and
a step of determining a region of cells that have undergone necrosis inside the cell cluster by the method according to any one of [1] to [11].

[14] The method according to [13], wherein the cell cluster is a cell cluster obtained by culturing stem cells collected from an animal including a human.

[15] The method according to [13], wherein the cell cluster is a cell cluster obtained by culturing stem cells collected from a human.

[16] An apparatus for determining a region of cells that have undergone necrosis, the apparatus including:

a data acquisition unit for acquiring refractive index distribution data of an observation object; and
a determination unit for determining a region of cells that have undergone necrosis in the observation object, by the method according to any one of [1] to [11].

[17] An information processing program for causing a computer to execute a determination step of determining a region of cells that have undergone necrosis in an observation object by the method according to any one of [1] to [11].

[18] An information processing program for causing a computer to execute:

a data acquisition step of acquiring refractive index distribution data of an observation object; and
a determination step of determining a region of cells that have undergone necrosis in the observation object, by the method according to any one of [1] to [11].

[19] The method, apparatus, or information processing program according to any one of [1] to [12] and [16] to [18], wherein the observation object is a cell cluster.

[20] The method, apparatus, or information processing program according to any one of [1] to [12] and [16] to [18], wherein the observation object is a cell cluster, and the region of cells that have undergone necrosis exists inside the cell cluster.

[21] The method, apparatus, or information processing program according to any one of [1] to [20], wherein the necrosis is necroptosis.

## Advantageous Effects of Invention

According to the present disclosure, a method, an apparatus, and an information processing program for non-invasively determining a region of cells that have undergone necrosis in particular, among regions of dead cells in an observation object, can be provided.

## Brief Description of Drawings

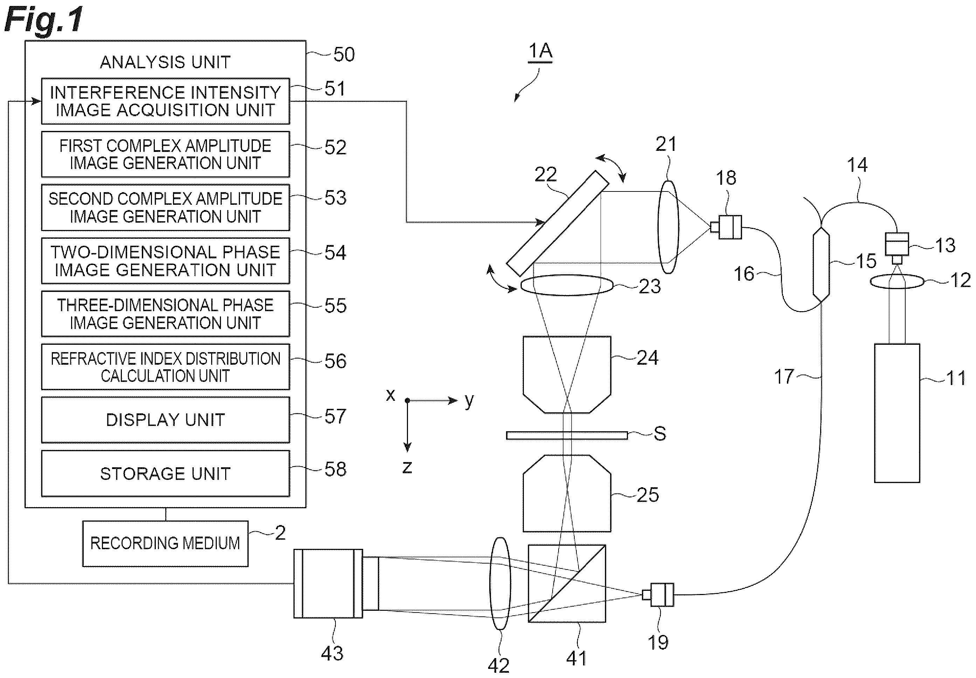

[FIG. 1] FIG. 1 (hereinafter also referred to as "FIG. A01") is a diagram illustrating a configuration of an observation apparatus 1A.

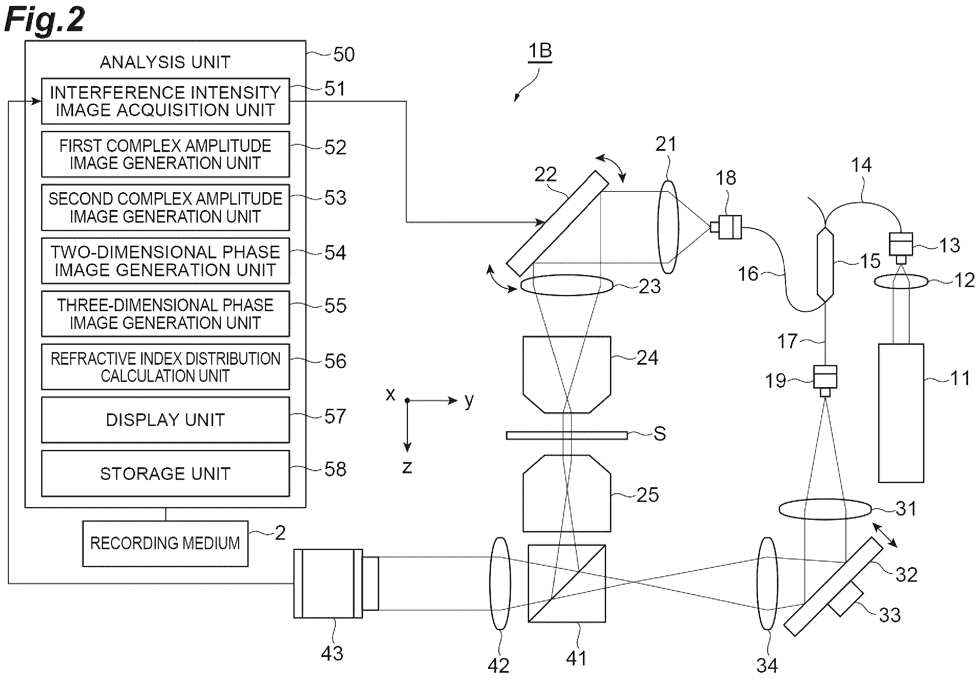

[FIG. 2] FIG. 2 (hereinafter also referred to as "FIG. A02") is a diagram illustrating a configuration of an observation apparatus 1B.

[FIG. 3] FIG. 3 (hereinafter also referred to as "FIG. A03") is a diagram illustrating a configuration of an observation apparatus 1C.

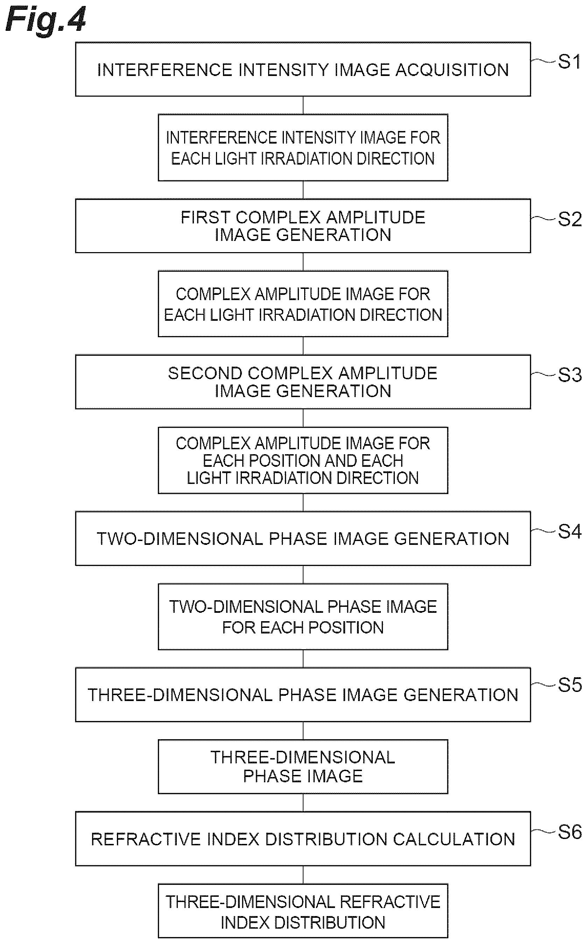

[FIG. 4] FIG. 4 (hereinafter also referred to as "FIG. A04") is a flowchart of a refractive index distribution measuring method A.

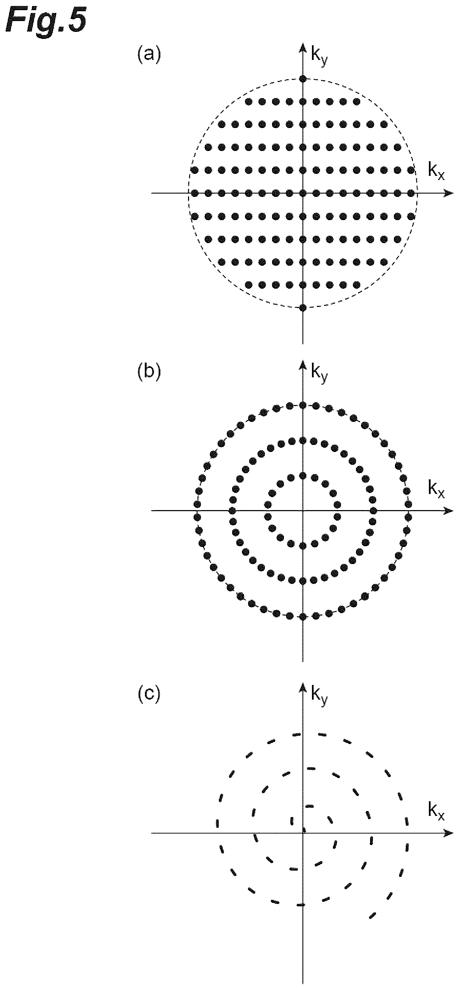

[FIG. 5] FIG. 5 (hereinafter also referred to as "FIG. A05") includes (a) - (c) diagrams illustrating examples of scanning of a light irradiation direction on an observation object S in an interference intensity image acquisition step S1.

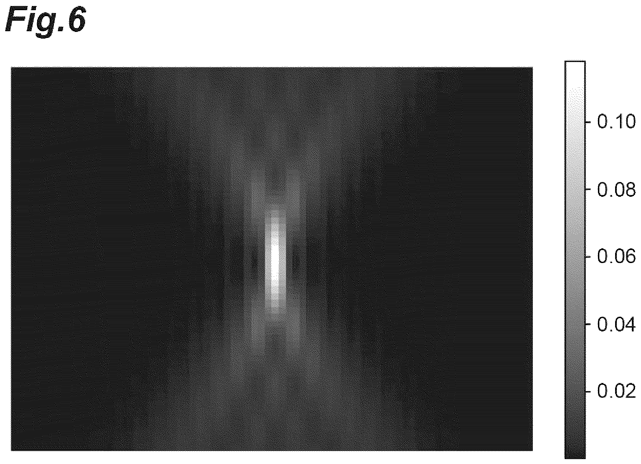

[FIG. 6] FIG. 6 (hereinafter also referred to as "FIG. A06") is a diagram showing a kernel function g.

[FIG. 7] FIG. 7 (hereinafter also referred to as "FIG. A07") includes (a) - (b) diagrams illustrating examples of scanning of a light irradiation direction on an observation object S in an interference intensity image acquisition step S1.

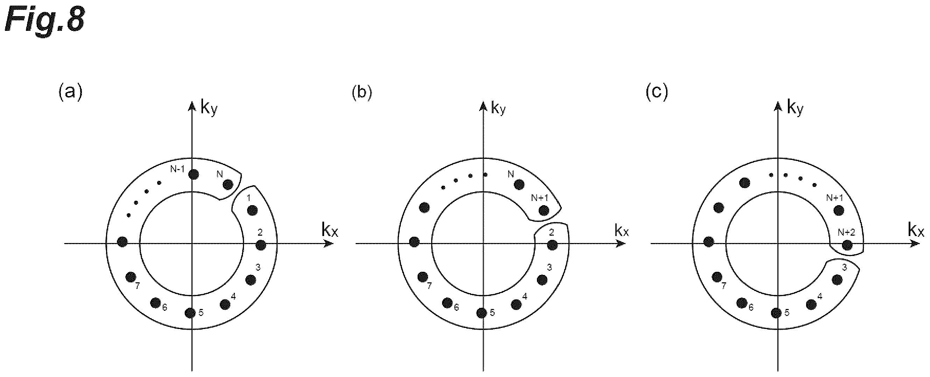

[FIG. 8] FIG. 8 (hereinafter also referred to as "FIG. A08") includes (a) - (c) diagrams illustrating examples of scanning of a light irradiation direction on an observation object S in an interference intensity image acquisition step S1.

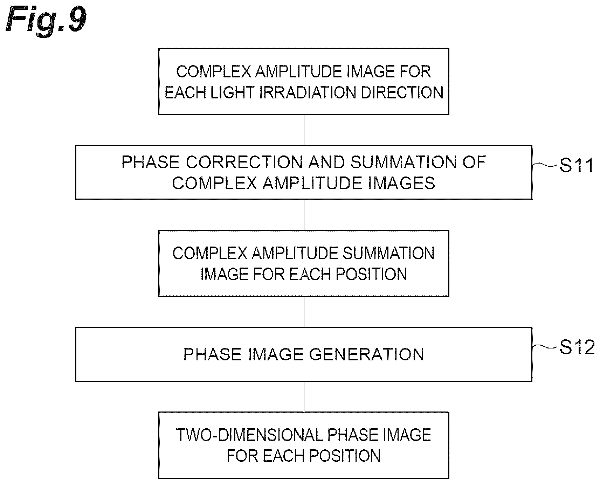

[FIG. 9] FIG. 9 (hereinafter also referred to as "FIG. A09") is a flowchart of a two-dimensional phase image generation step S4 in a refractive index distribution measuring method A1.

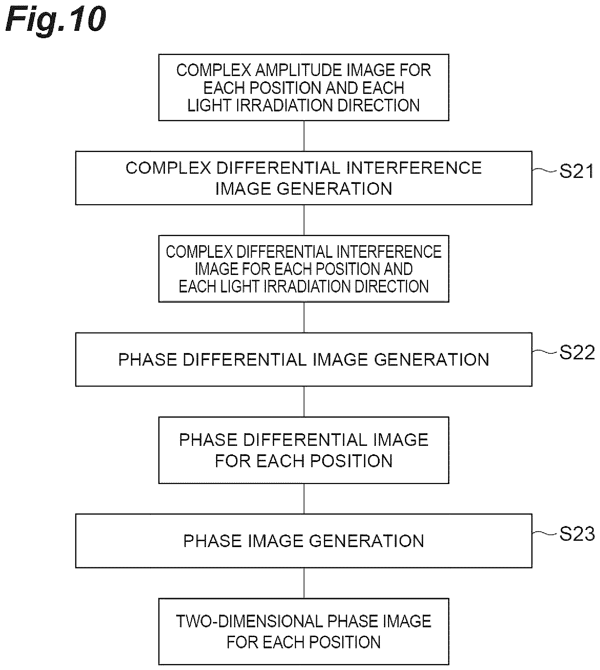

[FIG. 10] FIG. 10 (hereinafter also referred to as "FIG. A10") is a flowchart of a two-dimensional phase image generation step S4 in a refractive index distribution measuring method A2.

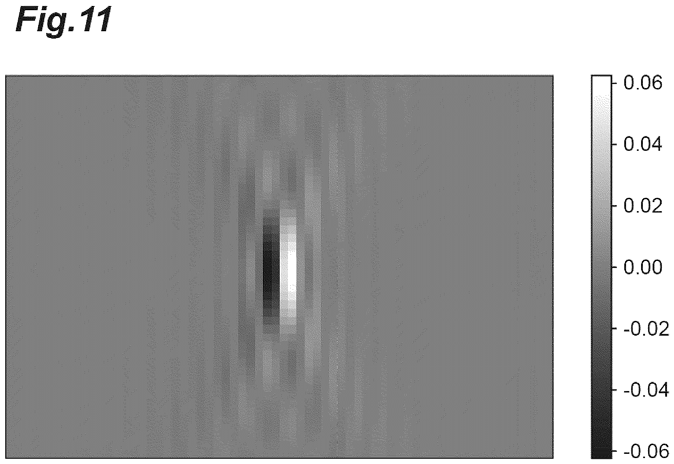

[FIG. 11] FIG. 11 (hereinafter also referred to as "FIG. A11") is a diagram showing the kernel function.

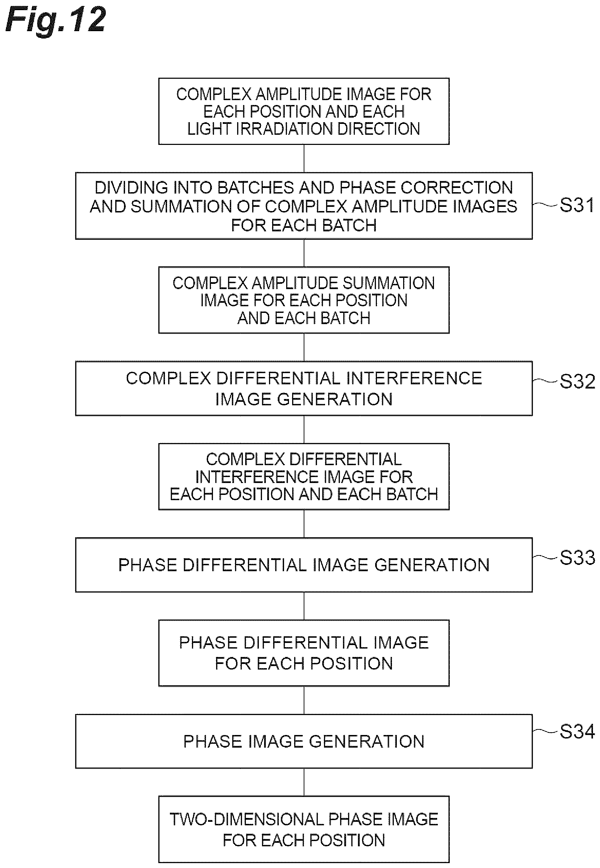

[FIG. 12] FIG. 12 (hereinafter also referred to as "FIG. A12") is a flowchart of a two-dimensional phase image generation step S4 in a refractive index distribution measuring method A3.

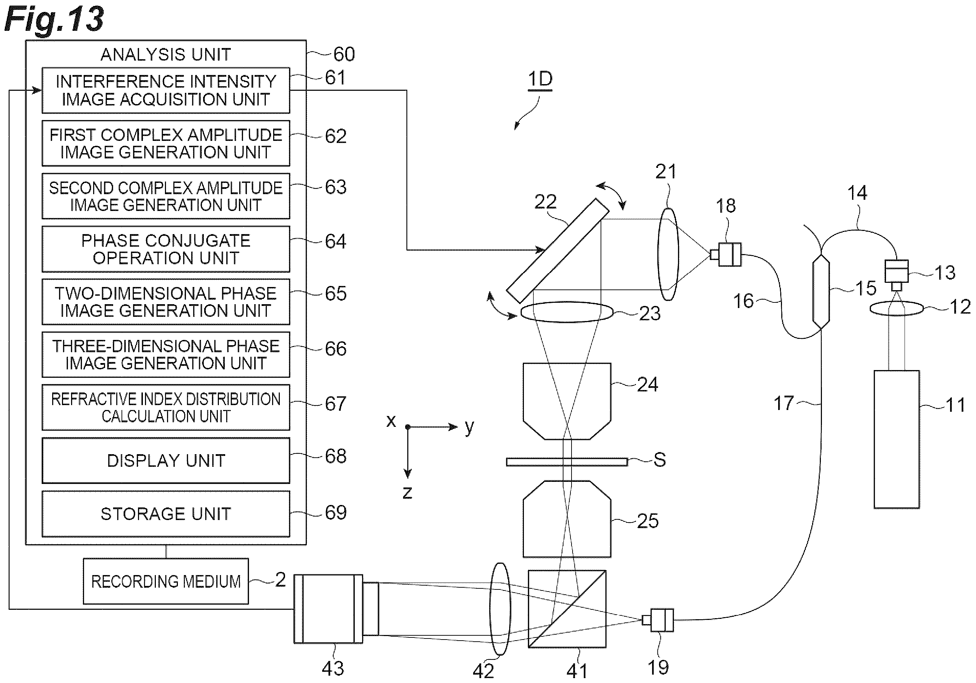

[FIG. 13] FIG. 13 (hereinafter also referred to as "FIG. B01") is a diagram illustrating a configuration of an observation apparatus 1D.

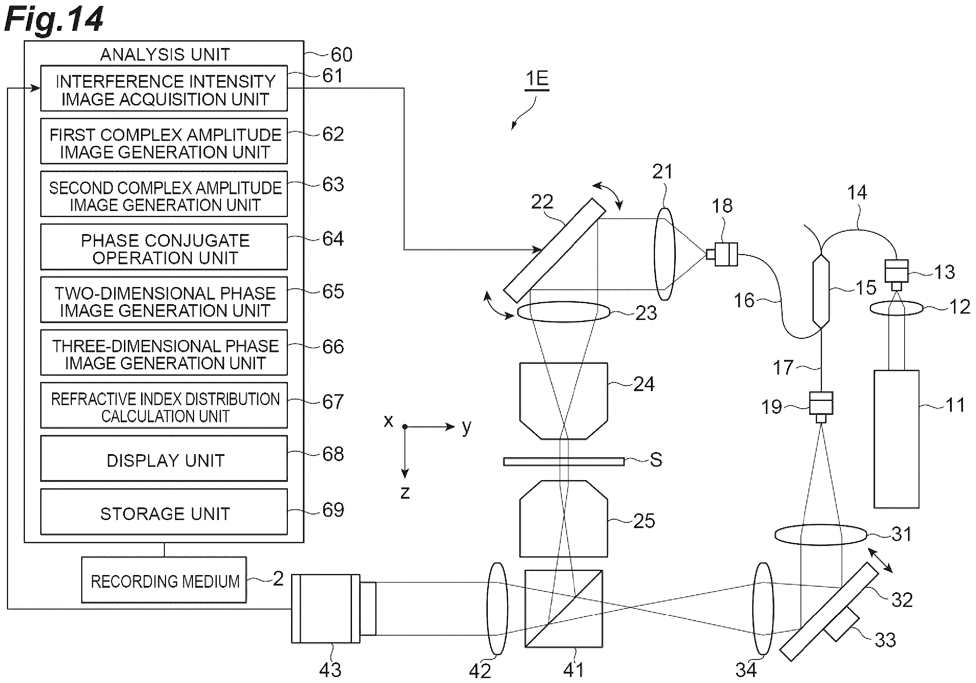

[FIG. 14] FIG. 14 (hereinafter also referred to as "FIG. B02") is a diagram illustrating a configuration of an observation apparatus 1E.

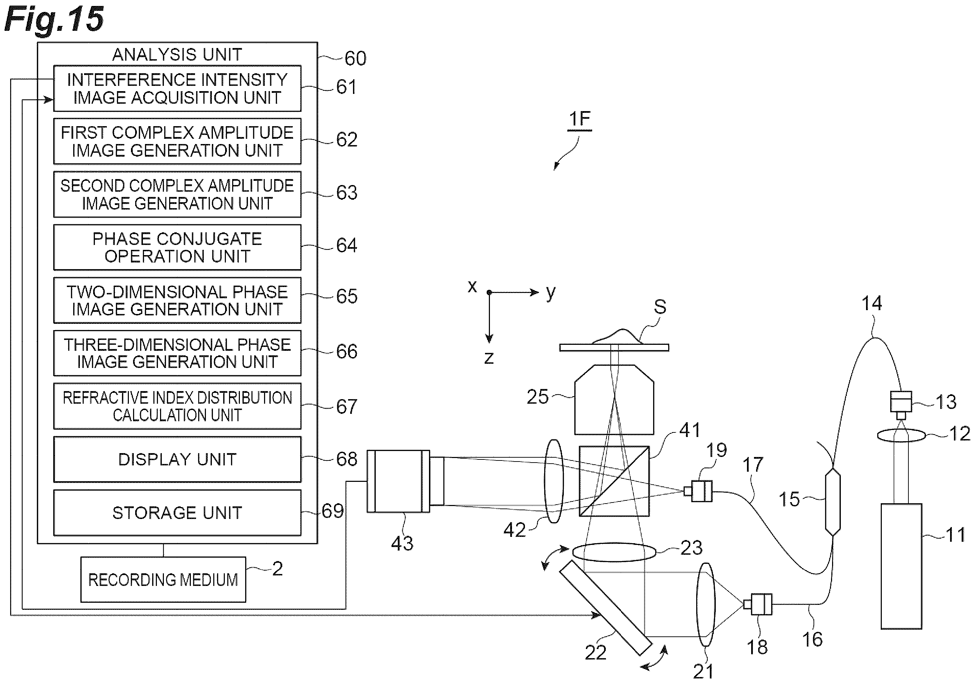

[FIG. 15] FIG. 15 (hereinafter also referred to as "FIG. B03") is a diagram illustrating a configuration of an observation apparatus 1F.

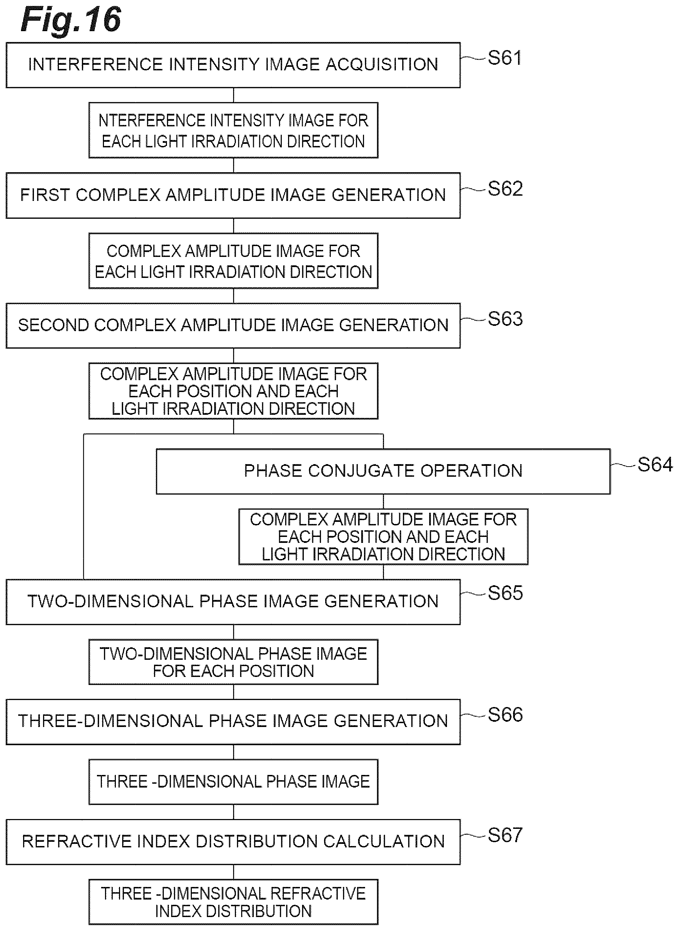

[FIG. 16] FIG. 16 (hereinafter also referred to as "FIG. B04") is a flowchart of a refractive index distribution measuring method B.

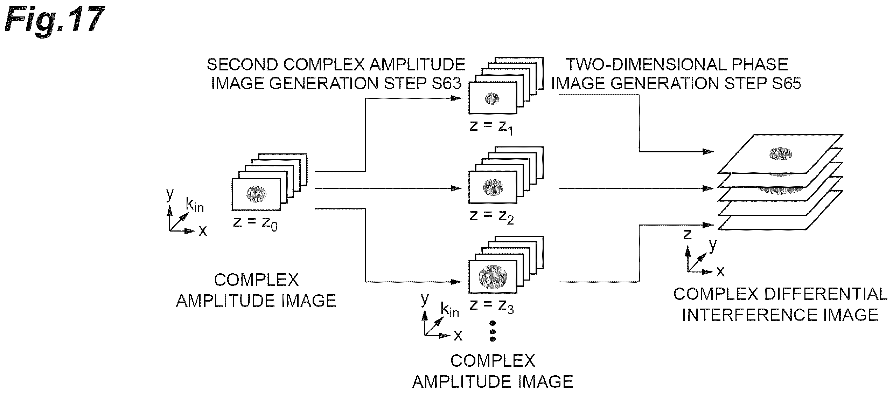

[FIG. 17] FIG. 17 (hereinafter also referred to as "FIG. B05") is a diagram illustrating images and an order of processing steps of a second complex amplitude image generation step S63 and a two-dimensional phase image generation step S65.

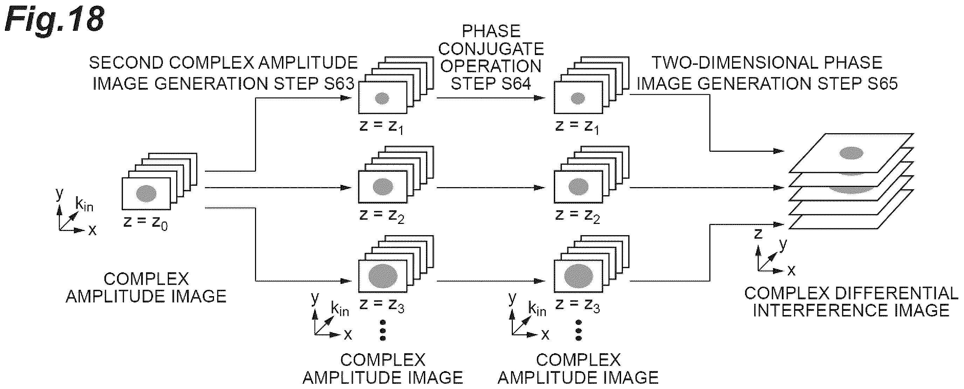

[FIG. 18] FIG. 18 (hereinafter also referred to as "FIG. B06") is a diagram illustrating images and an order of processing steps of the second complex amplitude image generation step S63, a phase conjugate operation step S64, and the two-dimensional phase image generation step S65.

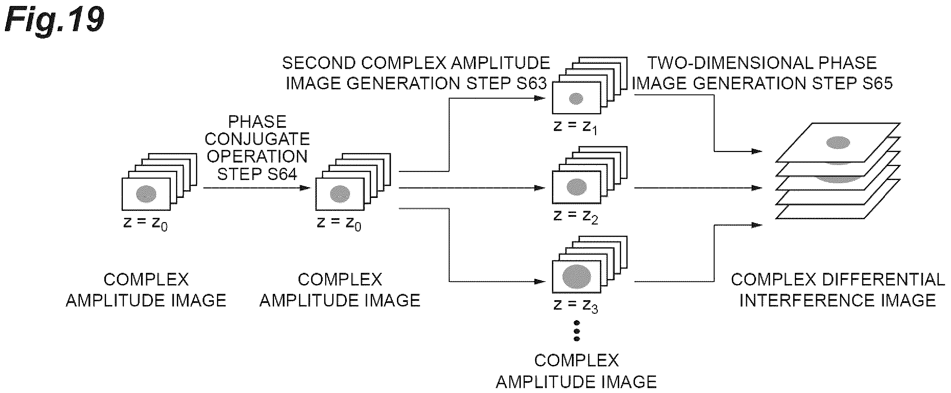

[FIG. 19] FIG. 19 (hereinafter also referred to as "FIG. B07") is a diagram illustrating images and an order of processing steps of the second complex amplitude image generation step S63, the phase conjugate operation step S64, and the two-dimensional phase image generation step S65.

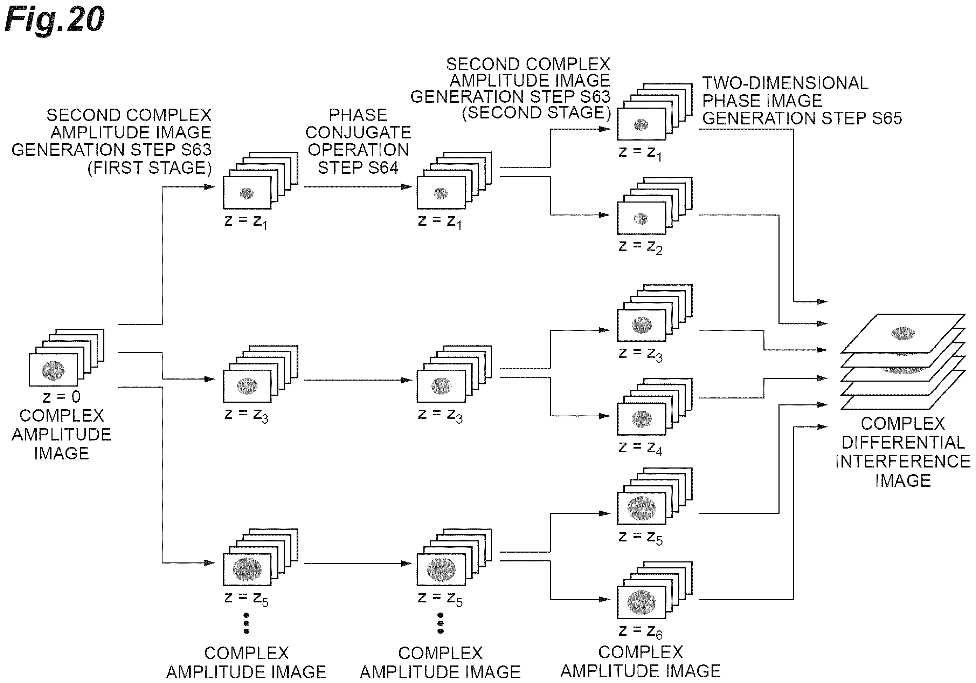

[FIG. 20] FIG. 20 (hereinafter also referred to as "FIG. B08") is a diagram illustrating images and an order of processing steps of the second complex amplitude image generation step S63, the phase conjugate operation step S64, and the two-dimensional phase image generation step S65.

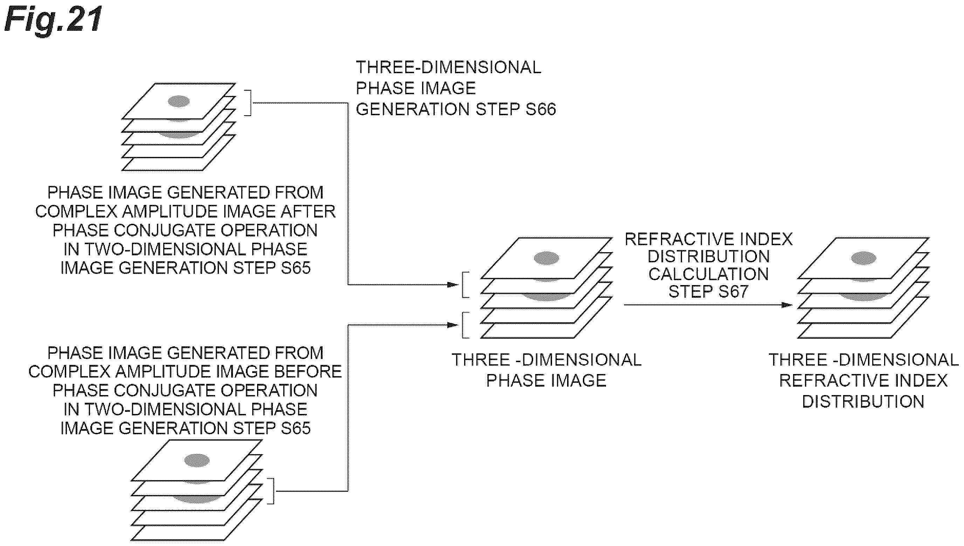

[FIG. 21] FIG. 21 (hereinafter also referred to as "FIG. B09") is a diagram illustrating images and an order of processing steps of a three-dimensional phase image generation step S66 and a refractive index distribution calculation step S67.

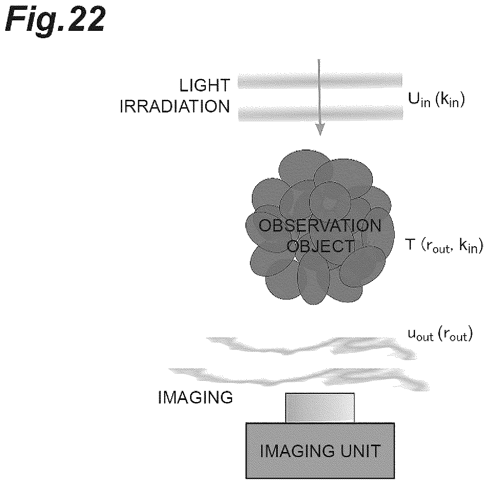

[FIG. 22] FIG. 22 (hereinafter also referred to as "FIG. B10") is a diagram for describing an outline of a phase conjugate operation, and is the diagram illustrating input light and output light when an interference intensity image is imaged by an imaging unit.

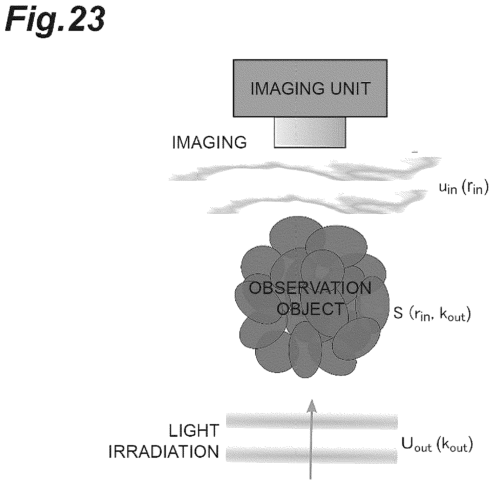

[FIG. 23] FIG. 23 (hereinafter also referred to as "FIG. B11") is a diagram for describing the outline of the phase conjugate operation, and is the diagram illustrating input light and output light in a case in which a relationship between light irradiation and imaging is reversed.

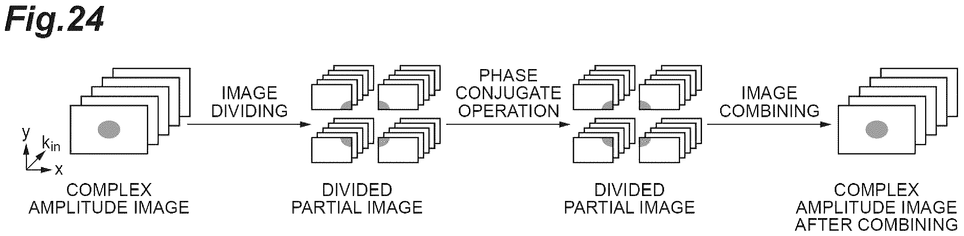

[FIG. 24] FIG. 24 (hereinafter also referred to as "FIG. B12") is a diagram illustrating image dividing, the phase conjugate operation, and image combining in the phase conjugate operation step S64.

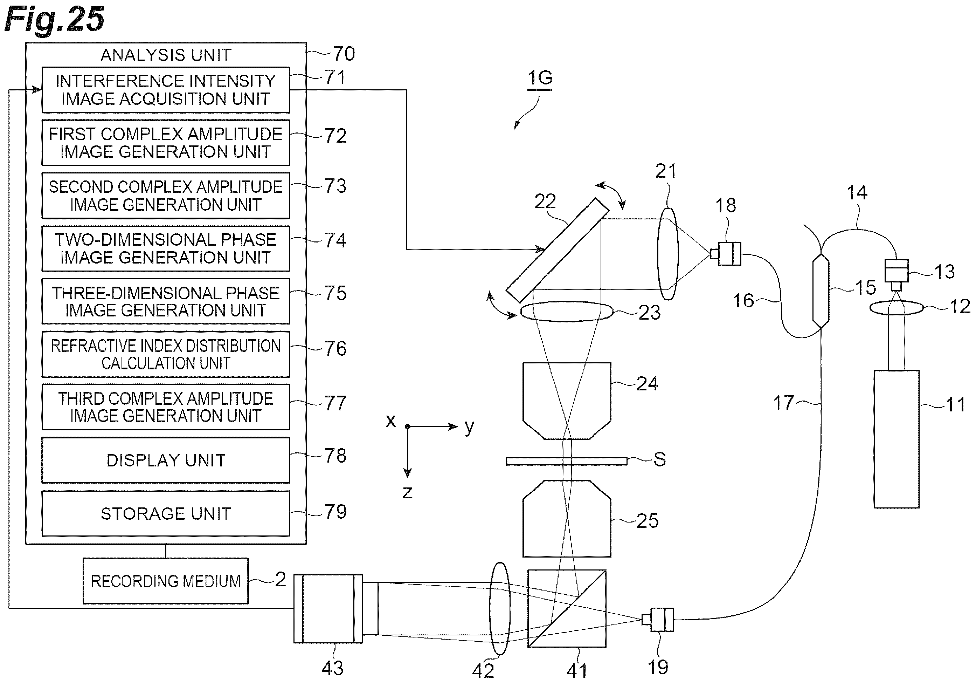

[FIG. 25] FIG. 25 (hereinafter also referred to as "FIG. C01") is a diagram illustrating a configuration of an observation apparatus 1G.

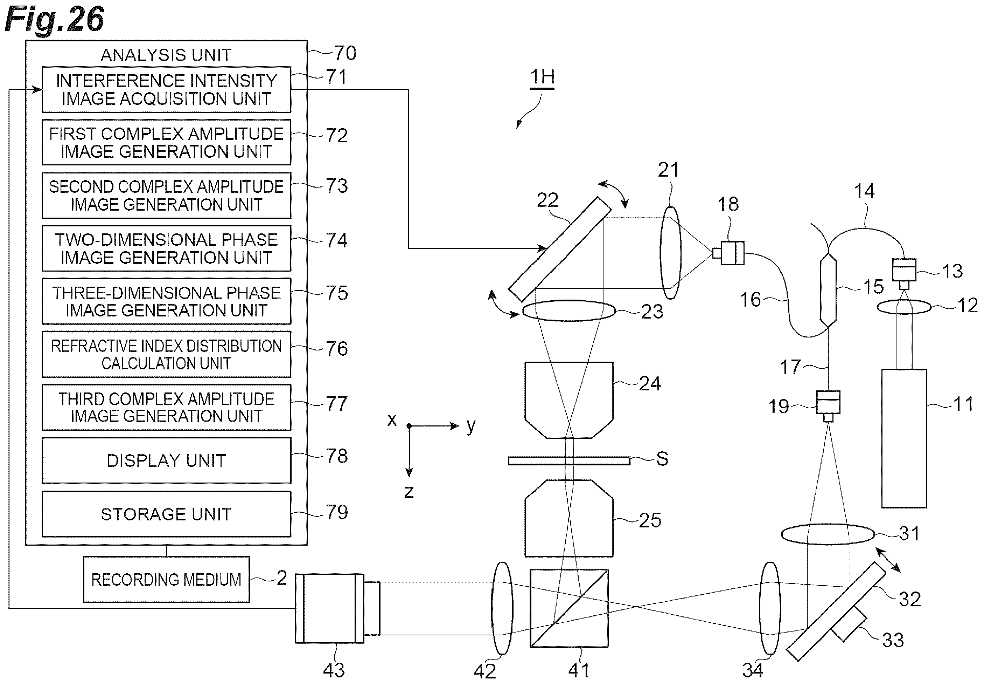

[FIG. 26] FIG. 26 (hereinafter also referred to as "FIG. C02") is a diagram illustrating a configuration of an observation apparatus 1H.

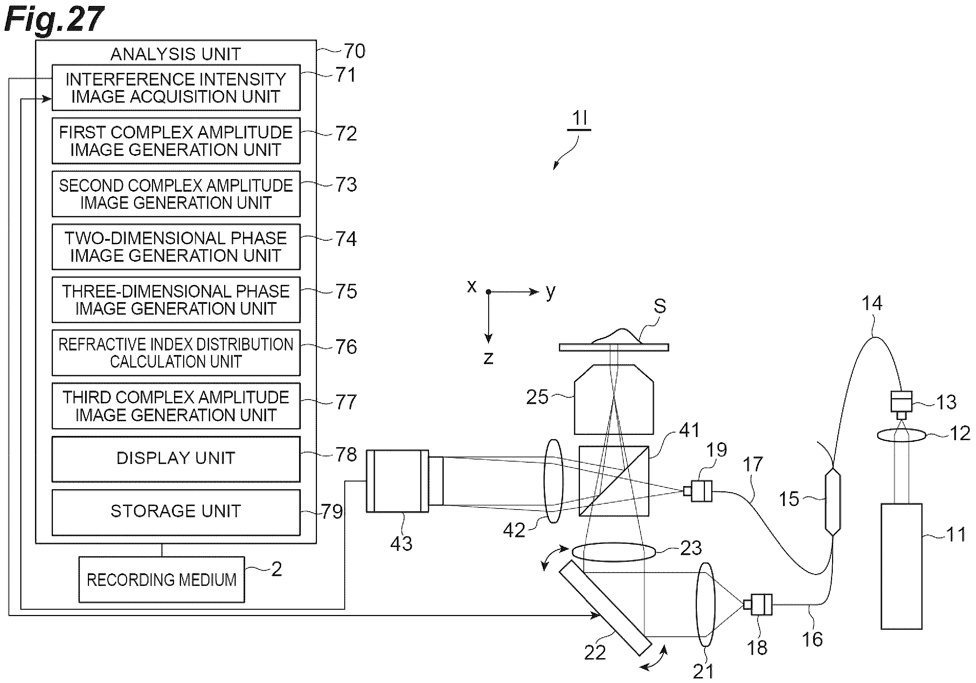

[FIG. 27] FIG. 27 (hereinafter also referred to as "FIG. C03") is a diagram illustrating a configuration of an observation apparatus 1I.

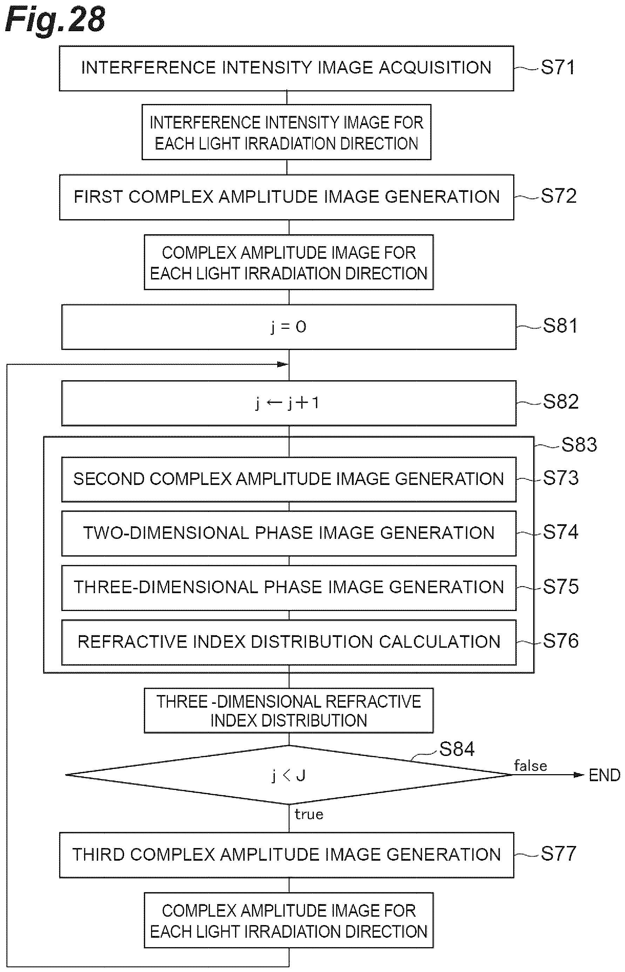

[FIG. 28] FIG. 28 (hereinafter also referred to as "FIG. C04") is a flowchart of a refractive index distribution measuring method C.

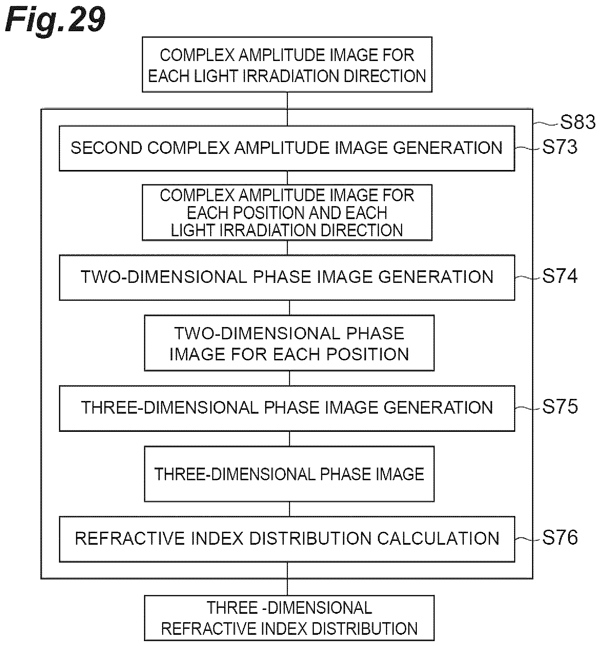

[FIG. 29] FIG. 29 (hereinafter also referred to as "FIG. C05") is a flowchart of a refractive index distribution measuring method C.

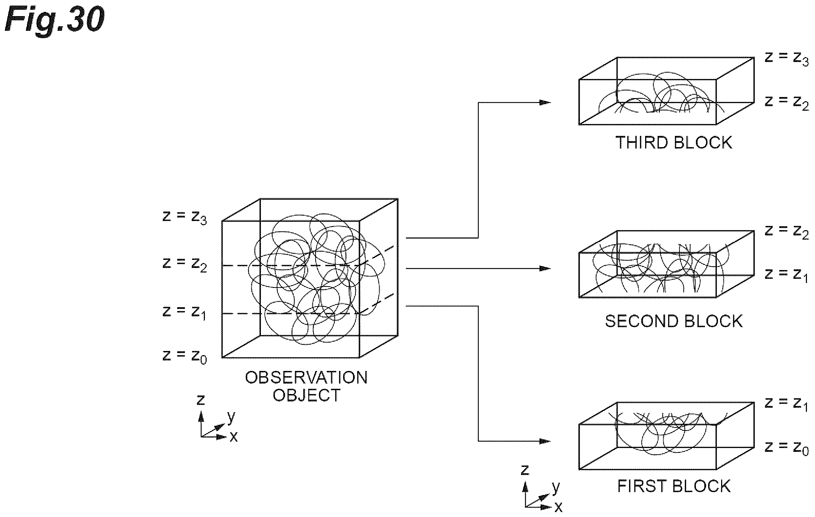

[FIG. 30] FIG. 30 (hereinafter also referred to as "FIG. C06") is a diagram illustrating a relationship between a region including an observation object and first to J-th blocks.

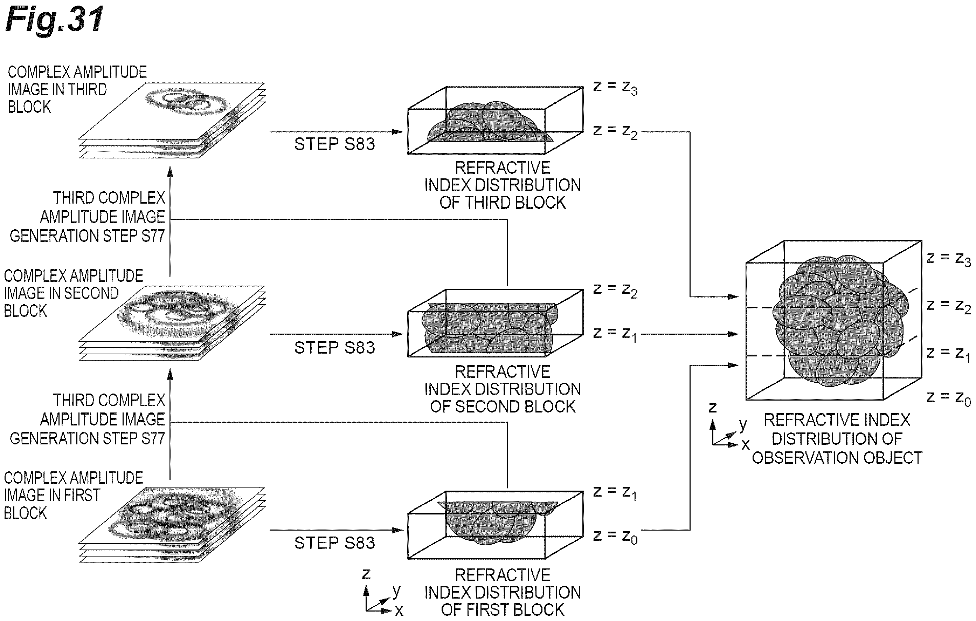

[FIG. 31] FIG. 31 (hereinafter also referred to as "FIG. C07") is a diagram illustrating a processing procedure for the first to J-th blocks.

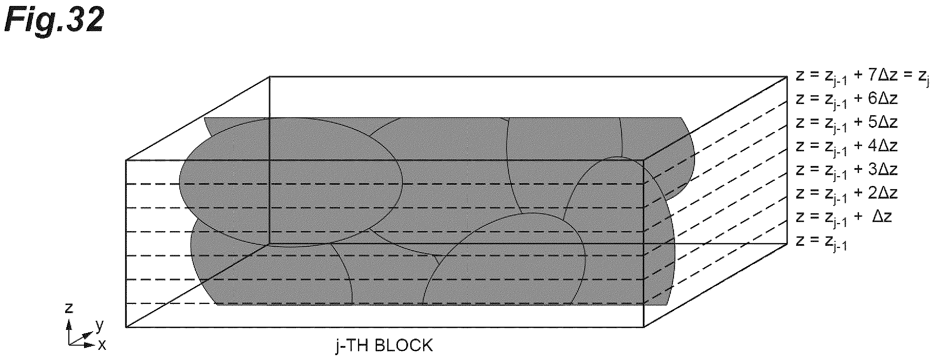

[FIG. 32] FIG. 32 (hereinafter also referred to as "FIG. C08") is a diagram illustrating processing contents of a BPM.

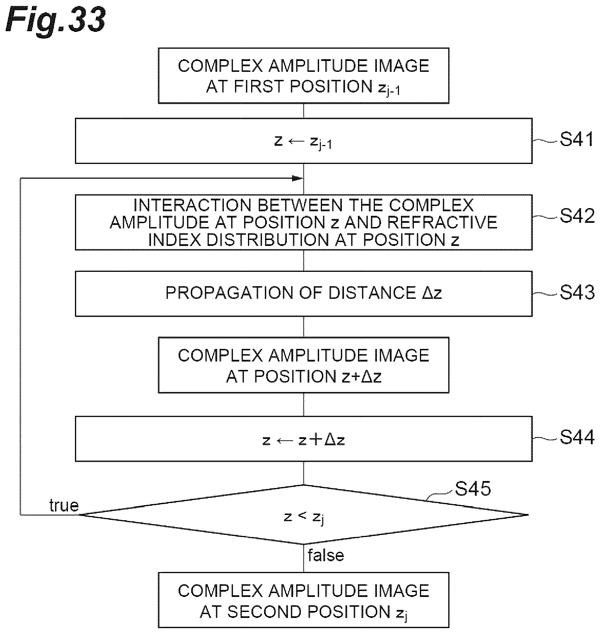

[FIG. 33] FIG. 33 (hereinafter also referred to as "FIG. C09") is a flowchart of a third complex amplitude image generation step S77.

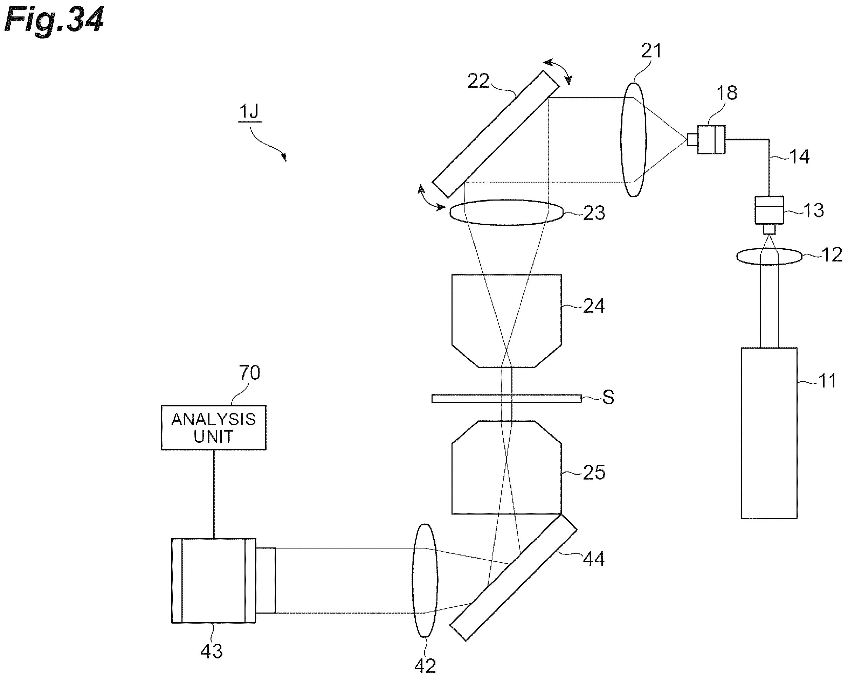

[FIG. 34] FIG. 34 (hereinafter also referred to as " FIG. C10") is a diagram illustrating a configuration of an observation apparatus 1J.

[FIG. 35] FIG. 35 is a diagram concerning a method for determining a region of cells that have undergone necrosis according to an aspect of the present disclosure, showing a typical flow in a case where the region is determined using a threshold method on the basis of having a feature in which an average value or a median value of the refractive index of a whole cell is equal to or less than a threshold value.

[FIG. 36] FIG. 36 is a diagram concerning the method for determining a region of cells that have undergone necrosis according to an aspect of the present disclosure, showing a typical flow in a case where the region is determined using a threshold method on the basis of having a feature in which statistical dispersion of the refractive index of the nucleus is equal to or greater than a threshold value.

[FIG. 37] FIG. 37 is a diagram concerning the method for determining a region of cells that have undergone necrosis according to an aspect of the present disclosure, showing a typical learning flow of a model in a learning step in a case where the region is determined by a machine learning method, when necrotic region data is created from fluorescence data of a reference observation object.

[FIG. 38] FIG. 38 is a diagram concerning a method for determining a region of cells that have undergone necrosis according to an aspect of the present disclosure, showing a typical inference flow in an inference step in a case where the region is determined by a machine learning method, when the necrotic region data is created from fluorescence data of a reference observation object.

[FIG. 39] FIG. 39 is a drawing concerning a method for evaluating a cancer cell cluster according to an aspect of the present disclosure, showing an example of a case in which a region of cells that have undergone necrosis inside a cancer cell cluster is large, and a cancer cell cluster existing closer to the central part is evaluated to be a cancer cell cluster of higher quality.

[FIG. 40] FIG. 40 is a drawing concerning a method for evaluating a cell cluster obtained by culturing stem cells according to an aspect of the present disclosure, showing an example of a step of producing a cell cluster.

[FIG. 41] FIG. 41 is a drawing concerning a method for evaluating a cell cluster obtained by culturing stem cells according to an aspect of the present disclosure, showing an example of a case in which a cell cluster having a smaller region of cells that have undergone necrosis inside the cell cluster is evaluated as a cell cluster of higher quality.

[FIG. 42] FIG. 42 is images showing representative refractive index tomography data extracted from refractive index distribution data of a HepG2 single cell obtained under the conditions without freeze-thawing or with freeze-thawing in Example 1.

[FIG. 43] FIG. 43 is images showing representative refractive index tomography data extracted from the refractive index distribution data of an A549 cell cluster that has undergone necrosis by a treatment of being left to stand at room temperature in Example 2.

[FIG. 44] FIG. 44 is images showing representative refractive index tomography data extracted from the refractive index distribution data of a HepG2 cell cluster that has undergone necrosis by a high-concentration ethanol treatment in Example 3.

[FIG. 45] FIG. 45 is images showing representative refractive index tomography data extracted from the refractive index distribution data of an A549 cell cluster that has undergone necrosis by a high-concentration ethanol treatment in Example 4.

[FIG. 46] FIG. 46 is images showing representative refractive index tomography data extracted from the refractive index distribution data of a HepG2 cell cluster including cells that have undergone necrosis in Example 5.

[FIG. 47] FIG. 47 is a diagram showing a flow in a case where a region of cells that have undergone necrosis is determined by a threshold method utilizing a feature of having a region equivalent to the nucleus and a feature in which the average value of the refractive index of a whole cell is equal to or less than a threshold value, as features of necrotic cells in Example 5.

[FIG. 48] FIG. 48 is a diagram showing a flow in a case where a region of cells that have undergone necrosis is determined by a threshold method utilizing a feature of having a region equivalent to the nucleus and a feature in which statistical dispersion of the refractive index of the nucleus is equal to or greater than a threshold value, as features of necrotic cells in Example 5.

**Description of Embodiments**

Hereinafter, embodiments will be specifically described with reference to the drawings. Incidentally, in the description of the drawings, the same elements will be assigned with the same reference numerals, and any overlapping descriptions will not be repeated. Actual embodiments are not limited to these examples.

Necrosis is a cell death occurring when cells are exposed to external factors, for example, hypoxia, high temperature, poisons, nutrient deficiency, and cell membrane damage. Cells that have undergone necrosis are characterized by morphology such as swelling of cells and disruption of cell membranes, while conspicuous nuclear fragmentation as in the case of apoptosis is not observed, and the features of the nucleus are maintained.

Cells that have undergone necrosis first undergo disruption of cell membranes and then undergo time-dependent processes such as leakage of the contents and subsequent disruption of cells including the nuclei, eventually reaching cell death, and therefore, it is thought that cells that are undergoing necrosis also undergo morphological changes similar to those of cells that have undergone necrosis. Therefore, in an embodiment, the method, apparatus, or information processing program according to an embodiment of the present disclosure may determine a region of cells that are undergoing necrosis, instead of a region of cells that have undergone necrosis.

When cell deaths are roughly classified into two classes of: (I) accidental cell death (ACD); and (II) programmed cell death (PCD) or regulated cell death (RCD), necrosis is classified into the former class. On the other hand, while the method, apparatus, or information processing program according to an embodiment of the present disclosure determines a region of cells that have undergone necrosis on the basis of having features of necrotic cells, in regard to necroptosis, which is one type of programmed cell deaths, it is known that morphological changes similar to those observed in necrosis, that is, swelling of cells, disruption of cell membranes, maintenance of features of the nucleus, and the like, and therefore, the method, apparatus, or information processing program according to an embodiment of the present disclosure can also determine a region of cells that have undergone necroptosis. That is, the method, apparatus, or information processing program according to an embodiment of the present disclosure may determine a region of cells that have undergone necrosis-like cell death instead of a region of cells that have undergone necrosis, or may determine, for example, a region of cells that have undergone necroptosis.

Necroptosis is a programmed cell death that exhibits morphology similar to those of necrosis (necrotic death), and is a process of self-destruction of cells that are activated when, for example, apoptosis is inhibited. Cells that have undergone necroptosis are characterized by morphology such as cell swelling and cell membrane disintegration similar to those of cells that have undergone necrosis, while on the other hand, conspicuous nuclear fragmentation as in the case of apoptosis is not observed.

The observation object according to an embodiment of the present disclosure is an object composed of cells as a main component; however, the observation object may also include components that may be contained in tissue samples other than cells, for example, extracellular matrix and neutral lipids. From the viewpoint of achieving both high reproducibility and the ease of sample preparation, the observation object according to an embodiment of the present disclosure may be a culture product of cells, and from the viewpoint of also achieving an environment close to a physiological environment as well, the observation object may be a cell cluster. When the observation object is a cell cluster, it is possible to determine a region of cells that have undergone necrosis inside a cell cluster that cannot be evaluated using the appearance as an indicator. That is, a region of cells that have undergone necrosis in the observation object according to an embodiment is a region of cells that have undergone necrosis inside a cell cluster. The inside of a cell cluster means cells that are not exposed to the surface of the cell cluster, among the cells forming the cell cluster. When the observation object is a culture product of cells, the culture product may be composed of one kind of cells or may include two or more kinds of cells. When the observation object is a cell cluster, the maximum diameter thereof may be 100 to 200 $\mu$m.

The refractive index distribution data is data indicating a three-dimensional distribution of the refractive index of each voxel in a space including the observation object, or data indicating a two-dimensional distribution of the refractive index for each pixel in a tomographic plane in a predetermined direction in a space including the observation object. Furthermore, the refractive index tomography data is data indicating a two-dimensional distribution of the refractive index for each pixel in a tomographic plane in a predetermined direction in a space including the observation object, among the refractive index distribution data.

A method for acquiring refractive index distribution data according to an embodiment of the present disclosure (hereinafter, also referred to as "method for measuring refractive index distribution") is not particularly limited; however, one method will be described in detail below. As a method for measuring the refractive index distribution of an observation object in a non-staining and non-invasive manner, optical diffraction tomography (ODT) is known. ODT is a result of developing quantitative phase imaging (QPI) into a technology capable of three-dimensional imaging, and can realize three-dimensional refractive index tomography of an observation object.

Further, refractive index distribution measurement methods A to C described below are used. Embodiments of the refractive index distribution measurement method A include refractive index distribution measurement methods A1 to A3. The refractive index distribution measurement method A is an all-inclusive term for the refractive index distribution measurement methods A1 to A3. In such refractive index distribution measurement methods A to C, even in a case where the observation object is a multiple scatterer, it is possible to attain the three-dimensional refractive index tomography on which the influence of multiple scattering light is reduced.

Optical Coherence Tomography (OCT) is also known as another staining and non-invasive imaging technique. However, the resolution of OCT is about 10 $\mu$m, whereas the resolution of ODT and refractive index distribution measuring methods A to C is about 1 $\mu$m. In addition, OCT does not obtain a refractive index distribution, and it is difficult to biologically interpret a signal obtained by imaging. In these respects, ODT and the refractive index distribution measuring methods A to C are superior to OCT.

First, the refractive index distribution measuring method A (A1 to A3) will be described. FIG. A01 to A03 are diagrams showing respective configurations of observation apparatuses 1A to 1C that can be used when measuring the refractive index distribution by the refractive index distribution measuring method A.

FIG. A01 is a diagram illustrating a configuration of an observation apparatus 1A. The observation apparatus 1A includes a light source 11, a lens 12, a lens 21, a mirror 22, a lens 23, a condenser lens 24, an objective lens 25, a beam splitter 41, a lens 42, an imaging unit 43, and an analysis unit 50.

The light source 11 outputs spatially and temporally coherent light, and is preferably a laser light source. The lens 12 is optically coupled to the light source 11, focuses the light output from the light source 11 on a light input end 13 of an optical fiber 14, and inputs the light to the light input end 13. The optical fiber 14 guides the light input to the light input end 13 by the lens 12 to a fiber coupler 15. The fiber coupler 15 couples the light between the optical fiber 14 and optical fibers 16 and 17, splits the light guided by and arriving from the optical fiber 14 into two light beams, guides one split light by the optical fiber 16, and guides the other split light by the optical fiber 17. The light guided by the optical fiber 16 is output as diverging light from a light output end 18. The light guided by the optical fiber 17 is output as diverging light from a light output end 19.

The lens 21 is optically coupled to the light output end 18, and collimates the light output as the diverging light from the light output end 18. The mirror 22 is optically coupled to the lens 21, and reflects the light arriving from the lens 21 to the lens 23. An orientation of a reflection surface of the mirror 22 is changeable. The lens 23 is optically coupled to the mirror 22. The condenser lens 24 is optically coupled to the lens 23. The lens 23 and the condenser lens 24 preferably constitute a 4f optical system. The lens 23 and the condenser lens 24 irradiate an observation object S with the light from a light irradiation direction according to the orientation of the reflection surface of the mirror 22. The objective lens 25 is optically coupled to the condenser lens 24. The observation object S is disposed between the objective lens 25 and the condenser lens 24. The objective lens 25 inputs the light (object light) output from the condenser lens 24 and passed through the observation object S, and outputs the light to the beam splitter 41.

The beam splitter 41 is optically coupled to the objective lens 25, and further, is optically coupled also to the light output end 19. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images an interference fringe image (interference intensity image) generated by interference between the object light and the reference light arriving from the lens 42. An incident direction of the reference light is inclined with respect to an incident direction of the object light on an imaging plane of the imaging unit 43. A position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

The analysis unit 50 is electrically connected to the imaging unit 43, and inputs the interference intensity image captured by the imaging unit 43. The analysis unit 50 calculates a three-dimensional refractive index distribution of the observation object S by processing the input interference intensity image. The analysis unit 50 may be a computer. The analysis unit 50 includes an interference intensity image acquisition unit 51, a first complex amplitude image generation unit 52, a second complex amplitude image generation unit 53, a two-dimensional phase image generation unit 54, a three-dimensional phase image generation unit 55, a refractive index distribution calculation unit 56, a display unit 57, and a storage unit 58.

The interference intensity image acquisition unit 51 irradiates the observation object S with the light along each of a plurality of light irradiation directions by changing the orientation of the reflection surface of the mirror 22. Further, the interference intensity image acquisition unit 51 acquires the interference intensity image at a reference position for each of the plurality of light irradiation directions from the imaging unit 43. The interference intensity image acquisition unit 51 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 52, the second complex amplitude image generation unit 53, the two-dimensional phase image generation unit 54, the three-dimensional phase image generation unit 55, and the refractive index distribution calculation unit 56 perform processing based on the interference intensity images, and include a processing device such as a CPU, a GPU, a DSP, or an FPGA. The display unit 57 displays an image to be processed, an image in the middle of the processing, an image after the processing, and the like, and includes, for example, a liquid crystal display. The storage unit 58 stores data of various images, and includes a hard disk drive, a flash memory, a RAM, a ROM, and the like. The first complex amplitude image generation unit 52, the second complex amplitude image generation unit 53, the two-dimensional phase image generation unit 54, the three-dimensional phase image generation unit 55, the refractive index distribution calculation unit 56, and the storage unit 58 may be constituted by a cloud computing.

The storage unit 58 also stores a program for causing the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56, to execute respective steps of the processing. The program may be stored in the storage unit 58 at the time of manufacture or shipment of the observation apparatus 1A, may be acquired via a communication line after shipment and then stored in the storage unit 58, or may be recorded in a computer readable recording medium 2 and then stored in the storage unit 58. The recording medium 2 may be an arbitrary medium such as a flexible disk, a CD-ROM, a DVD-ROM, a BD-ROM, a USB memory, or the like.

The details of the processing step of each of the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56 will be described later.

FIG. A02 is a diagram illustrating a configuration of an observation apparatus 1B. The observation apparatus 1B illustrated in FIG. A02 includes a lens 31, a mirror 32, and a lens 34 in addition to the configuration of the observation apparatus 1A illustrated in FIG. A01.

The lens 31 is optically coupled to the light output end 19, and collimates the light (reference light) output as diverging light from the light output end 19. The mirror 32 is optically coupled to the lens 31, and reflects the light arriving from the lens 31 to the lens 34. The lens 34 is optically coupled to the mirror 32, and outputs the light arriving from the mirror 32 to the beam splitter 41. The light output from the lens 34 is once focused before the beam splitter 41, and then input to the beam splitter 41 as diverging light. The beam splitter 41 combines the light (object light) output and arriving from the objective lens 25 and the light (reference light) output and arriving from the lens 34, and outputs the light to the lens 42 in a coaxial manner. The imaging unit 43 images the interference fringe image (interference intensity image) generated by interference between the object light and the reference light arriving from the lens 42. The incident direction of the reference light is parallel to the incident direction of the object light on the imaging plane of the imaging unit 43.

A drive unit 33 moves the mirror 32 in a direction perpendicular to a reflection surface of the mirror 32. The drive unit 33 is, for example, a piezoelectric actuator. The movement of the mirror 32 changes an optical path difference (phase difference) of the object light and the reference light from light splitting by the fiber coupler 15 to combining by the beam splitter 41. When the optical path difference is different, the interference intensity image captured by the imaging unit 43 is also different.

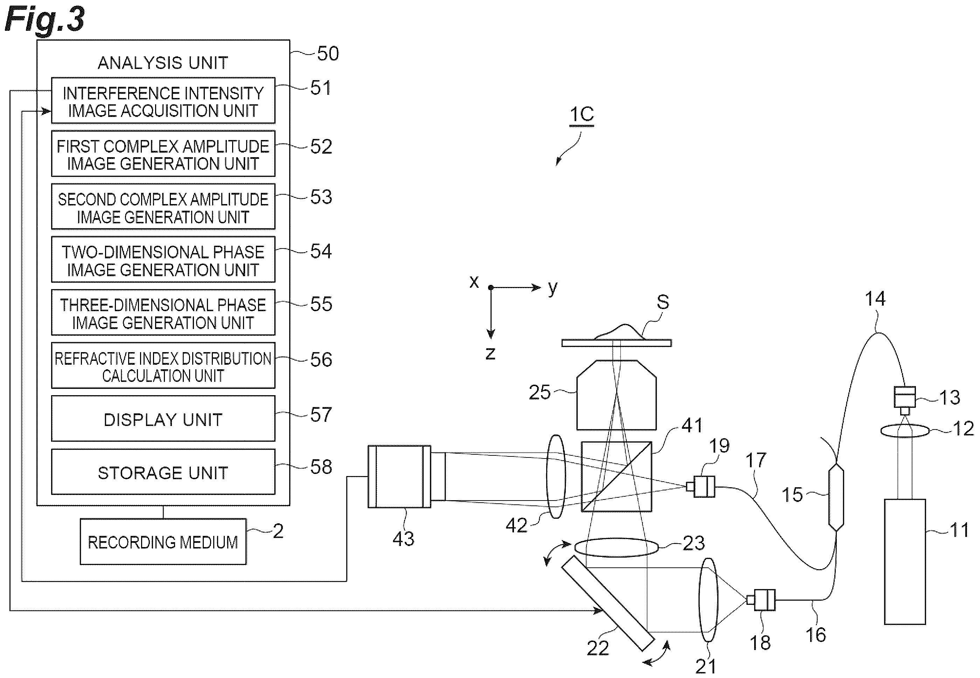

The observation apparatus is not limited to the configuration examples illustrated in FIG. A01 and FIG. A02, and various modifications are possible. In the configuration of the observation apparatus 1A (FIG. A01) and the observation apparatus 1B (FIG. A02), the object light transmitted through the observation object S is observed, and the object light reflected by the observation object S may be observed as in a configuration of an observation apparatus 1C (FIG. 3) described below.

FIG. A03 is a diagram illustrating a configuration of an observation apparatus 1C. The observation apparatus 1C includes the light source 11, the lens 12, the lens 21, the mirror 22, the lens 23, the objective lens 25, the beam splitter 41, the lens 42, the imaging unit 43, and the analysis unit 50. Hereinafter, differences from the observation apparatus 1A (FIG. A01) will be mainly described.

The lens 21 is optically coupled to the light output end 18 of the optical fiber 16, and collimates the light output as diverging light from the light output end 18. The mirror 22 is optically coupled to the lens 21, and reflects the light arriving from the lens 21 to the lens 23. The orientation of the reflection surface of the mirror 22 is changeable. The lens 23 is optically coupled to the mirror 22. The objective lens 25 is optically coupled to the lens 23. The beam splitter 41 is disposed between the lens 23 and the objective lens 25. The lens 23 and the objective lens 25 preferably constitute a 4f optical system. The lens 23 and the objective lens 25 irradiate the observation object S with the light from the light irradiation direction according to the orientation of the reflection surface of the mirror 22. The objective lens 25 inputs the light (object light) reflected from the observation object S, and outputs the light to the beam splitter 41.

The beam splitter 41 is optically coupled to the objective lens 25, and further, is optically coupled also to the light output end 19 of the optical fiber 17. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images the interference fringe image (interference intensity image) generated by interference between the object light and the reference light arriving from the lens 42. The incident direction of the reference light is inclined with respect to the incident direction of the object light on the imaging plane of the imaging unit 43. The position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

In the configuration of the observation apparatus 1C (FIG. A03), as in the observation apparatus 1B (FIG. A02), the mechanism (the lens 31, the mirror 32, the drive unit 33, and the lens 34 in FIG. A02) for changing the optical path length of the reference light may be provided for changing the optical path difference (phase difference) of the object light and the reference light from light splitting by the fiber coupler 15 to combining by the beam splitter 41. In this case, the incident direction of the reference light may be parallel to the incident direction of the object light on the imaging plane of the imaging unit 43.

FIG. A04 is a flowchart of a refractive index distribution measuring method A. The refractive index distribution measuring method A can be applied to each of the observation apparatus 1A to 1C. The refractive index distribution measuring method A includes an interference intensity image acquisition step S1, a first complex amplitude image generation step S2, a second complex amplitude image generation step S3, a two-dimensional phase image generation step S4, a three-dimensional phase image generation step S5, and a refractive index distribution calculation step S6.

The processing step of the interference intensity image acquisition step S1 is performed by the interference intensity image acquisition unit 51. The processing step of the first complex amplitude image generation step S2 is performed by the first complex amplitude image generation unit 52. The processing step of the second complex amplitude image generation step S3 is performed by the second complex amplitude image generation unit 53. The processing step of the two-dimensional phase image generation step S4 is performed by the two-dimensional phase image generation unit 54. The processing step of the three-dimensional phase image generation step S5 is performed by the three-dimensional phase image generation unit 55. The processing step of the refractive index distribution calculation step S6 is performed by the refractive index distribution calculation unit 56.

In the interference intensity image acquisition step S1, the interference intensity image acquisition unit 51 irradiates the observation object S with the light along each of the plurality of light irradiation directions by changing the orientation of the reflection surface of the mirror 22. Further, the interference intensity image acquisition unit 51 acquires the interference intensity image at the reference position for each of the plurality of light irradiation directions from the imaging unit 43.

In each of FIG. A01 to FIG. A03, an xyz orthogonal coordinate system is illustrated for convenience of explanation. The z axis is parallel to the optical axis of the objective lens 25. The reference position is the image plane position having a conjugate relationship with respect to the imaging plane of the imaging unit 43. This position is set to z = 0. The light irradiation direction on the observation object S can be represented by $k_x$ and $k_y$ in a wavenumber vector ($k_x$, $k_y$, $k_z$) of the irradiation light.

(a) to (c) in FIG. A05 are diagrams illustrating examples of scanning of the light irradiation direction on the observation object S in the interference intensity image acquisition step S1. In the diagram, a position of each circular point represents the light irradiation direction in the $k_x k_y$ plane in which the horizontal axis is set to $k_x$ and the vertical axis is set to $k_y$. The scanning of the light irradiation direction may be arranged in a rectangular lattice shape in the $k_x k_y$ plane as illustrated in (a) in FIG. A05, may be arranged on a circumference of each of a plurality of concentric circles in the $k_x k_y$ plane as illustrated in (b) in FIG. A05, or may be arranged in a spiral shape in the $k_x k_y$ plane as illustrated in (c) in FIG. A05. In any of the cases, the light irradiation direction can be scanned as far as it is allowed by Numerical Aperture (NA) of the condenser lens 24. Raster scan or random scan may be used. In the case of the raster scan, return scan may be performed or may not be performed.

In the first complex amplitude image generation step S2, the first complex amplitude image generation unit 52 generates, for each of the plurality of light irradiation directions, a complex amplitude image based on the interference intensity image acquired by the interference intensity image acquisition unit 51. In the case of the observation apparatus 1A (FIG. A01) or the observation apparatus 1C (FIG. A03), the first complex amplitude image generation unit 52 can generate the complex amplitude image based on one interference intensity image by a Fourier fringe analysis method. In the case of the observation apparatus 1B (FIG. A02), the first complex amplitude image generation unit 52 can generate the complex amplitude image based on three or more interference intensity images having different optical path differences (phase differences) between the object light and the reference light by a phase shift method.

In the second complex amplitude image generation step S3, the second complex amplitude image generation unit 53 generates, for each of the plurality of light irradiation directions, a complex amplitude image at each of a plurality of z direction positions based on the complex amplitude image at a reference position (z = 0) generated by the first complex amplitude image generation unit 52. Assuming that a two-dimensional Fourier transform of the complex amplitude image u(x, y, 0) at the reference position is U($k_x$, $k_y$, 0), the complex amplitude image u(x, y, d) at the position of z = d and the two-dimensional Fourier transform $U(k_x, k_y, d)$ of the complex amplitude image $u(x, y, d)$ are represented by the following Formulas. i is an imaginary unit, and $k_0$ is a wavenumber of the light in the observation object.

[Formula 1]

$$U(k_x, k_y, d) = U(k_x, k_y, 0) \exp\left(i\sqrt{k_0^2 - k_x^2 - k_y^2}\, d\right) \qquad (1)$$

[Formula 2]

$$u(x, y, d) = \int U(k_x, k_y, d) \exp(-ik_x x - ik_y y)\, dk_x dk_y \qquad (2)$$

In the two-dimensional phase image generation step S4, the two-dimensional phase image generation unit 54 generates, for each of the plurality of positions, a two-dimensional phase image based on the complex amplitude image of each of the plurality of light irradiation directions generated by the second complex amplitude image generation unit 53. The two-dimensional phase image generated in this step corresponds to a phase image centered on the focused z direction position. The details of the two-dimensional phase image generation step S4 will be described below.

In addition, the two-dimensional phase image generation step S4 and the subsequent steps may be performed after all the complex amplitude images at the plurality of positions are generated for each of the plurality of light irradiation directions in the second complex amplitude image generation step S3. Further, processes of generating the complex amplitude image at one certain z direction position for each of the plurality of light irradiation directions in the second complex amplitude image generation step S3 and generating the two-dimensional phase image at the position in the two-dimensional phase image generation step S4 may be set as a unit, and the unit process may be repeatedly performed while scanning the z direction position. The latter case is preferable in that a capacity of image data to be stored in the storage unit 58 can be reduced.

In the three-dimensional phase image generation step S5, the three-dimensional phase image generation unit 55 generates a three-dimensional phase image based on the two-dimensional phase image at each of the plurality of positions generated by the two-dimensional phase image generation unit 54. The three-dimensional phase image generated in this step is an image in which the positions x and y in the two-dimensional phase image and the position z of the two-dimensional phase image are variables.

In the refractive index distribution calculation step S6, the refractive index distribution calculation unit 56 obtains a three-dimensional refractive index distribution of the observation object by deconvolution based on the three-dimensional phase image generated by the three-dimensional phase image generation unit 55. Assuming that the refractive index distribution of the observation object is n(x, y, z), an electric susceptibility distribution is f(x, y, z), and a refractive index of a background medium is $n_m$, there is a relationship of the following Formula (3) between them. The three-dimensional phase image $\Phi(x, y, z)$ generated by the three-dimensional phase image generation unit 55 is represented by convolution of a kernel function g(x, y, z) and the electric susceptibility distribution f(x, y, z) as shown in the following Formula (4). Therefore, the three-dimensional refractive index distribution n(x, y, z) of the observation object can be obtained by deconvolution based on the three-dimensional phase image $\Phi(x, y, z)$.

[Formula 3]

$$f(x, y, z) = k_0^2 \left[ \left( n(x, y, z)/n_m \right)^2 - 1 \right] \qquad (3)$$

[Formula 4]

$$\Phi(x, y, z) = \int g(x - x', y - y', z - z')\, f(x', y', z')\, dx'dy'dz' \qquad (4)$$

In addition, the kernel function g is a function based on a Green function corresponding to a solution of a wave equation. FIG. A06 is a diagram showing the kernel function g. In this diagram, a center position having the largest value of the kernel function g is the origin, the vertical direction is the z axis, and the horizontal direction is the direction perpendicular to the z axis.

Each of the processing steps of the first complex amplitude image generation step S2, the second complex amplitude image generation step S3, the two-dimensional phase image generation step S4, the three-dimensional phase image generation step S5, and the refractive index distribution calculation step S6 may be performed each time the interference intensity image of each of a predetermined number of light irradiation directions is acquired in the interference intensity image acquisition step S1 (FIG. A07), or may be performed each time the interference intensity image of one light irradiation direction is acquired in the interference intensity image acquisition step S1 (FIG. A08).

FIG. A07 and FIG. A08 are diagrams illustrating examples of scanning of the light irradiation direction on the observation object S in the interference intensity image acquisition step S1. In these diagrams, a position of each circular point represents the light irradiation direction in the $k_x k_y$ plane in which the horizontal axis is set to $k_x$ and the vertical axis is set to $k_y$. In the examples of scanning of the light irradiation direction illustrated in these diagrams, the light irradiation direction is sequentially changed, and the light irradiation direction at the time of acquisition of the (N+n)-th interference intensity image is made to coincide with the light irradiation direction at the time of acquisition of the n-th interference intensity image. n is a positive integer, and N is an integer of 2 or more.

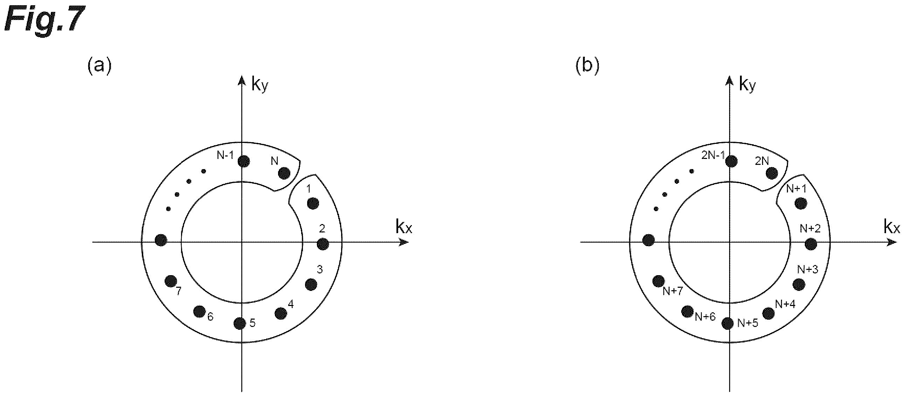

In the example illustrated in FIG. A07, when the first to N-th interference intensity images are acquired in the interference intensity image acquisition step S61, the respective processing steps of the steps S2 to S6 are performed based on the first to N-th interference intensity images ((a) in FIG. 7). Next, when the (N+1)-th to 2N-th interference intensity images are acquired in the interference intensity image acquisition step S1, the respective processing steps of the steps S2 to S6 are performed based on the (N+1)-th to 2N-th interference intensity images ((b) in FIG. A07). Next, when the (2N+1)-th to 3N-th interference intensity images are acquired in the interference intensity image acquisition step S1, the respective processing steps of the steps S2 to S6 are performed based on the (2N+1)-th to 3N-th interference intensity images. The same applies thereafter.

In the example illustrated in FIG. A08, when the first to N-th interference intensity images are acquired in the interference intensity image acquisition step S1, the respective processing steps of the steps S2 to S6 are performed based on the first to N-th interference intensity images ((a) in FIG. A08). Next, when the (N+1)-th interference intensity image is acquired in the interference intensity image acquisition step S61, the respective processing steps of the steps S2 to S6 are performed based on the latest N interference intensity images (the second to (N+1)-th interference intensity images) including the (N+1)-th interference intensity image ((b) in FIG. A08). Next, when the (N+2)-th interference intensity image is acquired in the interference intensity image acquisition step S1, the respective processing steps of the steps S2 to S6 are performed based on the latest N interference intensity images (the third to (N+2)-th interference intensity images) including the (N+2)-th interference intensity image ((c) in FIG. A08). The same applies thereafter, and when the (N+n)-th interference intensity image is acquired in the interference intensity image acquisition step S1, the respective processing steps of the steps S2 to S6 are performed based on the latest N interference intensity images (the (1+n)-th to (N+n)-th interference intensity images) including the (N+n)-th interference intensity image.

Compared with the example illustrated in FIG. A07, in the example illustrated in FIG. A08, each time the interference intensity image of one light irradiation direction is acquired in the interference intensity image acquisition step S1, the respective processing steps of the steps S2 to S6 are performed based on the plurality of latest interference intensity images including the acquired interference intensity image, and thus, the number of images obtained per unit time by the respective processing steps of the steps S2 to S6 is large.

Next, the details of the two-dimensional phase image generation step S4 in the refractive index distribution measuring method A will be described. In the two-dimensional phase image generation step S4, the two-dimensional phase image generation unit 54 generates, for each of the plurality of positions, the two-dimensional phase image based on the complex amplitude image of each of the plurality of light irradiation directions generated by the second complex amplitude image generation unit 53. The two-dimensional phase image generation step S4 depends on the refractive index distribution measuring method A1 to A3.

FIG. A09 is a flowchart of the two-dimensional phase image generation step S4 in the refractive index distribution measuring method A1. In the refractive index distribution measuring method A1, for each of the plurality of positions, the two-dimensional phase image generation step S4, in a step S11, corrects the phase of the complex amplitude image of each of the plurality of light irradiation directions based on the light irradiation direction, and then generates a complex amplitude summation image representing a summation of the complex amplitude images after the correction, and in a step S12, generates the two-dimensional phase image based on the complex amplitude summation image.

The processing of the step S11 is based on a CASS (Collective Accumulation of Single Scattering; Sungsam Kang, et al, "Imaging deep within a scattering medium using collective accumulation of single-scattered waves," NATURE PHOTONICS, Vol.9, pp.253-258 (2015)) technique. In the light with which the object is irradiated along a certain light irradiation direction and passed through the object, a spatial frequency distribution of the single scattered light which interacts with the object only once is shifted according to the light irradiation direction, whereas a spatial frequency distribution of the multiple scattered light which interacts with the object a plurality of times randomly changes according to the light irradiation direction. The CASS technique uses the above difference between the light irradiation direction dependencies of the spatial frequency distributions of the single scattered light and the multiple scattered light.

That is, in the step S11, the phase of the complex amplitude image of each of the plurality of light irradiation directions is corrected based on the light irradiation direction (that is, the spatial frequency distribution of the complex amplitude image is shifted in parallel according to the light irradiation direction in the spatial frequency domain), so that the spatial frequency distribution of the single scattered light component in the complex amplitude image has a shape and arrangement independent of the light irradiation direction, while the spatial frequency distribution of the multiple scattered light component in the complex amplitude image has a random shape and arrangement. Further, in the step S11, the complex amplitude summation image representing the summation of the plurality of complex amplitude images after the above correction is generated (that is, synthetic aperture processing is performed) to coherently sum the single scattered light components in the complex amplitude images, while the multiple scattered light components in the complex amplitude images cancel each other out.

Therefore, the influence of the multiple scattered light is reduced in the complex amplitude summation image generated in the step S11. Further, the three-dimensional refractive index distribution obtained finally in the refractive index distribution calculation step S6 also reduces the influence of the multiple scattered light, suppresses the speckles, and improves the Single-scattering to Multi-scattering Ratio (SMR).

FIG. A10 is a flowchart of the two-dimensional phase image generation step S4 in the refractive index distribution measuring method A2. In the refractive index distribution measuring method A2, for each of the plurality of positions, the two-dimensional phase image generation step S4, in a step S21, generates a complex differential interference image of each of the plurality of light irradiation directions based on the complex amplitude image of each of the plurality of light irradiation directions. In a step S22, the step generates a phase differential image based on a summation of the complex differential interference images of the plurality of light irradiation directions. In a step S23, the step generates the two-dimensional phase image based on the phase differential image.

Assuming that the complex amplitude image at the position of z = d is u(x, y, d), the complex differential interference image q(x, y, d) generated in the step S21 is represented by the following Formula (5). At least one of $\delta x$ and $\delta y$ is non-zero. When $\delta x \neq 0$ and $\delta y = 0$, the complex differential interference image q in which the x direction is a shear direction is obtained. When $\delta x = 0$ and $\delta y \neq 0$, the complex differential interference image q in which the y direction is the shear direction is obtained. When $\delta x \neq 0$ and $\delta y \neq 0$, the complex differential interference image q with the shear direction different from both of the x direction and the y direction is obtained. In addition, the complex differential interference image q(x, y, d) may be obtained by Formula (5) after transforming the complex amplitude image u(x, y, d) as in the following Formula (6).

[Formula 5]

$$q(x,y,d) = u^{*}(x+\delta x, y+\delta y, d) \cdot u(x,y,d) \qquad (5)$$

[Formula 6]

$$u(x,y,d)\exp(-ik_{x}x - ik_{y}y) \qquad (6)$$

Assuming that the summation of the complex differential interference images q of the plurality of light irradiation directions is $q_{sum}(x, y, d)$, the phase differential image $\phi(x, y, z)$ generated in the step S22 is represented by the following Formula (7) as the phase of $q_{sum}(x, y, d)$. In the step S23, the two-dimensional phase image can be generated by performing integration or deconvolution of the phase differential image $\phi(x, y, z)$.

[Formula 7]

$$\phi(x,y,d) = \angle q_{sum}(x,y,d) \qquad (7)$$

In addition, in the step S21, the complex differential interference image may be generated for each of a plurality of shear directions different from each other on the complex amplitude image. In this case, for each of the plurality of positions, the two-dimensional phase image generation step S4, in the step S21, generates the complex differential interference image of each of the plurality of light irradiation directions for each of the plurality of shear directions on the image different from each other based on the complex amplitude image of each of the plurality of light irradiation directions. In the step S22, the step generates the phase differential image based on the summation of the complex differential interference images of the plurality of light irradiation directions for each of the plurality of shear directions. In the step S23, the step generates the two-dimensional phase image based on the phase differential image of each of the plurality of shear directions.

The influence of the multiple scattered light is reduced in the phase differential image generated based on the summation of the complex differential interference image of each of the plurality of light irradiation directions in the step S22. Further, the three-dimensional refractive index distribution obtained finally in the refractive index distribution calculation step S6 also reduces the influence of the multiple scattered light, and suppresses the speckles. Further, when the complex differential interference image is generated for each of the plurality of shear directions different from each other on the complex amplitude image in the step S21, it is possible to suppress the appearance of linear noises in the two-dimensional phase image obtained in the step S23.

In the above description, the case in which the two-dimensional phase image is generated by performing integration or deconvolution of the phase differential image in the step S23 is described. However, the phase differential image may also be treated as the two-dimensional phase image. In this case, the three-dimensional refractive index distribution of the observation object can be obtained from the phase differential image (two-dimensional phase image) generated in the step S22 by using a kernel (FIG. A11) including a kernel used in deconvolution of the step S23, in deconvolution of the refractive index distribution calculation step S67, without performing the step S23. The kernel shown in FIG. A11 is obtained by convolution integration of the kernel shown in FIG. A06 and the kernel used in deconvolution of the step S23.

FIG. A12 is a flowchart of the two-dimensional phase image generation step S4 in the refractive index distribution measuring method A3. In the refractive index distribution measuring method A3, for each of the plurality of positions, the two-dimensional phase image generation step S4, in a step S31, divides the complex amplitude image of each of the plurality of light irradiation directions into a plurality of batches, corrects the phase of the complex amplitude image included in the batch based on the light irradiation direction for each of the plurality of batches, and then generates the complex amplitude summation image representing the summation of the complex amplitude images after the correction, in a step S32, generates the complex differential interference image of each of the plurality of batches based on the complex amplitude summation image of each of the plurality of batches, in a step S33, generates the phase differential image based on the summation of the complex differential interference images of the plurality of batches, and in a step S34, generates the two-dimensional phase image based on the phase differential image.

The processing of the step S31 in the refractive index distribution measuring method A3 corresponds to dividing the complex amplitude image of each of the plurality of light irradiation directions into the plurality of batches, and then performing the processing of the step S11 in the refractive index distribution measuring method A1 for each of the plurality of batches. The processing of the steps S32 and S33 in the refractive index distribution measuring method A3 corresponds to performing the processing of the steps S21 and S22 in the refractive index distribution measuring method A2 for each of the plurality of batches. The processing of the step S34 in the refractive index distribution measuring method A3 corresponds to performing the processing of the step S23 in the refractive index distribution measuring method A2.

In addition, in the step S32, the complex differential interference image may be generated for each of the plurality of shear directions different from each other on the complex amplitude image. In this case, the two-dimensional phase image generation step S4, in the step S32, generates the complex differential interference image of each of the plurality of batches for each of the plurality of shear directions on the image different from each other based on the complex amplitude summation image of each of the plurality of batches, in the step S33, generates the phase differential image based on the summation of the complex differential interference images of the plurality of batches for each of the plurality of shear directions, and in the step S34, generates the two-dimensional phase image based on the phase differential image of each of the plurality of shear directions.

The suppression of the speckles in the refractive index distribution measuring method A3 is comparable with the refractive index distribution measuring method A1 and the refractive index distribution measuring method A2. The improvement of the SMR in the refractive index distribution measuring method A3 is an intermediate degree between the refractive index distribution measuring method A1 and the refractive index distribution measuring method A2.

In the above description also, the case in which the two-dimensional phase image is generated by performing integration or deconvolution of the phase differential image in the step S34 is described. However, the phase differential image may also be treated as the two-dimensional phase image. In this case, the three-dimensional refractive index distribution of the observation object can be obtained from the phase differential image (two-dimensional phase image) generated in the step S33 by using the kernel including the kernel used in deconvolution of the step S34, in deconvolution of the refractive index distribution calculation step S6, without performing the step S34.

Next, the refractive index distribution measuring method B will be will be described. FIG. B01 to B03 are diagrams showing respective configurations of observation apparatuses 1D to 1F that can be used when measuring the refractive index distribution by the refractive index distribution measuring method B. The observation apparatus 1D illustrated in FIG. B01, as compared with the configuration of the observation apparatus 1A illustrated in FIG. A01, has the common configuration for the optical system from the light source 11 to the imaging unit 43, and further, is different in that an analysis unit 60 is provided instead of the analysis unit 50. The observation apparatus 1E illustrated in FIG. B02, as compared with the configuration of the observation apparatus 1B illustrated in FIG. A02, has the common configuration for the optical system from the light source 11 to the imaging unit 43, and further, is different in that the analysis unit 60 is provided instead of the analysis unit 50. The observation apparatus 1F illustrated in FIG. B03, as compared with the configuration of the observation apparatus 1C illustrated in FIG. A03, has the common configuration for the optical system from the light source 11 to the imaging unit 43, and further, is different in that the analysis unit 60 is provided instead of the analysis unit 50.

The analysis unit 60 is electrically connected to the imaging unit 43, and inputs the interference intensity image output from the imaging unit 43. The analysis unit 60 calculates a three-dimensional refractive index distribution of the observation object S by processing the input interference intensity image. The analysis unit 60 may be a computer. The analysis unit 60 includes an interference intensity image acquisition unit 61, a first complex amplitude image generation unit 62, a second complex amplitude image generation unit 63, a phase conjugate operation unit 64, a two-dimensional phase image generation unit 65, a three-dimensional phase image generation unit 66, a refractive index distribution calculation unit 67, a display unit 68, and a storage unit 69.

The interference intensity image acquisition unit 61 irradiates the observation object S with the light along each of a plurality of light irradiation directions by changing the orientation of the reflection surface of the mirror 22. Further, the interference intensity image acquisition unit 61 acquires the interference intensity image at a reference position for each of the plurality of light irradiation directions from the imaging unit 43. The interference intensity image acquisition unit 61 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 perform processing based on the interference intensity images, and include a processing device such as a CPU, a GPU, a DSP, or an FPGA. The display unit 68 displays an image to be processed, an image in the middle of the processing, an image after the processing, and the like, and includes, for example, a liquid crystal display. The storage unit 69 stores data of various images, and includes a hard disk drive, a flash memory, a RAM, a ROM, and the like. The first complex amplitude image generation unit 62, the second complex amplitude image generation unit 63, the phase conjugate operation unit 64, the two-dimensional phase image generation unit 65, the three-dimensional phase image generation unit 66, the refractive index distribution calculation unit 67, and the storage unit 68 may be constituted by a cloud computing.

The storage unit 69 also stores a program for causing the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67, to execute respective steps of the processing. The program may be stored in the storage unit 69 at the time of manufacture or shipment of the observation apparatus 1D to 1F, may be acquired via a communication line after shipment and then stored in the storage unit 69, or may be recorded in a computer readable recording medium 2 and then stored in the storage unit 69. The recording medium 2 may be an arbitrary medium such as a flexible disk, a CD-ROM, a DVD-ROM, a BD-ROM, a USB memory, or the like.

The details of the processing step of each of the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 will be described later.

FIG. B04 is a flowchart of the refractive index distribution measuring method B. The refractive index distribution measuring method B can be applied to each of the observation apparatus 1D to 1F. The refractive index distribution measuring method B includes an interference intensity image acquisition step S61, a first complex amplitude image generation step S62, a second complex amplitude image generation step S63, a phase conjugate operation step S64, a two-dimensional phase image generation step S65, a three-dimensional phase image generation step S66, and a refractive index distribution calculation step S67.

The processing step of the interference intensity image acquisition step S61 is performed by the interference intensity image acquisition unit 61. The processing step of the first complex amplitude image generation step S62 is performed by the first complex amplitude image generation unit 62. The processing step of the second complex amplitude image generation step S63 is performed by the second complex amplitude image generation unit 63. The processing step of the phase conjugate operation step S64 is performed by the phase conjugate operation unit 64. The processing step of the two-dimensional phase image generation step S65 is performed by the two-dimensional phase image generation unit 65. The processing step of the three-dimensional phase image generation step S66 is performed by the three-dimensional phase image generation unit 66. The processing step of the refractive index distribution calculation step S67 is performed by the refractive index distribution calculation unit 67.

In the interference intensity image acquisition step S61, the interference intensity image acquisition unit 61 irradiates the observation object S with the light along each of the plurality of light irradiation directions by changing the orientation of the reflection surface of the mirror 22. Further, the interference intensity image acquisition unit 61 acquires the interference intensity image at the reference position for each of the plurality of light irradiation directions from the imaging unit 43.

In the first complex amplitude image generation step S62, the first complex amplitude image generation unit 62 generates, for each of the plurality of light irradiation directions, a complex amplitude image based on the interference intensity image of the reference position acquired by the interference intensity image acquisition unit 61. In the case of the observation apparatus 1D (FIG. B01) or the observation apparatus 1F (FIG. B03), the first complex amplitude image generation unit 62 can generate the complex amplitude image based on one interference intensity image by a Fourier fringe analysis method. In the case of the observation apparatus 1E (FIG. B02), the first complex amplitude image generation unit 62 can generate the complex amplitude image based on three or more interference intensity images having different optical path differences (phase differences) between the object light and the reference light by a phase shift method.

In the second complex amplitude image generation step S63, the second complex amplitude image generation unit 63 generates, for each of the plurality of light irradiation directions, a complex amplitude image at each of a plurality of z direction positions based on the complex amplitude image at the reference position (z = 0) generated by the first complex amplitude image generation unit 62.

The interference intensity image acquisition step S61, the first complex amplitude image generation step S62, and the second complex amplitude image generation step S63 in the refractive index distribution measuring method B respectively perform the same processing steps as the interference intensity image acquisition step S1, the first complex amplitude image generation step S2, and the second complex amplitude image generation step S3 in the refractive index distribution measuring method A.

The phase conjugate operation step S64 is performed after the processing step of the second complex amplitude image generation step S63. The phase conjugate operation step S64 may be performed before the processing step of the second complex amplitude image generation step S63 (which will be described later). Further, when the second complex amplitude image generation step S63 generates the complex amplitude image at a certain z position through a plurality of stages from the complex amplitude image at the reference position, the phase conjugate operation step S64 may be performed between a certain stage and a next stage in the plurality of stages (which will be described later). In the phase conjugate operation step S64, the phase conjugate operation unit 64 performs a phase conjugate operation on the complex amplitude image of each of the plurality of light irradiation directions to generate a complex amplitude image of each of the plurality of light irradiation directions when the relationship between the light irradiation and the imaging for the observation object is reversed.

In addition, the phase conjugate operation is an operation for the complex amplitude image based on a phase conjugate method, and is an operation of calculating a transmission matrix representing the relationship between the light irradiation and the light output for the object, and including an inverse matrix calculation thereof and coordinate conversion. The phase conjugate method may be referred to as a phase conjugation, a time reversal method, a time reversal, a digital phase conjugation, a digital phase conjugate method, or the like. The details will be described later.

In the two-dimensional phase image generation step S65, the two-dimensional phase image generation unit 65 generates, for each of the plurality of positions, a two-dimensional phase image based on the complex amplitude image of each of the plurality of light irradiation directions generated by the second complex amplitude image generation unit 63 or the phase conjugate operation unit 64. The two-dimensional phase image generated in this step corresponds to a phase image centered on the focused z direction position.

In the two-dimensional phase image generation step S65, when a phase image generated based on the complex amplitude image before performing the processing step of the phase conjugate operation step S64 is set as a first phase image, and a phase image generated based on the complex amplitude image obtained by performing the processing step of the phase conjugate operation step S64 is set as a second phase image, for the plurality of positions, the two-dimensional phase image is generated mainly based on the first phase image at a position relatively close to the imaging unit, and the two-dimensional phase image is generated mainly based on the second phase image at a position relatively far from the imaging unit.

In addition, the phase conjugate operation unit 64 and the subsequent processing steps may be performed after all the complex amplitude images at the plurality of positions are generated for each of the plurality of light irradiation directions in the second complex amplitude image generation step S63. Further, processes of generating the complex amplitude image at one certain z direction position for each of the plurality of light irradiation directions in the second complex amplitude image generation step S63 and generating the two-dimensional phase image at the position in the two-dimensional phase image generation step S65 may be set as a unit, and the unit process may be repeatedly performed while scanning the z direction position. The latter case is preferable in that a capacity of image data to be stored in the storage unit 69 can be reduced.

In the three-dimensional phase image generation step S66, the three-dimensional phase image generation unit 66 generates a three-dimensional phase image based on the two-dimensional phase image at each of the plurality of positions generated by the two-dimensional phase image generation unit 65. The three-dimensional phase image generated in this step is an image in which the positions x and y in the two-dimensional phase image and the position z of the two-dimensional phase image are variables.

In the refractive index distribution calculation step S67, the refractive index distribution calculation unit 67 obtains a three-dimensional refractive index distribution of the observation object by deconvolution based on the three-dimensional phase image generated by the three-dimensional phase image generation unit 66.

The two-dimensional phase image generation step S65, the three-dimensional phase image generation step S66, and the refractive index distribution calculation step S67 in the refractive index distribution measuring method B respectively perform the same processing steps as the two-dimensional phase image generation step S4, the three-dimensional phase image generation step S5, and the refractive index distribution calculation step S6 in the refractive index distribution measuring method A.

FIG. B05 is a diagram illustrating the images and the order of the respective processing steps of the second complex amplitude image generation step S63 and the two-dimensional phase image generation step S65. This diagram illustrates a configuration in which the processing step of the phase conjugate operation step S64 is not performed. In this configuration, in the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

Each of FIG. B06 to FIG. B08 is a diagram illustrating the images and the order of the respective processing steps of the second complex amplitude image generation step S63, the phase conjugate operation step S64, and the two-dimensional phase image generation step S65. Each of these diagrams illustrates a configuration in which the processing step of the phase conjugate operation step S64 is performed before, during, or after the processing step of the second complex amplitude image generation step S63.

A first configuration illustrated in FIG. B06 corresponds to the flowchart illustrated in FIG. B04. In the first configuration, the phase conjugate operation step S64 is performed after the processing step of the second complex amplitude image generation step S63. In the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation.

In the first configuration, subsequently, in the phase conjugate operation step S64, for each of the plurality of positions, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated. Further, in the two-dimensional phase image generation step S65, for each of the plurality of positions, the complex differential interference image is generated based on the complex amplitude image of each of the plurality of light irradiation directions generated in the phase conjugate operation step S64, and in addition, the phase differential image is generated.

In a second configuration illustrated in FIG. B07, the phase conjugate operation step S64 is performed before the processing step of the second complex amplitude image generation step S63. In the phase conjugate operation step S64, for each of the plurality of light irradiation directions, the phase conjugate operation is performed on the complex amplitude image at the reference position ($z = 0$) generated in the first complex amplitude image generation step S62, and the complex amplitude image of each of the plurality of light irradiation directions in the case in which the relationship between the light irradiation and the imaging for the observation object is reversed is generated.

In the second configuration, subsequently, in the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In a third configuration illustrated in FIG. B08, in the case in which the second complex amplitude image generation step S63 generates the complex amplitude image at each of the plurality of positions from the complex amplitude image at the reference position through two stages, the phase conjugate operation step S64 is performed between a first stage and a second stage in the two stages.

In the third configuration, in the first stage of the second complex amplitude image generation step S63, for each of the plurality of light irradiation directions, the complex amplitude image at each of the plurality of z direction positions ($z = z_1, z_3, z_5$ in this diagram) is generated based on the complex amplitude image at the reference position ($z = 0$) generated in the first complex amplitude image generation step S62 by the above Formulas (1) and (2) of the formulas of the free propagation. Subsequently, in the phase conjugate operation step S64, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated.

In the third configuration, further subsequently, in the second stage of the second complex amplitude image generation step S63, for each of the plurality of light irradiation directions, the complex amplitude image at each of the z direction positions ($z = z_2, z_4, z_6$) is generated based on the complex amplitude images at the z direction positions ($z = z_1, z_3, z_5$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In the first configuration, the second configuration, and the third configuration described above, the number of times of the phase conjugate operation on the complex amplitude image in the phase conjugate operation step S64 is different. The overall processing time of the phase conjugate operation step S64 is shorter in the third configuration than in the first configuration, and is even shorter in the second configuration.

FIG. B09 is a diagram illustrating the images and the order of the respective processing steps of the three-dimensional phase image generation step S66 and the refractive index distribution calculation step S67. In the three-dimensional phase image generation step S66, the three-dimensional phase image is generated based on the two-dimensional phase image of each of the plurality of positions generated in the two-dimensional phase image generation step S65. In this case, for the position which is relatively close to the imaging unit, the two-dimensional phase image generated based on the complex amplitude image before performing the processing step of the phase conjugate operation step S64 (the two-dimensional phase image generated in the configuration illustrated in FIG. B05) is mainly used. On the other hand, for the position which is relatively far from the imaging unit, the two-dimensional phase image generated based on the complex amplitude image after performing the processing step of the phase conjugate operation step S64 (the two-dimensional phase image generated in any one of the configurations illustrated in FIG. B06 to FIG. B08) is mainly used. Subsequently, in the refractive index distribution calculation step S67, the three-dimensional refractive index distribution of the observation object is obtained by deconvolution based on the three-dimensional phase image generated in the three-dimensional phase image generation step S66. Each refractive index distribution data constituting the three-dimensional refractive index distribution (for example, a two-dimensional refractive index distribution data constituting the three-dimensional refractive index distribution in FIG. B09) can be a refractive index cross sectional data.

The generation of the two-dimensional phase image at each position in the z direction includes the following three configurations. The phase image generated based on the complex amplitude image before performing the processing step of the phase conjugate operation step S64 (the phase image generated in the configuration illustrated in FIG. B05) is set as the first phase image $\phi_1$. The phase image generated based on the complex amplitude image after performing the processing step of the phase conjugate operation step S64 (the phase image generated in any one of the configurations illustrated in FIG. B06 to FIG. B08) is set as the second phase image $\phi_2$. A weight function $\alpha$ having a differential coefficient of 0 or less with respect to the variable z representing the distance from the imaging unit along the light propagation path is used. The value of the weight function is 0 or more and 1 or less.

In the first configuration, it is assumed that the weight function $\alpha$ has a positive value (for example, 1) in a range in which z is threshold value $z_{th}$ or less, and has a value of 0 in a range other than the above range. That is, the two-dimensional phase image is represented by the following Formula (8).
[Formula 8]

$$\phi(x, y, z) = \begin{cases} \phi_1(x, y, z) & z \leq z_{th} \\ \phi_2(x, y, z) & z > z_{th} \end{cases} \qquad (8)$$

In the second configuration, it is assumed that the weight function $\alpha$ is a function having a value which continuously changes in at least a partial range in the z direction. That is, the two-dimensional phase image is represented by the following Formula (9).
[Formula 9]

$$\phi(x, y, z) = \alpha(z) \cdot \phi_1(x, y, z) + [1 - \alpha(z)] \phi_2(x, y, z) \qquad (9)$$

In the third configuration, it is assumed that the weight function $\alpha$ has a value according to the position (x, y) on the plane perpendicular to the optical axis (the z direction). That is, the two-dimensional phase image is represented by the following Formula (10).
[Formula 10]

$$\phi(x,y,z) = \alpha(x,y,z) \cdot \phi_1(x,y,z) + \left[1 - \alpha(x,y,z)\right]\phi_2(x,y,z) \qquad (10)$$

Next, the contents of the phase conjugate operation by the phase conjugate operation step S64 will be described with reference to FIG. B10 and FIG. B11.

FIG. B10 is a diagram illustrating input light $U_{in}(k_{in})$ and output light $u_{out}(r_{out})$ when the interference intensity image is imaged by the imaging unit. $U_{in}(k_{in})$ represents a complex amplitude of a wavenumber $k_{in}$ of the light with which the observation object is irradiated. $u_{out}(r_{out})$ represents a complex amplitude of a position $r_{out}$ of the light output from the observation object. The relationship between $U_{in}(k_{in})$ and $u_{out}(r_{out})$ is represented by the following Formula (11). An n-th element $U_{in}(k_{in}^n)$ of a column vector $U_{in}$ represents a complex amplitude of a plane wave of a wavenumber of $k_{in}^n$. An n-th element $u_{out}(r_{out}^n)$ of a column vector $u_{out}$ represents a complex amplitude of the light observed at a position $r_{out}^n$. A matrix $T(r_{out}, k_{in})$ of N rows and N columns represents a linear relationship between $U_{in}(k_{in})$ and $u_{out}(r_{out})$, and is referred to as a transmission matrix. A scattering process of the light in the observation object can be represented by the transmission matrix described above. An element $T_{n1,n2}$ of an n1-th row and an n2-th column of the matrix $T(r_{out}, k_{in})$ represent a complex amplitude of the light observed at a position $r_{out}^{n1}$ when the plane wave having a wavenumber of $k_{in}^{n2}$ and an amplitude of 1 is input.

[Formula 11]

$$
\begin{pmatrix} u_{out}(\mathbf{r}_{out}^1) \\ \vdots \\ u_{out}(\mathbf{r}_{out}^N) \end{pmatrix} = \begin{pmatrix} T(\mathbf{r}_{out}^1, \mathbf{k}_{in}^1) & \cdots & T(\mathbf{r}_{out}^1, \mathbf{k}_{in}^N) \\ \vdots & \ddots & \vdots \\ T(\mathbf{r}_{out}^N, \mathbf{k}_{in}^1) & \cdots & T(\mathbf{r}_{out}^N, \mathbf{k}_{in}^N) \end{pmatrix} \begin{pmatrix} U_{in}(\mathbf{k}_{in}^1) \\ \vdots \\ U_{in}(\mathbf{k}_{in}^N) \end{pmatrix}
$$
$$
= \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^1) \\ \vdots \\ U_{in}(\mathbf{k}_{in}^N) \end{pmatrix} \qquad (11)
$$

FIG. B11 is a diagram illustrating input light $U_{out}(k_{out})$ and output light $u_{in}(r_{in})$ in the case in which the relationship between the light irradiation and the imaging is reversed. In this case, $U_{out}(k_{out})$ represents a complex amplitude of a wavenumber $k_{out}$ of the light with which the observation object is irradiated. $u_{in}(r_{in})$ represents a complex amplitude of a position $r_{in}$ of the light output from the observation object. The relationship between $U_{out}(k_{out})$ and $u_{in}(r_{in})$ is represented by the following Formula (12). An n-th element $U_{out}(k_{out}^n)$ of a column vector $U_{out}$ represents a complex amplitude of a plane wave of a wavenumber of $k_{out}^n$. An n-th element $u_{in}(r_{in}^n)$ of a column vector $u_{in}$ represents a complex amplitude of the light observed at a position $r_{in}^n$. A matrix $S(r_{in}, k_{out})$ of N rows and N columns represents a linear relationship between $U_{out}(k_{out})$ and $u_{in}(r_{in})$, and is a transmission matrix in the case in which the relationship between the light irradiation and the imaging is reversed.

[Formula 12]

$$
\begin{pmatrix} u_{in}(\mathbf{r}_{in}^1) \\ \vdots \\ u_{in}(\mathbf{r}_{in}^N) \end{pmatrix} = \begin{pmatrix} S(\mathbf{r}_{in}^1, \mathbf{k}_{out}^1) & \cdots & S(\mathbf{r}_{in}^1, \mathbf{k}_{out}^N) \\ \vdots & \ddots & \vdots \\ S(\mathbf{r}_{in}^N, \mathbf{k}_{out}^1) & \cdots & S(\mathbf{r}_{in}^N, \mathbf{k}_{out}^N) \end{pmatrix} \begin{pmatrix} U_{out}(\mathbf{k}_{out}^1) \\ \vdots \\ U_{out}(\mathbf{k}_{out}^N) \end{pmatrix}
$$
$$
= \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix} \qquad (12)
$$

$U_{in}(k_{in})$ is represented by the Fourier transform of $u_{in}(r_{in})$ as shown in the following Formula (13). $U_{out}(k_{out})$ is represented by the Fourier transform of $u_{out}(r_{out})$ as shown in the following Formula (14). When Formulas (11) to (14) are used, the transmission matrix $S(r_{in}, k_{out})$ in the case in which the relationship between the light irradiation and the imaging is reversed is represented by the following Formula (15) by using a matrix representing the inverse Fourier transform and the transmission matrix $T(r_{out}, k_{in})$.

[Formula 13]

$$\begin{pmatrix} U_{in}(\mathbf{k}_{in}^1) \\ \vdots \\ U_{in}(\mathbf{k}_{in}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{in}(\mathbf{r}_{in}^1) \\ \vdots \\ u_{in}(\mathbf{r}_{in}^N) \end{pmatrix} \qquad (1\,3)$$

[Formula 14]

$$\begin{pmatrix} U_{out}(\mathbf{k}_{out}^1) \\ \vdots \\ U_{out}(\mathbf{k}_{out}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{out}(\mathbf{r}_{out}^1) \\ \vdots \\ u_{out}(\mathbf{r}_{out}^N) \end{pmatrix} \qquad (1\,4)$$

[Formula 15]

$$\begin{pmatrix} S_{1,1} & \cdots & S_{1,N} \\ \vdots & \ddots & \vdots \\ S_{N,1} & \cdots & S_{N,N} \end{pmatrix} = \begin{pmatrix} F_{1,1} & \cdots & F_{1,N} \\ \vdots & \ddots & \vdots \\ F_{N,1} & \cdots & F_{N,N} \end{pmatrix}^{-1} \begin{pmatrix} T_{1,1} & \cdots & T_{1,N} \\ \vdots & \ddots & \vdots \\ T_{N,1} & \cdots & T_{N,N} \end{pmatrix}^{-1} \begin{pmatrix} F_{1,1} & \cdots & F_{1,N} \\ \vdots & \ddots & \vdots \\ F_{N,1} & \cdots & F_{N,N} \end{pmatrix}^{-1} \qquad (1\,5)$$

In the phase conjugate operation step S64, first, the transmission matrix $T(r_{out}, k_{in})$ when the interference intensity image is imaged by the imaging unit is obtained based on the complex amplitude image. Next, based on the above transmission matrix $T(r_{out}, k_{in})$ and the above Formula (15), the transmission matrix $S(r_{in}, k_{out})$ in the case in which the relationship between the light irradiation and the imaging is reversed is obtained. Further, based on the above transmission matrix $S(r_{in}, k_{out})$, the complex amplitude image in the case in which the relationship between the light irradiation and the imaging is reversed is obtained.

The vector $U_{in}^n(k_{in})$ of the input light of the n-th light irradiation direction when the interference intensity image is imaged by the imaging unit for each of the plurality of light irradiation directions is represented by the following Formula (16), in which only the value of the n-th element is 1 and the values of the other elements are 0. For the above input light $U_{in}^n(k_{in})$, the output light $u_{out}^n(r_{out})$ is represented by the following Formula (17). The Formula (17) corresponds to the complex amplitude obtained for the n-th light irradiation direction.

[Formula 16]

$$U_{in}^n(\mathbf{k}_{in}) = \begin{pmatrix} U_{in}^n(\mathbf{k}_{in}^1) \\ \vdots \\ U_{in}^n(\mathbf{k}_{in}^N) \end{pmatrix} = \begin{pmatrix} 0 \\ \vdots \\ 0 \\ 1 \\ 0 \\ \vdots \\ 0 \end{pmatrix} \qquad (1\,6)$$

[Formula 17]

$$u_{out}^n(\mathbf{r}_{out}) = \begin{pmatrix} u_{out}^n(\mathbf{r}_{out}^1) \\ \vdots \\ u_{out}^n(\mathbf{r}_{out}^N) \end{pmatrix} \qquad (1\,7)$$

From the Formula (16) and the above Formula (11), the following Formula (18) is obtained. Further, the following Formula (19) is obtained by similarly obtaining for each of the plurality of light irradiation directions. In this way, the

transmission matrix $T(r_{out}, k_{in})$ can be obtained. In addition, from the Formula (19) and the above Formula (15), the transmission matrix $S(r_{in}, k_{out})$ in the case in which the relationship between the light irradiation and the imaging is reversed can be obtained.

[Formula 18]

$$\begin{pmatrix} u_{out}^n(\mathbf{r}_{out}^1) \\ \vdots \\ u_{out}^n(\mathbf{r}_{out}^N) \end{pmatrix} = \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^1) \\ \vdots \\ U_{in}(\mathbf{k}_{in}^N) \end{pmatrix} = \begin{pmatrix} T_{1,n} \\ \vdots \\ T_{N,n} \end{pmatrix} \qquad (18)$$

[Formula 19]

$$\begin{pmatrix} T_{1,1} & \cdots & T_{1,N} \\ \vdots & \ddots & \vdots \\ T_{N,1} & \cdots & T_{N,N} \end{pmatrix} = \begin{pmatrix} u_{out}^1(\mathbf{r}_{out}^1) & \cdots & u_{out}^N(\mathbf{r}_{out}^1) \\ \vdots & \ddots & \vdots \\ u_{out}^1(\mathbf{r}_{out}^N) & \cdots & u_{out}^N(\mathbf{r}_{out}^N) \end{pmatrix} \qquad (19)$$

The input light $U_{out}^n(k_{out})$ of the n-th light irradiation direction out of the plurality of light irradiation directions in the case in which the relationship between the light irradiation and the imaging is reversed is represented by the following Formula (20), in which only the value of the n-th element is 1 and the values of the other elements are 0. From this Formula, the output light $u_{in}^n(r_{in})$ for the input light $U_{out}^n(k_{out})$ is represented by the following Formula (21). The Formula (21) represents the complex amplitude when the relationship between the light irradiation and the imaging is reversed. In this way, the complex amplitude image in the case in which the relationship between the light irradiation and the imaging is reversed can be obtained.

[Formula 20]

$$U_{out}^n(\mathbf{k}_{out}) = \begin{pmatrix} U_{out}^n(\mathbf{k}_{out}^1) \\ \vdots \\ U_{out}^n(\mathbf{k}_{out}^N) \end{pmatrix} = \begin{pmatrix} 0 \\ \vdots \\ 0 \\ 1 \\ 0 \\ \vdots \\ 0 \end{pmatrix} \qquad (20)$$

[Formula 21]

$$\begin{pmatrix} u_{in}^n(\mathbf{r}_{in}^1) \\ \vdots \\ u_{in}^n(\mathbf{r}_{in}^N) \end{pmatrix} = \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix} = \begin{pmatrix} S_{1,n} \\ \vdots \\ S_{N,n} \end{pmatrix} \qquad (21)$$

When the transmission matrix $S(r_{in}, k_{out})$ in the case in which the relationship between the light irradiation and the imaging is reversed is obtained, it is necessary to calculate the inverse matrix of the transmission matrix $T(r_{out}, k_{in})$ as shown in the above Formula (15). Therefore, the transmission matrix T needs to be a square matrix in which the number of row elements and the number of column elements are equal to each other. That is, a matrix dimension in a light irradiation side wavenumber space for the observation object in the interference intensity image acquisition step S61 and the number of pixels of the complex amplitude image need to be equal to each other.

In order to make them equal to each other, the matrix dimension in the light irradiation side wavenumber space for the observation object in the interference intensity image acquisition step S61 may be made equal to the number of pixels, or only a partial range of the image acquired by the imaging unit may be used in the subsequent processing steps. However, in general, the number of pixels of the image acquired by the imaging unit is, for example, 1024 × 1024, and thus, it is not easy to make the matrix dimension in the light irradiation side wavenumber space for the observation object equal to the number of pixels. Further, it is not preferable to use only the partial range of the image out of the image acquired by the imaging unit in the subsequent processing steps because this leads to a decrease in resolution.

Therefore, as illustrated in FIG. B12, in the phase conjugate operation step S64, it is preferable to divide the complex amplitude image into a plurality of partial images each having the same number of pixels as the matrix dimension in the light irradiation side wavenumber space for the observation object, perform the phase conjugate operation on each of the plurality of partial images, and then combine the plurality of partial images. In this case, any two or more partial images out of the plurality of partial images may have a common region.

Next, the refractive index distribution measuring method C will be described. FIG. C01 to C03 are diagrams showing respective configurations of observation apparatuses 1G to 1I that can be used when measuring the refractive index distribution by the refractive index distribution measuring method C. The observation apparatus 1G illustrated in FIG. C01, as compared with the configuration of the observation apparatus 1A illustrated in FIG. A01, has the common configuration for the optical system from the light source 11 to the imaging unit 43, and further, is different in that an analysis unit 70 is provided instead of the analysis unit 50. The observation apparatus 1H illustrated in FIG. C02, as compared with the configuration of the observation apparatus 1B illustrated in FIG. A02, has the common configuration for the optical system from the light source 11 to the imaging unit 43, and further, is different in that the analysis unit 70 is provided instead of the analysis unit 50. The observation apparatus 1I illustrated in FIG. C03, as compared with the configuration of the observation apparatus 1C illustrated in FIG. A03, has the common configuration for the optical system from the light source 11 to the imaging unit 43, and further, is different in that the analysis unit 70 is provided instead of the analysis unit 50.

The analysis unit 70 is electrically connected to the imaging unit 43, and inputs the interference intensity image output from the imaging unit 43. The analysis unit 70 calculates a three-dimensional refractive index distribution of the observation object S by processing the input interference intensity image. The analysis unit 70 may be a computer. The analysis unit 70 includes an interference intensity image acquisition unit 71, a first complex amplitude image generation unit 72, a second complex amplitude image generation unit 73, a two-dimensional phase image generation unit 74, a three-dimensional phase image generation unit 75, a refractive index distribution calculation unit 76, a third complex amplitude image generation unit 77, a display unit 78, and a storage unit 79.

The interference intensity image acquisition unit 71 irradiates the observation object S with the light along each of a plurality of light irradiation directions by changing the orientation of the reflection surface of the mirror 22. Further, the interference intensity image acquisition unit 71 acquires the interference intensity image at a reference position for each of the plurality of light irradiation directions from the imaging unit 43. The interference intensity image acquisition unit 71 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 perform processing based on the interference intensity images, and include a processing device such as a CPU, a GPU, a DSP, or an FPGA. The display unit 78 displays an image to be processed, an image in the middle of the processing, an image after the processing, and the like, and includes, for example, a liquid crystal display. The storage unit 79 stores data of various images, and includes a hard disk drive, a flash memory, a RAM, a ROM, and the like. The first complex amplitude image generation unit 72, the second complex amplitude image generation unit 73, the two-dimensional phase image generation unit 74, the three-dimensional phase image generation unit 75, the refractive index distribution calculation unit 76, the third complex amplitude image generation unit 77, and the storage unit 79 may be constituted by a cloud computing.

The storage unit 79 also stores a program for causing the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 to execute respective steps of the processing. The program may be stored in the storage unit 79 at the time of manufacture or shipment of the observation apparatus 1G to 1I, may be acquired via a communication line after shipment and then stored in the storage unit 79, or may be recorded in a computer readable recording medium 2 and then stored in the storage unit 79. The recording medium 2 may be an arbitrary medium such as a flexible disk, a CD-ROM, a DVD-ROM, a BD-ROM, a USB memory, or the like.

The details of the processing step of each of the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 will be described later.

FIG. C04 and FIG. C05 are flowcharts of the refractive index distribution measuring method C. FIG. C05 illustrates a part of the flowchart illustrated in FIG. C04. The refractive index distribution measuring method C can be applied to each of the observation apparatus 1G to 1I. The observation method includes an interference intensity image acquisition step S71, a first complex amplitude image generation step S72, a second complex amplitude image generation step S73, a two-dimensional phase image generation step S74, a three-dimensional phase image generation step S75, a refractive index distribution calculation step S76, and a third complex amplitude image generation step S77.

The processing step of the interference intensity image acquisition step S71 is performed by the interference intensity image acquisition unit 71. The processing step of the first complex amplitude image generation step S72 is performed by the first complex amplitude image generation unit 72. The processing step of the second complex amplitude image generation step S73 is performed by the second complex amplitude image generation unit 73. The processing step of the two-dimensional phase image generation step S74 is performed by the two-dimensional phase image generation unit 74. The processing step of the three-dimensional phase image generation step S75 is performed by the three-dimensional phase image generation unit 75. The processing step of the refractive index distribution calculation step S76 is performed by the refractive index distribution calculation unit 76. The processing step of the third complex amplitude image generation step S77 is performed by the third complex amplitude image generation unit 77.

In the interference intensity image acquisition step S71, the interference intensity image acquisition unit 71 irradiates the observation object S with the light along each of the plurality of light irradiation directions by changing the orientation of the reflection surface of the mirror 22. Further, the interference intensity image acquisition unit 71 acquires the interference intensity image at the reference position for each of the plurality of light irradiation directions from the imaging unit 43.

In the first complex amplitude image generation step S72, the first complex amplitude image generation unit 72 generates, for each of the plurality of light irradiation directions, a complex amplitude image based on the interference intensity image acquired by the interference intensity image acquisition unit 71. In the case of the observation apparatus 1G (FIG. C01) or the observation apparatus 1I (FIG. C03), the first complex amplitude image generation unit 72 can generate the complex amplitude image based on one interference intensity image by a Fourier fringe analysis method. In the case of the observation apparatus 1H (FIG. C02), the first complex amplitude image generation unit 72 can generate the complex amplitude image based on three or more interference intensity images having different optical path differences (phase differences) between the object light and the reference light by a phase shift method. The complex amplitude image generated in the first complex amplitude image generation step S72 may be at the same reference position as the interference intensity image or may be at another position generated based on the complex amplitude image at the reference position.

In the second complex amplitude image generation step S73, the second complex amplitude image generation unit 73 generates, for each of the plurality of light irradiation directions, a complex amplitude image at each of a plurality of z direction positions between a first position and a second position based on the complex amplitude image at the first position with respect to a distance from the imaging unit 43 along a light propagation path.

In the two-dimensional phase image generation step S74, the two-dimensional phase image generation unit 74 generates, for each of the plurality of positions, a two-dimensional phase image based on the complex amplitude image of each of the plurality of light irradiation directions generated by the second complex amplitude image generation unit 73. The two-dimensional phase image generated in this step corresponds to a phase image centered on the focused z direction position.

In the three-dimensional phase image generation step S75, the three-dimensional phase image generation unit 75 generates a three-dimensional phase image between the first position and the second position based on the two-dimensional phase image at each of the plurality of positions generated by the two-dimensional phase image generation unit 74. The three-dimensional phase image generated in this step is an image in which the positions x and y in the two-dimensional phase image and the position z of the two-dimensional phase image are variables.

In the refractive index distribution calculation step S76, the refractive index distribution calculation unit 76 obtains a three-dimensional refractive index distribution of the observation object between the first position and the second position by deconvolution based on the three-dimensional phase image generated by the three-dimensional phase image generation unit 75.

The interference intensity image acquisition step S71, the first complex amplitude image generation step S72, the second complex amplitude image generation step S73, the two-dimensional phase image generation step S74, the three-dimensional phase image generation step S75, and the refractive index distribution calculation step S76 in the refractive index distribution measuring method C respectively perform substantially the same processing steps as the interference intensity image acquisition step S1, the first complex amplitude image generation step S2, the second complex amplitude image generation step S3, the two-dimensional phase image generation step S4, the three-dimensional phase image generation step S5, and the refractive index distribution calculation step S6 in the refractive index distribution measuring method A.

In the third complex amplitude image generation step S77, the third complex amplitude image generation unit 77 generates, for each of the plurality of light irradiation directions, a complex amplitude image at the second position based on the complex amplitude image at the first position used in the second complex amplitude image generation step S73 and the three-dimensional refractive index distribution of the observation object between the first position and the second position calculated in the refractive index distribution calculation step S76.

In the step S83 including the second complex amplitude image generation step S73, the two-dimensional phase image generation step S74, the three-dimensional phase image generation step S75, and the refractive index distribution calculation step S76, the three-dimensional refractive index distribution of the observation object between the first position and the second position is obtained based on the complex amplitude image at the first position with respect to the distance from the imaging unit 43 along the light propagation path. The processing steps of the step S83 and the third complex amplitude image generation step S77 are repeatedly performed. This will be described with reference to FIG. C04 to FIG. C07.

FIG. C06 is a diagram illustrating a relationship between a region including the observation object and first to J-th blocks. As illustrated in this diagram, the region including the observation object is divided into the first to J-th blocks in order based on the distance from the imaging unit along the light propagation path (z direction). In this diagram, it is set to J = 3. The j-th block in the first to J-th blocks is a region from $z = z_{j-1}$ to $z = z_j$. In each j-th block, a position (near end) of $z = z_{j-1}$ closest to the imaging unit is set as the first position, and a position (far end) of $z = z_j$ farthest from the imaging unit is set as the second position.

FIG. C07 is a diagram illustrating a processing procedure for the first to J-th blocks. As illustrated in this diagram, for each j-th block, in the step S83, the complex amplitude image and the two-dimensional phase image at each of the plurality of z direction positions from the first position to the second position are generated based on the complex amplitude image at the first position, the three-dimensional phase image between the first position and the second position is generated, and the three-dimensional refractive index distribution is obtained. For each j-th block, in the third complex amplitude image generation step S77, the complex amplitude image at the second position is generated based on the complex amplitude image at the first position and the three-dimensional refractive index distribution calculated in the step S83.

The complex amplitude image at the second position in the (j-1)-th block generated in the third complex amplitude image generation step S77 is used as the complex amplitude image at the first position in the next j-th block, and the processing steps of the step S83 and the third complex amplitude image generation step S77 are performed for the j-th block. When the three-dimensional refractive index distribution is obtained for each of the first to J-th blocks, the three-dimensional refractive index distribution of the entire observation object is obtained by combining these distributions. The three-dimensional refractive index distribution of each of the first to J blocks (for example, the refractive index distribution of the first block, the refractive index distribution of the second block, and the refractive index distribution of the third block in FIG. C07) can be a refractive index cross sectional data.

As illustrated in FIG. C04 and FIG. C05, in the step S81 after the first complex amplitude image generation step S72, it is set to j = 0, and in the subsequent step S82, the value of j is increased as j = 1, and the processing steps of the step S83 and the third complex amplitude image generation step S77 are performed for the first block. That is, for the first block closest to the imaging unit, based on the complex amplitude image generated in the first complex amplitude image generation step S72, a position of $z = z_0$ (near end) closest to the imaging unit is set as the first position, a position of $z = z_1$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the j-th block (in this case, j is 2 or more and less than J), based on the complex amplitude image generated for the (j-1)-th block in the third complex amplitude image generation step S77, a position of $z = z_{j-1}$ (near end) closest to the imaging unit is set as the first position, a position of $z = z_j$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the J-th block which is the last block farthest from the imaging unit, based on the complex amplitude image generated for the (J-1)-th block in the third complex amplitude image generation step S77, a position of $z = z_{J-1}$ (near end) closest to the imaging unit is set as the first position, a position of $z = z_J$ (far end) farthest from the imaging unit is set as the second position, and the processing step of the step S83 (the second complex amplitude image generation step S73, the two-dimensional phase image generation step S74, the three-dimensional phase image generation step S75, and the refractive index distribution calculation step S76) is performed.

For the J-th block, it is determined to be the last block in the step S84 after the step S83, and may be ended without proceeding to the third complex amplitude image generation step S77. In addition, for the J-th block, it may be determined to be the last block after the three-dimensional phase image generation step S75, and may be ended without proceeding to the refractive index distribution calculation step S76, and in this case, the three-dimensional phase image of the entire observation object is obtained.

In addition, the region including the observation object may be divided into the two blocks in order based on the distance from the imaging unit along the light propagation path (z direction), and in this case, the processing for the first block and the processing for the last J-th block described above may be performed. Further, the region including the observation object may not be divided into the plurality of blocks, and in this case, the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 may be sequentially performed only once.

Next, the details of the third complex amplitude image generation step S77 will be described. When acquiring the interference intensity image by irradiating the observation object with the light, in the j-th block, a light wavefront at the second position ($z = z_j$) propagates inside the j-th block to reach the first position ($z = z_{j-1}$) and further propagates to the imaging unit. Therefore, in the third complex amplitude image generation step S77, the light wavefront at the first position ($z = z_{j-1}$) is reversely propagated inside the j-th block by numerical calculation in consideration of the refractive index distribution of the j-th block, thereby obtaining the light wavefront at the second position ($z = z_j$). That is, in the third complex amplitude image generation step S77, for each of the plurality of light irradiation directions, the complex amplitude image at the second position ($z = z_j$) of the j-th block is generated based on the complex amplitude image at the first position ($z = z_{j-1}$) of the j-th block and the refractive index distribution of the j-th block. In the above processing, a method of numerically calculating the propagation of the light wavefront in consideration of the refractive index distribution of the medium is used. A beam propagation method (BPM), a split-step non-paraxial (SSNP), and the like are known as the numerical calculation method of the inhomogeneous medium propagation described above. Hereinafter, the processing using the BPM in the third complex amplitude image generation step S77 will be described.

FIG. C08 is a diagram illustrating processing contents of the BPM. This diagram illustrates an arbitrary j-th block. As illustrated in this diagram, the j-th block is divided into M slices (7 slices in this diagram) (first to M-th slices) based on the distance from the imaging unit along the light propagation path (z direction). A thickness of each slice is about a wavelength.

The thickness of each slice may be constant. In this case, it is assumed that the thickness of each slice is a constant value of $\Delta z$. The m-th slice out of the first to M-th slices of the j-th block is from a position ($z_{j-1} + (m-1)\Delta z$) to a position ($z_{j-1} + m\Delta z$). In order from the first position ($z = z_{j-1}$) of the j-th block to the second position ($z = z_j$), a phase change according to the refractive index distribution is sequentially applied in each of the first to M-th slices, and the light wavefront is reversely propagated by $\Delta z$.

In addition, the thickness $\Delta z$ of each slice in the processing of the third complex amplitude image generation step S77 may be different from or may coincide with the position interval when generating the complex amplitude image of each of the plurality of z direction positions from the first position to the second position in the processing of the second complex amplitude image generation step S73.

The phase change o(x, y, z) applied to the light wavefront when reversely propagating the slice of the thickness $\Delta z$ at the position z is represented by the following Formula (22). In the Formula (22), $k_v$ is a wavenumber of the light in vacuum. $\delta n(x, y, z)$ is a difference between the refractive index distribution n(x, y, z) of the observation object at the position z and the refractive index $n_b$ of the background (medium), and is represented by the following Formula (23). Further, $\cos\theta$ is represented by the following Formula (24).

[Formula 22]

$$o\left(x, y, z\right) = \exp\left(-ik_v \delta n\left(x, y, z\right)\frac{\Delta z}{\cos\theta}\right) \qquad (22)$$

[Formula 23]

$$\delta n\left(x, y, z\right) = n\left(x, y, z\right) - n_b \qquad (23)$$

[Formula 24]

$$\cos\theta = \frac{\sqrt{n_b^2 k_v^2 - k_x^2 - k_y^2}}{n_b k_v} \qquad (24)$$

Assuming that the complex amplitude of the light at the position ($z = z_{j-1} + (m-1)\Delta z$) of the m-th slice is u(x, y, z), the complex amplitude u(x, y, z + Δz) of the light at the position (z + Δz) after the light reversely propagates inside the m-th slice is represented by the following Formula (25). In the Formula (25), P($k_x$, $k_y$; Δz) is represented by the following Formula (26). The Formula (25) indicates that the complex amplitude u(x, y, z + Δz) of the light at the position (z + Δz) after propagating the slice of the thickness Δz is obtained by performing Fourier transform on a product of the complex amplitude u(x, y, z) of the light and the phase change o(x, y, z), and performing inverse Fourier transform on a product of a result of the above Fourier transform and P($k_x$, $k_y$; Δz). $P_{Δz}$ is a function for performing calculation of the light propagation of Δz.
[Formula 25]

$$u\left(x, y, z + \Delta z\right) = P_{\Delta z}\left[o\left(x, y, z\right) \cdot u\left(x, y, z\right)\right]$$
$$\equiv F^{-1}\left[P\left(k_x, k_y; \Delta z\right) \cdot F\left[o\left(x, y, z\right) \cdot u\left(x, y, z\right)\right]\right] \quad (25)$$

[Formula 26]

$$P\left(k_x, k_y; \Delta z\right) = \exp\left(-i\sqrt{n_b^2 k_v^2 - k_x^2 - k_y^2}\,\Delta z\right) \quad (26)$$

The propagation of the light wavefront in each slice of the j-th block is represented by the following Formulas (27) to (29). That is, when the complex amplitude of the light at the first position (z = $z_{j-1}$) of the j-th block is set to u(x, y, $z_{j-1}$), the complex amplitude u(x, y, $z_{j-1}$ + Δz) of the light after propagating the first slice of the j-th block is represented by the following Formula (27). When the complex amplitude of the light after propagating the (m-1)-th slice of the j-th block is set to u(x, y, $z_{j-1}$ + (m-1)Δz), the complex amplitude u(x, y, $z_{j-1}$ + mΔz) of the light after propagating the m-th slice of the j-th block is represented by the following Formula (28). When the complex amplitude of the light after propagating the (M-1)-th slice of the j-th block is set to u(x, y, $z_{j-1}$ + (M-1)Δz), the complex amplitude u(x, y, $z_j$) of the light at the second position (z = $z_j$) after propagating the M-th slice of the j-th block is represented by the following Formula (29).
[Formula 27]

$$u\left(x, y, z_{j-1} + \Delta z\right) = P_{\Delta z}\left[o\left(x, y, z_{j-1}\right) \cdot u\left(x, y, z_{j-1}\right)\right] \quad (27)$$

[Formula 28]

$$u\left(x, y, z_{j-1} + m\Delta z\right) = P_{\Delta z}\left[o\left(x, y, z_{j-1} + (m-1)\Delta z\right) \cdot u\left(x, y, z_{j-1} + (m-1)\Delta z\right)\right] \quad (28)$$

[Formula 29]

$$u\left(x, y, z_j\right) = P_{\Delta z}\left[o\left(x, y, z_{j-1} + (M-1)\Delta z\right) \cdot u\left(x, y, z_{j-1} + (M-1)\Delta z\right)\right] \quad (29)$$

As described above, in the third complex amplitude image generation step S77, the light wavefront at the first position (z = $z_{j-1}$) is sequentially and reversely propagated inside the j-th block for each slice by the numerical calculation in consideration of the refractive index distribution of the j-th block, and thus, the light wavefront at the second position (z = $z_j$) can be obtained.

FIG. C09 is a flowchart of the third complex amplitude image generation step S77. In a step S41, the position z is initialized to the first position (z = $z_{j-1}$) of the j-th block. In a step S42, interaction between the complex amplitude u(x, y, z) of the light at the position z and the phase change o(x, y, z) is obtained. In a step S43, the wavefront of the light after the interaction is propagated by the distance Δz, and the complex amplitude u(x, y, z + Δz) of the light at the position z + Δz is obtained. In a step S44, z obtained by adding Δz is set as new z. In a step S44, when it is determined that the position z has not yet reached the second position (z = $z_j$) of the j-th block, the process returns to the step S42 to repeat the steps S42 to S44. In the step S44, when it is determined that the position z reaches the second position (z = $z_j$) of the j-th block, the processing of the third complex amplitude image generation step S77 is ended. The complex amplitude of the light acquired at the end becomes the complex amplitude at the second position (z = $z_j$) of the j-th block.

Any of the refractive index distribution measuring methods A to C described above can realize three-dimensional refractive index tomography in which the influence of multiple scattered light is reduced even when the observation object is a multiple scattering object. Any of the refractive index distribution measuring methods A to C is suitable for measuring the refractive index distribution of a three-dimensional culture as an observation object.

In addition, self-interference may be used in the observation apparatus and the refractive index distribution measuring methods. For example, an observation apparatus 1J illustrated in FIG. C10 includes a light source 11, a lens 12, a lens 21, a mirror 22, a lens 23, a condenser lens 24, an objective lens 25, a mirror 44, a lens 42, an imaging unit 43, and an analysis unit 70. Compared with the configuration of the observation apparatus described above, the observation apparatus 1J is different in that the light output from the light source 11 is guided by the optical fiber 14, and then output from the light output end 18 without being split into two light beams. Further, the observation apparatus 1J is different in that the mirror 44 is provided instead of the beam splitter 41. The observation apparatus 1J does not include an interference optical system. The imaging unit 43 can image the interference intensity image at the reference position generated by self-interference of the light irradiating the observation object S along each of the plurality of light irradiation directions and passed through the observation object S. The analysis unit 70 can perform the same image processing as described above using the interference intensity image due to self-interference.

Further, the three-dimensional refractive index distribution of the observation object S from the first position to the second position may not be the refractive index distribution based on the three-dimensional phase image, and may be acquired separately by using a refractive index distribution acquisition apparatus capable of acquiring the refractive index distribution. In this case, the observation apparatus may include (1) an interference intensity image acquisition unit for acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation unit for generating, for each of the plurality of light irradiation directions, a complex amplitude image based on the interference intensity image, (3) a refractive index distribution acquisition unit for acquiring a three-dimensional refractive index distribution of the observation object between a first position and a second position with respect to a distance from the imaging unit along a light propagation path, and (4) a second complex amplitude image generation unit for generating, for each of the plurality of light irradiation directions, a complex amplitude image at the second position based on a complex amplitude image at the first position and the three-dimensional refractive index distribution (corresponding to the third complex amplitude image generation unit provided in the observation apparatuses 1A to 1D).

Further, in this case, the refractive index distribution measuring method may include (1) an interference intensity image acquisition step of acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation step of generating, for each of the plurality of light irradiation directions, a complex amplitude image based on the interference intensity image, (3) a refractive index distribution acquisition step of acquiring a three-dimensional refractive index distribution of the observation object between a first position and a second position with respect to a distance from the imaging unit along a light propagation path, and (4) a second complex amplitude image generation step of generating, for each of the plurality of light irradiation directions, a complex amplitude image at the second position based on a complex amplitude image at the first position and the three-dimensional refractive index distribution.

An aspect of the present disclosure is a method for determining a region of cells that have undergone necrosis in an observation object on the basis of refractive index distribution data of an observation object.

According to an embodiment of the present disclosure, determination of a region of cells that have undergone necrosis is carried out on the basis of the fact that a region of each cell included in the refractive index distribution data has features of necrotic cells. That is, a region in an observation object corresponding to a region of each cell having features of necrotic cells is determined to be a region of cells that have undergone necrosis.

The above-described features of the necrotic cells include at least features of having a region equivalent to the nucleus. The nucleus (cell nucleus) is one of organelles that eukaryotic cells possess, and particularly in mammalian cells, the nucleus exists in a shape close to a sphere having a radius of about 1 to 10 $\mu$m except for in the mitotic phase. Normally, one nucleus exists in one cell. The nucleus is separated from the cytoplasm by a nuclear membrane and has structures such as nucleoli and chromatin (thread-like structure consisting of intranuclear DNA) in the inside. Therefore, a region equivalent to the nucleus is, for example, an approximately circular or approximately spherical region that exists within the region of each cell included in the refractive index distribution data, and is a region in which the statistic value of the refractive index is greater than the statistic value of the refractive index of the whole cell. The statistic value is, for example, an average value, a median value, a maximum value, or a minimum value, and may be an average value or a median value. An approximately circular region indicates a region whose degree of true circularity is equal to or greater than a threshold value, and an approximately spherical region indicates a region whose degree of sphericity is equal to or greater than a threshold value. The above-described threshold value can be defined in accordance with the type and condition of the observation object, the type and content of the cells included in the observation object, and the like, and with regard to the refractive index distribution data of an observation object (reference) different from the observation object (sample) in which determination of the region of cells that have undergone necrosis is carried out, it can be defined that the region that is recognized to be a nucleus based on other structural features is determined to be a region equivalent to the nucleus, or it can be defined that a region fluorescently stained with 4',6-diamidino-2-phenylindole (DAPI), Hoechst 33258, Hoechst 33342, or the like, which are known nuclear staining reagents, is determined to be a region equivalent to the nucleus. The above-described threshold value may be, for example, 60%, may be 65%, may be 70%, may be 75%, or may be 80%. Furthermore, the region equivalent to the nucleus may be, for example, an approximately circular or approximately spherical region surrounded by a membrane structure with a high refractive index, which is equivalent to the nuclear membrane, the region existing within a region of each cell included in the refractive index distribution data. Furthermore, the region equivalent to the nucleus may be, for example, an approximately circular or approximately spherical region surrounded by a membrane structure with a high refractive index, which is equivalent to the nuclear membrane, the region existing within a region of each cell included in the refractive index distribution data, and may be a region having a nucleolus structure with a high refractive index and a thread-like chromatin structure with a refractive index lower than that of the nucleolus inside the region.

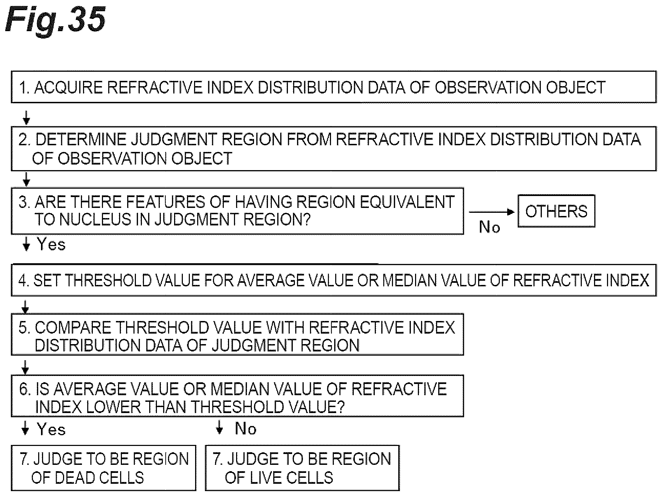

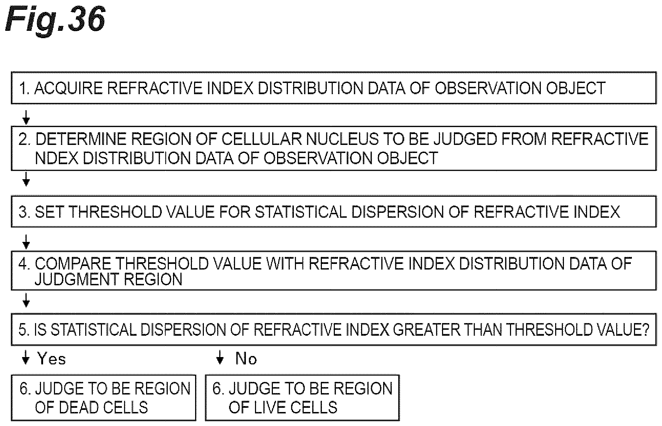

According to an embodiment of the present disclosure, the above-described features of necrotic cells further include one or more features selected from the group consisting of a feature in which the statistic value of the refractive index of the whole cell is equal to or less than a threshold value, and a feature in which the statistical dispersion of the refractive index of the nucleus is equal to or greater than the threshold value. The statistic value is, for example, an average value, a median value, a maximum value, or a minimum value, and may be an average value or a median value. In cells that have undergone necrosis, since the cellular contents leak due to cell rupture, the refractive index of the whole cell becomes lower than that of live cells. Furthermore, when focusing on the nucleus, the nucleoli within the nucleus have a relatively high refractive index even in cells that have undergone necrosis, and therefore, the statistical dispersion of the refractive index of the nucleus become large. Therefore, the region of cells that have undergone necrosis can be determined on the basis of the fact that the region of each cell included in the refractive index distribution data has one or more features selected from the group consisting of these features. The method for determining a region of cells that have undergone necrosis according to the present embodiment is referred to as "threshold method". In the determination of a region of cells that have undergone necrosis using the threshold method, FIG. 35 shows a typical flow in a case where the determination is made on the basis of having a feature in which the average value or median value of the refractive index of the whole cell is equal to or less than a threshold value, and FIG. 36 shows a typical flow in a case where the determination is made on the basis of having a feature in which the statistical dispersion of the refractive index of the nucleus is equal to or greater than a threshold value. The comparison between the statistic value of the refractive index of the whole cell or the statistical dispersion of the refractive index of the nucleus and the threshold value is carried out for each cell in a region of cells having a region equivalent to the nucleus, among the regions where the observation object exists in the refractive index distribution data (determination region). Incidentally, a region of cells that do not have a region equivalent to the nucleus may be considered as, for example, a region of cells that have undergone apoptosis. The statistical dispersion according to an embodiment of the present disclosure is not particularly limited as long as it is an indicator indicating the degree of unevenness of the data, and the statistical dispersion is selected from the group consisting of, for example, a standard deviation, a standard error, a variance, a coefficient of variation, and a difference between a maximum value and a minimum value. Regarding these statistical dispersions for each cell, a histogram may be calculated for the cell population, and features of the spread of the histogram may be extracted, or the threshold value that will be described below may be determined based on the above-described features. The threshold method may be carried out on unprocessed refractive index distribution data, or may be carried out on processed data that has been subjected to image processing emphasizing the amount of spatial variation of the refractive index. Furthermore, as one mechanism in which the nucleoli in necrotic cells have a relatively high refractive index, it may be considered that although nucleoli do not have a membrane structure, since they exist in a phase-separate independent state, leakage of the contents is less likely to occur.

The threshold value of the statistic value and the threshold value of the statistical dispersion can be defined in accordance with the type and condition of the observation object, the type and content of the cells included in the observation object, and the like, and for example, known information may be used, the threshold values may be defined such that the region of cells that can be judged to be cells that have undergone necrosis based on the morphology of the cells is determined to be the region of cells that have undergone necrosis, or the threshold values may be calculated by unsupervised machine learning such as clustering. Furthermore, regarding the refractive index distribution data of an observation object (reference) other than the observation object (sample) for which determination of a region of cells that have undergone necrosis is carried out, the threshold values can also be defined such that a region of cells that can be judged to be cells that have undergone necrosis is determined to be the region of cells that have undergone necrosis. In this case, when the sample is a cell cluster, the reference may be a cultured product of cells of the same cell type as the cells forming the sample, or may be a cell cluster consisting of cells of the same cell type. Furthermore, the reference may be an observation object in which the region of cells that have undergone necrosis can be clearly determined, one example may be an observation object in which at least cells that have undergone necrosis are fluorescently labeled, or another example may be an observation object in which at least cells that have undergone necrosis and live cells are fluorescently labeled.

The method for fluorescently labeling cells that have undergone necrosis is not particularly limited, and so long as the observation object is a cell cluster and culture conditions are such that programmed cell death such as apoptosis is not induced, for example, a fluorescent reagent capable of staining dead cells whose cell membranes have disintegrated, such as ethidium homodimer 1 or propidium iodide, can be used. Furthermore, according to an embodiment of the present disclosure, when the region of cells that have undergone necroptosis is determined instead of the region of cells that have undergone necrosis, for example, SMART (Shin Murai et. al., NATURE COMMUNICATIONS 9, 4457 (2018)) can be used as the fluorescent label for the cells that have undergone necroptosis.

The method of fluorescently labeling live cells is not particularly limited, and for example, Calcein-AM can be used.

According to an embodiment of the present disclosure, the determination of a region of cells that have undergone necrosis is carried out by inputting refractive index distribution data of an observation object into a learning model that has learned using training data including necrotic region data of a reference observation object and refractive index distribution data of the reference observation object corresponding to the above-described necrotic region data. According to an embodiment, the feature quantity utilized by the learning model includes the feature quantity corresponding to features of having a region equivalent to a nucleus. The reference observation object is an object composed of cells prepared or acquired by a method similar to that for the observation object, as a main component. The method for determining a region of cells that have undergone necrosis according to the present embodiment is referred to as "machine learning method".

A method for determining a region of cells that have undergone programmed cell death according to the machine learning method includes at least a learning step and an inference step.

In the learning step, a model is allowed to learn so as to infer a region of live cells and a region of cells that have undergone necrosis from feature quantities by machine learning, using training data including necrotic region data of a reference observation object and refractive index distribution data of the reference observation object corresponding to the above-described necrotic region data. The necrotic region data of the reference observation object is data indicating the spatial distribution of voxels or pixels occupied by cells that have undergone necrosis in a space including the reference observation object. The necrotic region data can be created from the data of any reference observation object that can reveal a region of cells that have undergone necrosis in the reference observation object; however, according to an embodiment, the above-described data of the reference observation object is fluorescence data of the reference observation object, that is, data indicating the spatial distribution of the fluorescence intensity of voxels or pixels in a space including the reference observation object.

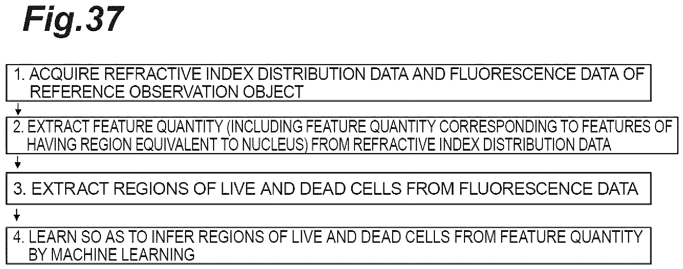

A typical learning flow of a model in the learning step in a case where the necrotic region data is created from fluorescence data of the reference observation object, is shown in FIG. 37. In a first stage of the learning step, refractive index distribution data and fluorescence data of a reference observation object in which at least cells that have undergone necrosis are fluorescently labeled, are acquired. The reference observation object may also be live cells that are fluorescently stained. Fluorescent labeling of the cells that have undergone necrosis and live cells can be carried out by, for example, a method similar to the method mentioned in the threshold method.

In a second stage of the learning step, the feature quantity is extracted from the refractive index distribution data. The above-described feature quantity is a feature quantity related to the spatial distribution of the refractive index and may be a vector or a scalar; however, the feature quantity includes at least a feature quantity corresponding to features of having a region equivalent to the nucleus.

In a third stage of the learning step, a region of cells that have undergone necrosis is extracted from the fluorescence data, and necrotic region data is created. In creating the necrotic region data, for example, on the basis of fluorescence data corresponding to the excitation wavelength and fluorescence wavelength of the dye labeling the cells that have undergone necrosis, a region of fluorescently labeled cells can be determined to be the region of cells that have undergone necrosis. When further determining a region of live cells, for example, on the basis of fluorescence data corresponding to the excitation wavelength and fluorescence data of the dye labeling live cells, a region of fluorescently labeled cells can be determined to be the region of live cells. Furthermore, for example, on the basis of fluorescence data corresponding to the excitation wavelength and fluorescence wavelength of the dye labeling all cells, among the regions of fluorescently labeled cells, a region other than the region that has been determined to be a region of cells that have undergone necrosis can be determined to be the region of live cells.

In a fourth stage of the learning step, a model is allowed to learn by machine learning using training data including the necrotic region data of the reference observation object and the refractive index distribution data of the reference observation object corresponding to the above-described necrotic region data, such that the region of cells that have undergone necrosis and the region of live cells determined in the third stage are inferred using the feature quantity extracted in the second stage as input. By using, as training data, the data of the region of cells that have undergone necrosis and the region of live cells, which have been subjected to verification such as same visual field image capturing of the fluorescence data and the refractive index distribution data, improvement in the accuracy of judgment can be expected.

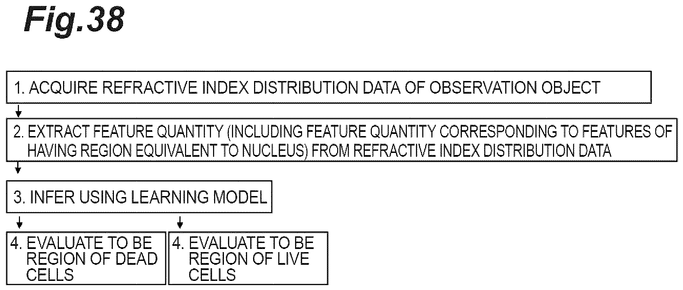

A typical inference flow in the inference step is shown in FIG. 38. In a first stage of the inference step, the refractive index distribution data of the observation object is acquired. In a subsequent second stage, the same feature quantity as that extracted in the second stage of the learning step is extracted from the refractive index distribution data. In a subsequent third stage, the region of cells that have undergone necrosis and the region of live cells of the observation object are inferred using the model that has been allowed to learn in the learning step.

Another aspect of the present disclosure is a method of determining a region of cells that have undergone necrosis in an observation object, the method including: a step of acquiring refractive index distribution data of an observation object; and a step of determining a region of cells that have undergone necrosis in the observation object using the refractive index distribution data of the observation object. The method of acquiring the refractive index distribution data is not particularly limited, and furthermore, the determination of the region of cells that have undergone necrosis may be carried out by the threshold method or may be carried out by the machine learning method.

Another aspect of the present disclosure is a method for evaluating quality of a cell cluster, the method including: a step of acquiring refractive index distribution data of a cell cluster; and a step of determining a region of cells that have undergone necrosis inside the cell cluster on a basis of the refractive index distribution data. Necrosis is typically a cell death occurring in cells exposed to stimuli such as hypoxia, high temperature, poisons, nutrient deficiency, and cell membrane damage in a living body, while on the other hand, even when cells are three-dimensionally cultured to create a cell cluster, regions with high cell density inside the cell cluster are prone to be placed in a hypoxic condition and/or a nutrient-deficient condition, and therefore, cells inside the cell cluster, particularly cells in the vicinity of the center, often undergo necrosis. Then, the quality of the cell cluster can be evaluated by determining a region of cells that have undergone necrosis inside the cell cluster on the basis of the refractive index distribution data, using the method for determining a region of cells that have undergone necrosis according to the present disclosure. That is, depending on the purpose, a cell cluster having a larger region of cells that have undergone necrosis inside the cell cluster can be evaluated as a cell cluster of higher quality, or a cell cluster having a smaller region of cells that have undergone necrosis inside the cell cluster can be evaluated as a cell cluster of higher quality.

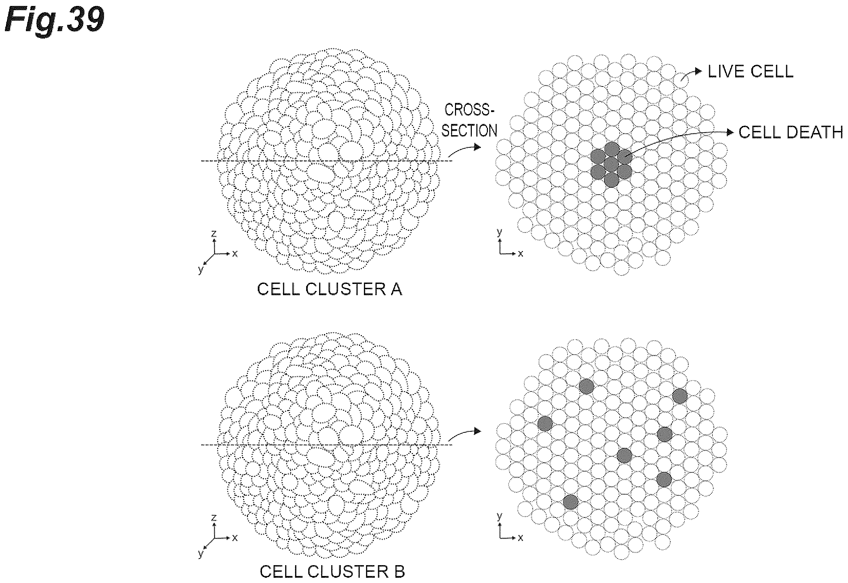

According to an embodiment, the above-described cell cluster is a cancer cell cluster. A cancer cell cluster is a cell cluster including cancer cells. According to an embodiment, the cancer cell cluster is a cell cluster in which the cells included in the cell cluster are cancer cells. An example of a case in which the above-described cell cluster is a cancer cell cluster, the region of cells that have undergone necrosis inside the cell cluster is large, and a cell cluster existing closer to the central part is evaluated as a cell cluster of higher quality, is shown in FIG. 39. A cancer microenvironment in the living body is generally a hypoxic condition and/or a nutrient-deficient condition. Therefore, a region of cells that are in a hypoxic condition and/or a nutrient-deficient condition in the vicinity of the center of a cancer cell cluster is considered to be a useful cancer research model that mimics the cancer microenvironment in the living body, and the size of the region of cells that have undergone necrosis inside the cancer cell cluster can be used as an indicator thereof. That is, a cancer cell cluster having a larger region of cells that have undergone necrosis inside the cancer cell cluster and existing closer to the central part is deemed to more highly mimic the cancer microenvironment and is evaluated to be cancer cells of higher quality, the cells in the region surrounding the region of cells that have undergone necrosis can be used as a cancer research model, and for example, in the example of FIG. 39, a cell cluster A can be evaluated as a cancer cell cluster of high quality, while the cell death caused by cells in the region surrounding the region of cells that have undergone necrosis can also be observed in a time-lapse manner.

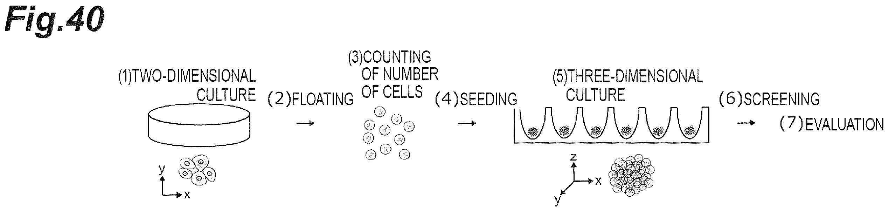

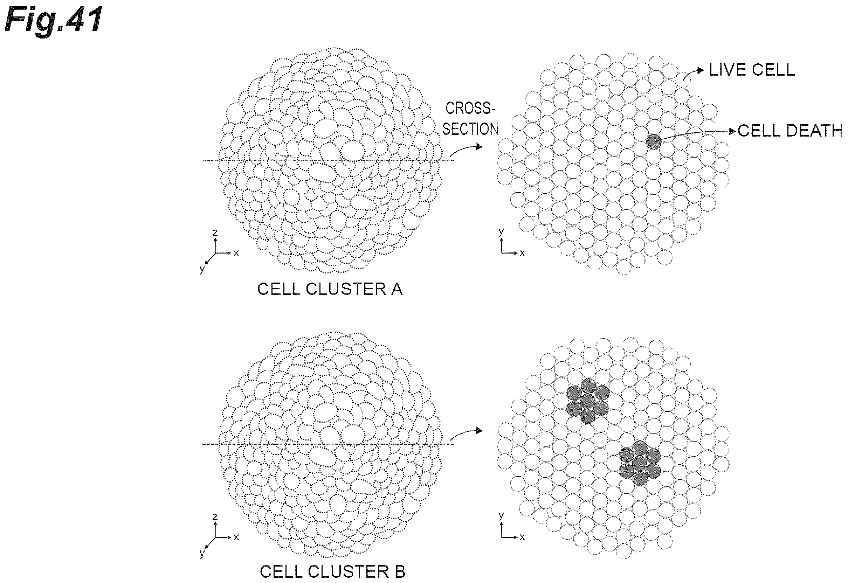

According to an embodiment, the above-described cell cluster is a cell cluster obtained by culturing stem cells, an example thereof is a cell cluster obtained by culturing stem cells collected from an animal including a human, and another example thereof is a cell culture obtained by culturing stem cells collected from a human. An example of a production process for a cell cluster in a case where the above-described cell cluster is a cell cluster obtained by culturing stem cells is shown in FIG. 40, and an example of a case in which, in the evaluation of the above-described preparation process, a cell cluster having a smaller region of cells that have undergone necrosis inside the cell cluster is evaluated as a cell cluster of higher quality, is shown in FIG. 41. While necrosis may occur during the production process and culturing process of cell clusters, in a case where a cell cluster obtained by culturing stem cells is used in regenerative medicine, it is more preferable when the region of cells that have undergone necrosis inside the cell cluster is smaller. That is, among the cell clusters obtained by culturing stem cells, a cell cluster having a small region of cells that have undergone necrosis inside the cell cluster can be evaluated to have high quality, and for example, in the example of FIG. 41, a cell cluster A can also be used in regenerative medicine as a cell cluster of high quality. An example of using a cell cluster obtained by culturing stem cells collected from a human in regenerative medicine may be a case in which a cell cluster obtained by extracting stem cells from fat cells collected from a subject and culturing the stem cells, is transplanted back into the same subject.

The present disclosure will be described in detail below by way of Examples and the like; however, the present disclosure is not limited to these.

**Examples**

[Example 1: Acquisition of Refractive Index Distribution Data of HepG2 Cells That Have Undergone Necrosis by Freeze-thawing Treatment]

In order to reveal the features of necrotic cells, the refractive index distribution data of HepG2 cells that had undergone necrosis by a freeze-thawing treatment was acquired.

1 mL of Trypsin-EDTA (0.25%), phenol red (manufactured by Gibco, Inc.) was added to HepG2 cells, a human hepatoma-derived cell line, which were adhered to a T25 flask, to cause the HepG2 cells to float. 5 mL of DMEM medium containing 10% fetal bovine serum was added thereto to inactivate trypsin, subsequently a centrifugal treatment was performed at 1000 rpm for 3 minutes to discard the supernatant, and 5 mL of DMEM medium containing 10% fetal bovine serum was added to cell pellets to suspend the cells. The solution containing the HepG2 cells in a floating state was left to stand at -80°C for 17.5 hours to be frozen, and then the solution was left to stand at room temperature for 2.5 hours to be thawed (freeze-thawing step).

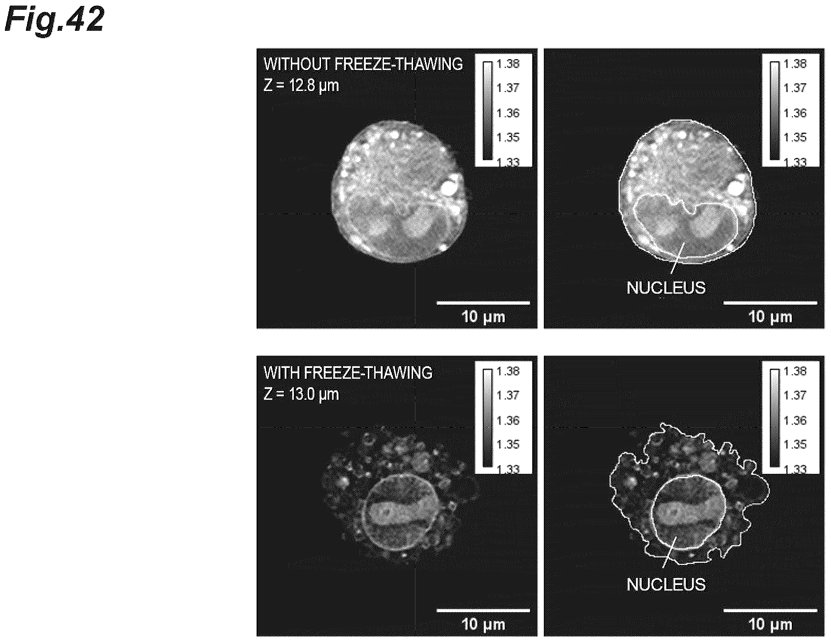

For the obtained HepG2 cells in a floating state, which had undergone necrosis (with freeze-thawing), and HepG2 cells in a floating state obtained by a similar method except that the freeze-thawing step was not carried out (without freeze-thawing), the refractive index distribution data was acquired using an observation apparatus 1A and the ODT shown in the refractive index distribution measurement method A2. An objective lens at a magnification of 60 times was used. Representative refractive index tomography data extracted from the refractive index distribution data obtained under the conditions without freeze-thawing or with freeze-thawing is shown in FIG. 42. In the drawing, the left-side column shows the extracted refractive index tomography data, and the right-side column shows the refractive index tomography data in which regions targeted for the calculation of an average value, a median value, and a standard deviation are surrounded by white lines, assuming that the regions are the whole cell (hereinafter, also described as the whole) or the nucleus. Furthermore, the average values, median values, and standard deviations of the refractive indices of the whole and the nucleus under the conditions without freeze-thawing or with freeze-thawing, which were calculated on the basis of the refractive index tomography data of FIG. 42, are shown in Table 1.

[Table 1]

| Freeze-thawing | Region | Average value | Median value | Standard deviation |
|---|---|---|---|---|
| Absent | Whole | 1.363 | 1.363 | 0.008 |
| Present | Whole | 1.344 | 1.342 | 0.008 |
| Absent | Nucleus | 1.361 | 1.361 | 0.005 |
| Present | Nucleus | 1.350 | 1.350 | 0.007 |

According to FIG. 42 and Table 1, in the cells in which necrosis was induced by freeze-thawing, a morphology that suggested leakage of contents along with disintegration of the cell membrane was observed, and the refractive index of the whole was low compared to the cells without freeze-thawing. On the other hand, under the conditions with freeze-thawing, an approximately circular region in which the average refractive index was higher than that of the whole, the region being surrounded by a nuclear membrane with a high refractive index, and having a region of nucleoli with a high refractive index and chromatin with a low refractive index inside the region, was observed within the cell in the refractive index tomography data. That is, cells that had undergone necrosis did not cause nuclear fragmentation, which is observed in cells that have undergone apoptosis, and had the features of the nucleus. In addition, under the conditions with freeze-thawing, the nucleoli in the nucleus had a high refractive index, while the chromatin region had a low refractive index, and therefore, the cells that had undergone necrosis had a larger standard deviation in the refractive index of the nucleus as compared with cells without freeze-thawing.

[Example 2: Acquisition of Refractive Index Distribution Data of A549 Cell cluster That Had Undergone Necrosis by Treatment of Leaving at Room Temperature]

In order to check whether the features of necrotic cells revealed in Example 1 would also apply to cell clusters, the refractive index distribution data of an A549 cell cluster that had undergone necrosis by a treatment of leaving at room temperature was acquired.

A549 cells, a human lung cancer-derived cell line, were seeded on an EZSPHERE 6-well plate (manufactured by AGC Techno Glass Co., Ltd.) at a density of $0.75 \times 10^5$ cells per well, and the cells were cultured in DMEM medium containing 10% fetal bovine serum and MEM Non-Essential Amino Acids Solution ($100\times$) (manufactured by Gibco, Inc.) at a final concentration of $1\times$ under the conditions of 5% CO2, 37°C, and 100% humidity for 3 days, to form cell clusters. The formed cell clusters were left to stand overnight under the conditions of room temperature in 2 mL of the medium inside a sealed 15 mL conical-type centrifuge tube in which the control of $CO_2$ and humidity was not achieved, and necrosis was induced.

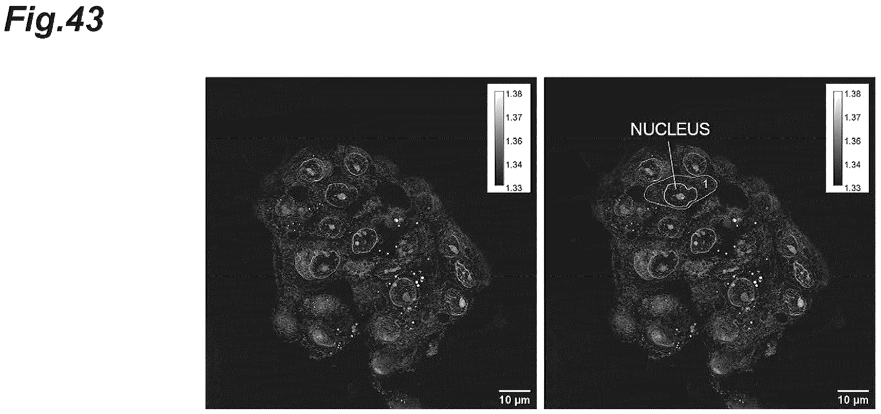

For the obtained cell clusters, the refractive index distribution data was acquired using the observation apparatus 1A and the ODT shown in the refractive index distribution measurement method C. Representative refractive index tomography data extracted from the obtained refractive index distribution data is shown in FIG. 43. In the drawing, the left-side column shows the extracted refractive index tomography data, and the right-side column shows the refractive index tomography data in which regions targeted for the calculation of an average value, a median value, and a standard deviation are surrounded by white lines, assuming that the regions are the whole cell or the nucleus as a target of analysis. Furthermore, the average values, median values, and standard deviations of the refractive indices of the whole and the nucleus calculated on the basis of the refractive index tomography data of FIG. 43, are shown in Table 2.

[Table 2]

| Region | Average value | Median value | Standard deviation |
|--------|---------------|--------------|--------------------|
| Whole | 1.339 | 1.337 | 0.006 |
| Nucleus | 1.340 | 1.337 | 0.007 |

According to FIG. 43 and Table 2, the cells that had undergone necrosis in the cell clusters left to stand at room temperature were such that the average refractive index of the whole (1.339) was low similarly to the case of the conditions with freeze-thawing in Example 1 (1.344), while the standard deviation of the refractive index of the nucleus (0.007) was high similarly to the case of the conditions with freeze-thawing in Example 1 (0.007), and therefore, it was found that the features of necrotic cells observed in the single cells in Example 1 also apply to the cell clusters left to stand at room temperature.

[Example 3: Acquisition of Refractive Index Distribution Data of HepG2 Cell cluster That Had Undergone Necrosis by High-concentration Ethanol Treatment]

In order to check whether the features of necrotic cells also apply to cells in cell clusters in which necrosis was induced by a method different from that of Example 2, the refractive index distribution data of HepG2 cell clusters that had undergone necrosis by a high-concentration ethanol treatment was acquired.

HepG2 cells, a human hepatoma-derived cell line, were seeded on an EZSPHERE 6-well plate (manufactured by AGC Techno Glass Co., Ltd.) at a density of $1\times10^5$ to $1.5\times10^5$ cells per well, and the cells were cultured in DMEM medium containing 10% fetal bovine serum under the conditions of 5% CO2, 37°C, and 100% humidity for 6 days, to form cell clusters. Ethanol (manufactured by FUJIFILM Wako Pure Chemical Corporation) was added thereto so as to obtain a final concentration of 10% by volume (1.7 mol/L), and the cells were left to stand under the conditions of 5% CO2, 37°C, and 100% humidity for 30 minutes to induce necrosis.

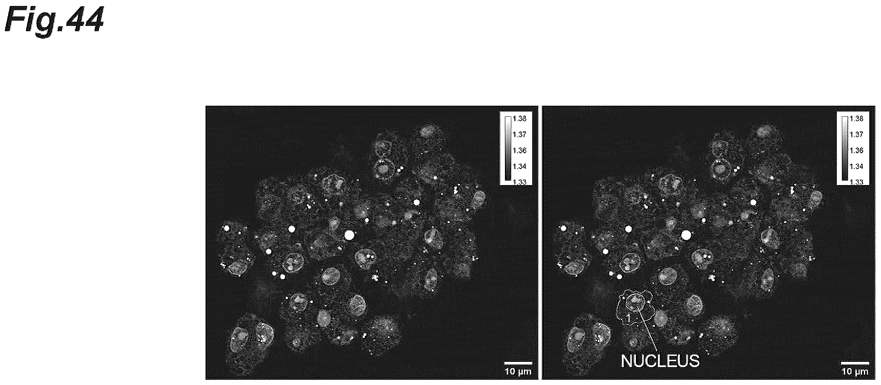

For the obtained cell clusters, the refractive index distribution data was acquired using the observation apparatus 1A and the ODT shown in the refractive index distribution measurement method C. Representative refractive index tomography data extracted from the obtained refractive index distribution data is shown in FIG. 44. In the drawing, the left-side column shows the extracted refractive index tomography data, and the right-side column shows the refractive index tomography data in which regions targeted for the calculation of an average value, a median value, and a standard deviation are surrounded by white lines, assuming that the regions are the whole cell or the nucleus as a target of analysis. Furthermore, the average values, median values, and standard deviations of the refractive indices of the whole and the nucleus calculated on the basis of the refractive index tomography data of FIG. 44, are shown in Table 3.

[Table 3]

| Region | Average value | Median value | Standard deviation |
|--------|---------------|--------------|--------------------|
| Whole | 1.341 | 1.339 | 0.008 |
| Nucleus | 1.350 | 1.348 | 0.008 |

According to FIG. 44 and Table 3, the cells that had undergone necrosis in the cell clusters treated with high-concentration ethanol were such that the average refractive index of the whole (1.341) was low similarly to the case of the cells in the cell clusters treated by being left to stand at room temperature in Example 2 (1.339), while the standard deviation of the refractive index of the nucleus (0.008) was high similarly to the case of the cells in the cell clusters treated by being left to stand at room temperature in Example 2 (0.007), and therefore, it was found that the features of necrotic cells observed in the cells in the cell clusters treated by being left to stand at room temperature in Example 2 also apply to the cell clusters treated with high-concentration ethanol.

[Example 4: Acquisition of Refractive Index Distribution Data of A549 Cell clusters That Had Undergone Necrosis by High-concentration Ethanol Treatment]

In order to check whether the features of cells in which necrosis was induced by a high-concentration ethanol treatment also apply to cells in cell clusters other than the HepG2 cell clusters, the refractive index distribution data of A549 cell clusters that had undergone necrosis by a high-concentration ethanol treatment was acquired.

A549 cells, a human lung cancer-derived cell line, were seeded on an EZSPHERE 6-well plate (manufactured by AGC Techno Glass Co., Ltd.) at a density of $1\times10^5$ cells per well, and the cells were cultured in MEM medium containing 10% fetal bovine serum and MEM Non-Essential Amino Acids Solution ($100\times$) (manufactured by Gibco, Inc.) at a final concentration of $1\times$ under the conditions of 5% CO2, 37°C, and 100% humidity for 4 days, to form cell clusters. Ethanol (manufactured by FUJIFILM Wako Pure Chemical Corporation) was added thereto so as to obtain a final concentration of 10% by volume (1.7 mol/L), and the cells were left to stand under the conditions of 5% CO2, 37°C, and 100% humidity for 10 minutes to induce necrosis.

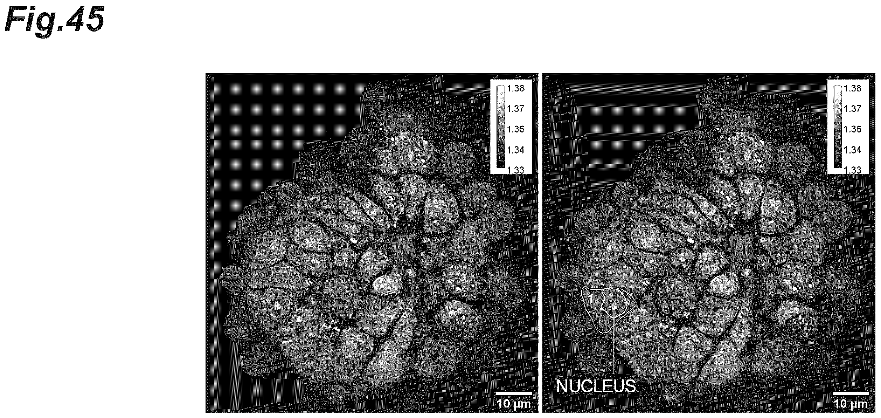

For the obtained cell clusters, the refractive index distribution data was acquired using the observation apparatus 1A and the ODT shown in the refractive index distribution measurement method C. Representative refractive index tomography data extracted from the obtained refractive index distribution data is shown in FIG. 45. In the drawing, the left-side column shows the extracted refractive index tomography data, and the right-side column shows the refractive index tomography data in which regions targeted for the calculation of an average value, a median value, and a standard deviation are surrounded by white lines, assuming that the regions are the whole cell or the nucleus as a target of analysis. Furthermore, the average values, median values, and standard deviations of the refractive indices of the whole and the nucleus calculated on the basis of the refractive index tomography data of FIG. 45, are shown in Table 4.

[Table 4]

| Region | Average value | Median value | Standard deviation |
|---|---|---|---|
| Whole | 1.350 | 1.350 | 0.006 |
| Nucleus | 1.353 | 1.352 | 0.006 |

According to FIG. 45 and Table 4, the features of necrotic cells similar to those of the cells in the HepG2 cell clusters treated with high-concentration ethanol were observed in the cells in the A549 cell clusters treated with high-concentration ethanol as well.

[Example 5: Acquisition of Refractive Index Distribution Data of HepG2 Cell cluster Including Cells That Have Undergone Necrosis under High-Density Cell Conditions within Cell cluster]

In order to examine whether it is possible to determine a region of cells that had undergone necrosis in any cross-section of a cell cluster in which live cells and dead cells were present in mixture, refractive index distribution data of HepG2 cell clusters containing cells that had undergone necrosis under high-density cell conditions within a cell cluster was acquired, and an examination was conducted to see whether it is possible to determine a region of cells that had undergone necrosis on the basis of the features of necrotic cells.

HepG2 cells, a human hepatoma-derived cell line, were seeded on an EZSPHERE 6-well plate (manufactured by AGC Techno Glass Co., Ltd.) at a density of $0.75\times10^5$ to $2\times10^5$ cells per well, and the cells were cultured in DMEM medium containing 10% fetal bovine serum under the conditions of 5% CO2, 37°C, and 100% humidity for 4 days, to form cell clusters.

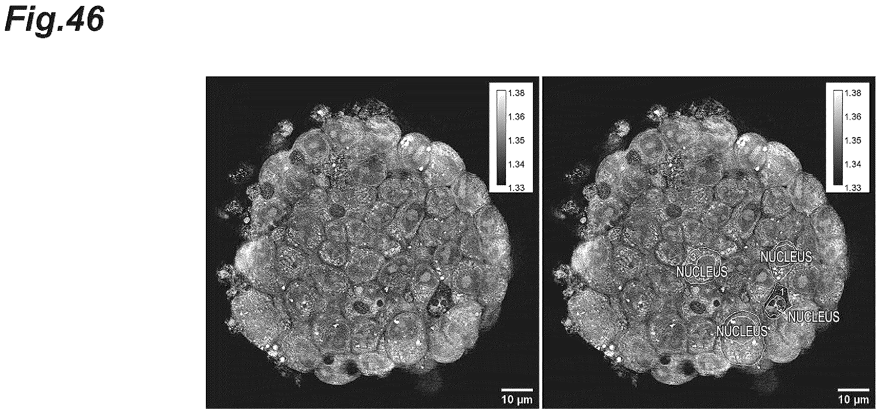

For the obtained cell clusters, the refractive index distribution data was acquired using the observation apparatus 1A and the ODT shown in the refractive index distribution measurement method C. Representative refractive index tomography data extracted from the obtained refractive index distribution data is shown in FIG. 46. In the drawing, the left-side column shows the extracted refractive index tomography data, and the right-side column shows the refractive index tomography data in which regions targeted for the calculation of an average value, a median value, and a standard deviation are surrounded by white lines, assuming that the regions are the whole cell or the nucleus as a target of analysis. Furthermore, the average values, median values, and standard deviations of the refractive indices of the whole and the nucleus calculated on the basis of the refractive index tomography data of FIG. 46, are shown in Table 5.

[Table 5]

| Cell | Region | Average value | Median value | Standard deviation |
|------|--------|---------------|--------------|--------------------|
| 1 | Whole | 1.345 | 1.344 | 0.007 |
| 2 | Whole | 1.356 | 1.356 | 0.006 |
| 3 | Whole | 1.352 | 1.352 | 0.005 |
| 4 | Whole | 1.351 | 1.351 | 0.006 |
| 1 | Nucleus | 1.349 | 1.348 | 0.007 |
| 2 | Nucleus | 1.354 | 1.353 | 0.005 |
| 3 | Nucleus | 1.351 | 1.351 | 0.003 |
| 4 | Nucleus | 1.350 | 1.349 | 0.005 |

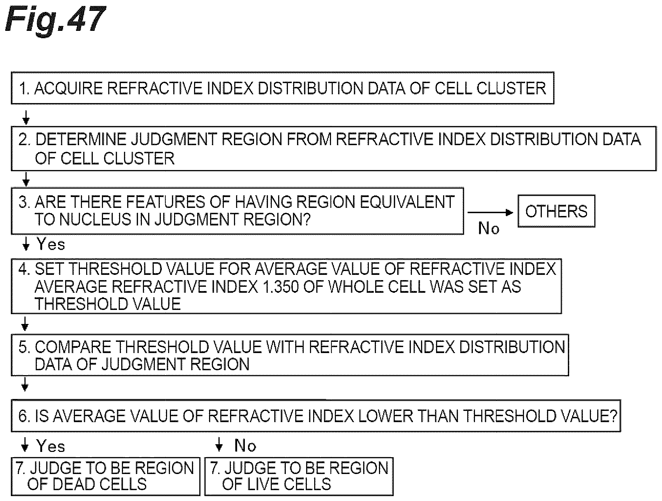

The results of FIG. 46 and Table 5 were examined according to the flow shown in FIG. 47 to see whether a region of cells that had undergone necrosis could be determined, on the basis of having the features of necrotic cells when the features of having a region equivalent to the nucleus and the features in which the average value of the refractive index of the whole cell was equal to or less than the threshold value were utilized as the features of necrotic cells. Although cells 1 to 4 in FIG. 46 all had regions equivalent to the nucleus, only cell 1 had a low refractive index of the whole (1.345), and therefore, by setting the threshold value of the average refractive index of the whole to 1.350, the cell 1 having a refractive index of the whole lower than the threshold value could be determined to be a region of dead cells (region of cells that had undergone necrosis).

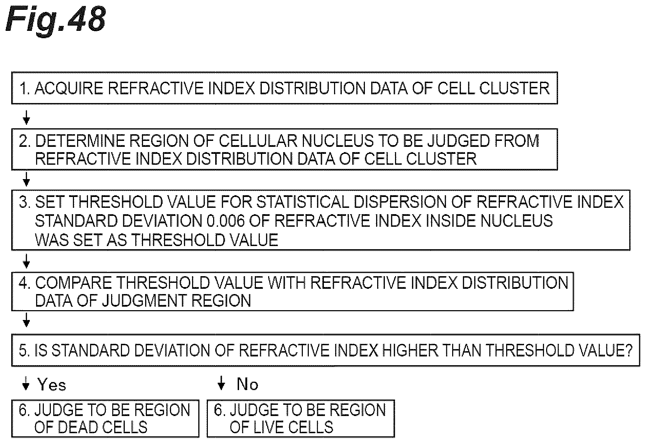

Furthermore, the results of FIG. 46 and Table 5 were examined according to the flow shown in FIG. 48 to see whether a region of cells that had undergone necrosis could be determined, on the basis of having the features of necrotic cells when the features of having a region equivalent to the nucleus and the features in which the statistical dispersion of the refractive index of the nucleus was equal to or greater than the threshold value were utilized as the features of necrotic cells. Although cells 1 to 4 in FIG. 46 all had regions equivalent to the nucleus, only cell 1 had a high standard deviation of the refractive index of the nucleus (0.007), and therefore, by setting the threshold value of the standard deviation of the nucleus to 0.006, the cell 1 having a refractive index of the nucleus higher than the threshold value could be determined to be a region of dead cells (region of cells that had undergone necrosis).

### Reference Signs List

1A to 1J: observation apparatus, 2: recording medium, 11: light source, 12: lens, 13: light input end, 14: optical fiber, 15: fiber coupler, 16, 17: optical fiber, 18, 19: light output end, 21: lens, 22: mirror, 23: lens, 24: condenser lens, 25: objective lens, 31: lens, 32: mirror, 33: drive unit, 34: lens, 41: beam splitter, 42: lens, 43: imaging unit, 44: mirror, 50: analysis unit, 51: interference intensity image acquisition unit, 52: first complex amplitude image generation unit, 53: second complex amplitude image generation unit, 54: two-dimensional phase image generation unit, 55: three-dimensional phase image generation unit, 56: refractive index distribution calculation unit, 57: display unit, 58: storage unit, 60: analysis unit, 61: interference intensity image acquisition unit, 62: first complex amplitude image generation unit, 63: second complex amplitude image generation unit, 64: phase conjugate operation unit, 65: two-dimensional phase image generation unit, 66: three-dimensional phase image generation unit, 67: refractive index distribution calculation unit, 68: display unit, 69: storage unit, 70: analysis unit, 71: interference intensity image acquisition unit, 72: first complex amplitude image generation unit, 73: second complex amplitude image generation unit, 74: two-dimensional phase image generation unit, 75: three-dimensional phase image generation unit, 76: refractive index distribution calculation unit, 77: third complex amplitude image generation unit, 78: display unit, 79: storage unit.

### Claims

1. A method for determining a region of cells that have undergone necrosis in an observation object on a basis of refractive index distribution data of the observation object.

2. The method according to claim 1, wherein determination of the region of cells that have undergone necrosis is carried out based on a fact that a region of each cell included in the refractive index distribution data has features of necrotic cells, and the features of the necrotic cells include features of having a region equivalent to a nucleus, and include one or more features selected from the group consisting of a feature in which a statistic value of a refractive index of a whole cell is equal to or less than a threshold value and a feature in which statistical dispersion of refractive index of a nucleus is equal to or greater than a threshold value.

3. The method according to claim 2, wherein the statistic value is an average value or a median value of the refractive index of the whole cell.

4. The method according to claim 2, wherein the region equivalent to the nucleus is an approximately circular or approximately spherical region and is a region in which a statistic value of refractive index is a value greater than a statistic value of refractive index of a whole cell.

5. The method according to claim 4, wherein the statistic value is an average value or a median value of the refractive index of the whole cell.

6. The method according to claim 1, wherein the refractive index distribution data is refractive index tomography data in a predetermined direction.

7. The method according to claim 1, wherein the determination of a region of cells that have undergone necrosis is carried out by inputting refractive index distribution data of an observation object into a learning model that has learned using training data including necrotic region data of a reference observation object and refractive index distribution data of the reference observation object corresponding to the necrotic region data, and a feature quantity utilized by the learning model includes a feature quantity corresponding to features of having a region equivalent to a nucleus.

8. A method for determining a region of cells that have undergone necrosis in an observation object, the method comprising:

    a step of acquiring refractive index distribution data of an observation object; and
    a step of determining a region of cells that have undergone necrosis by the method according to any one of claims 1 to 7.

9. A method for evaluating quality of a cell cluster,

    the observation object being a cell cluster,
    the method comprising:

        a step of acquiring refractive index distribution data of a cell cluster; and
        a step of determining a region of cells that have undergone necrosis inside the cell cluster by the method according to any one of claims 1 to 7.

10. An apparatus for determining a region of cells that have undergone necrosis, the apparatus comprising:

    a data acquisition unit for acquiring refractive index distribution data of an observation object; and
    a determination unit for determining a region of cells that have undergone necrosis in the observation object, by the method according to any one of claims 1 to 7.

11. An information processing program for causing a computer to execute a determination step of determining a region of cells that have undergone necrosis in an observation object, by the method according to any one of claims 1 to 7.

12. An information processing program for causing a computer to execute:

    a data acquisition step of acquiring refractive index distribution data of an observation object; and
    a determination step of determining a region of cells that have undergone necrosis in the observation object, by the method according to any one of claims 1 to 6.

# Fig.1

**ANALYSIS UNIT** — 50

- INTERFERENCE INTENSITY IMAGE ACQUISITION UNIT — 51
- FIRST COMPLEX AMPLITUDE IMAGE GENERATION UNIT — 52
- SECOND COMPLEX AMPLITUDE IMAGE GENERATION UNIT — 53
- TWO-DIMENSIONAL PHASE IMAGE GENERATION UNIT — 54
- THREE-DIMENSIONAL PHASE IMAGE GENERATION UNIT — 55
- REFRACTIVE INDEX DISTRIBUTION CALCULATION UNIT — 56
- DISPLAY UNIT — 57
- STORAGE UNIT — 58

RECORDING MEDIUM — 2

1A

EP 4 538 385 A1

# Fig.2

EP 4 538 385 A1

**Fig.3**

| | |
|---|---|
| ANALYSIS UNIT | 50 |
| INTERFERENCE INTENSITY IMAGE ACQUISITION UNIT | 51 |
| FIRST COMPLEX AMPLITUDE IMAGE GENERATION UNIT | 52 |
| SECOND COMPLEX AMPLITUDE IMAGE GENERATION UNIT | 53 |
| TWO-DIMENSIONAL PHASE IMAGE GENERATION UNIT | 54 |
| THREE-DIMENSIONAL PHASE IMAGE GENERATION UNIT | 55 |
| REFRACTIVE INDEX DISTRIBUTION CALCULATION UNIT | 56 |
| DISPLAY UNIT | 57 |
| STORAGE UNIT | 58 |
| RECORDING MEDIUM | 2 |

EP 4 538 385 A1

# Fig.4

INTERFERENCE INTENSITY IMAGE ACQUISITION — S1

INTERFERENCE INTENSITY IMAGE FOR
EACH LIGHT IRRADIATION DIRECTION

FIRST COMPLEX AMPLITUDE
IMAGE GENERATION — S2

COMPLEX AMPLITUDE IMAGE FOR
EACH LIGHT IRRADIATION DIRECTION

SECOND COMPLEX AMPLITUDE
IMAGE GENERATION — S3

COMPLEX AMPLITUDE IMAGE FOR
EACH POSITION AND EACH
LIGHT IRRADIATION DIRECTION

TWO-DIMENSIONAL PHASE IMAGE GENERATION — S4

TWO-DIMENSIONAL PHASE IMAGE
FOR EACH POSITION

THREE-DIMENSIONAL PHASE IMAGE GENERATION — S5

THREE-DIMENSIONAL
PHASE IMAGE

REFRACTIVE INDEX DISTRIBUTION CALCULATION — S6

THREE-DIMENSIONAL REFRACTIVE
INDEX DISTRIBUTION

*Fig.5*

(a)

(b)

(c)

Fig.6

# Fig.7

(a)

(b)

EP 4 538 385 A1

42

*Fig.8*

(a)  (b)  (c)

EP 4 538 385 A1

# Fig.9

```
┌─────────────────────────────────────┐
│  COMPLEX AMPLITUDE IMAGE FOR         │
│  EACH LIGHT IRRADIATION DIRECTION    │
└─────────────────────────────────────┘
                 │
┌─────────────────────────────────────┐
│  PHASE CORRECTION AND SUMMATION OF   │ ~S11
│  COMPLEX AMPLITUDE IMAGES            │
└─────────────────────────────────────┘
                 │
┌─────────────────────────────────────┐
│  COMPLEX AMPLITUDE SUMMATION         │
│  IMAGE FOR EACH POSITION             │
└─────────────────────────────────────┘
                 │
┌─────────────────────────────────────┐
│  PHASE IMAGE GENERATION              │ ~S12
└─────────────────────────────────────┘
                 │
┌─────────────────────────────────────┐
│  TWO-DIMENSIONAL PHASE IMAGE         │
│  FOR EACH POSITION                   │
└─────────────────────────────────────┘
```

# Fig.10

```
┌─────────────────────────────────┐
│    COMPLEX AMPLITUDE IMAGE FOR   │
│       EACH POSITION AND EACH     │
│      LIGHT IRRADIATION DIRECTION │
└─────────────────────────────────┘
                  │
┌─────────────────────────────────┐
│  COMPLEX DIFFERENTIAL INTERFERENCE│   ～S21
│        IMAGE GENERATION          │
└─────────────────────────────────┘
                  │
┌─────────────────────────────────┐
│ COMPLEX DIFFERENTIAL INTERFERENCE│
│    IMAGE FOR EACH POSITION AND   │
│    EACH LIGHT IRRADIATION DIRECTION│
└─────────────────────────────────┘
                  │
┌─────────────────────────────────┐
│  PHASE DIFFERENTIAL IMAGE GENERATION│   ～S22
└─────────────────────────────────┘
                  │
┌─────────────────────────────────┐
│      PHASE DIFFERENTIAL IMAGE    │
│         FOR EACH POSITION        │
└─────────────────────────────────┘
                  │
┌─────────────────────────────────┐
│       PHASE IMAGE GENERATION     │   ～S23
└─────────────────────────────────┘
                  │
┌─────────────────────────────────┐
│   TWO-DIMENSIONAL PHASE IMAGE    │
│        FOR EACH POSITION         │
└─────────────────────────────────┘
```

Fig.11

# Fig.12

COMPLEX AMPLITUDE IMAGE FOR
EACH POSITION AND EACH
LIGHT IRRADIATION DIRECTION

DIVIDING INTO BATCHES AND PHASE CORRECTION
AND SUMMATION OF COMPLEX AMPLITUDE IMAGES
FOR EACH BATCH —S31

COMPLEX AMPLITUDE SUMMATION
IMAGE FOR EACH POSITION
AND EACH BATCH

COMPLEX DIFFERENTIAL INTERFERENCE
IMAGE GENERATION —S32

COMPLEX DIFFERENTIAL
INTERFERENCE IMAGE FOR
EACH POSITION AND EACH BATCH

PHASE DIFFERENTIAL IMAGE GENERATION —S33

PHASE DIFFERENTIAL IMAGE
FOR EACH POSITION

PHASE IMAGE GENERATION —S34

TWO-DIMENSIONAL PHASE IMAGE
FOR EACH POSITION

Fig.13

Fig.14

Fig.15

EP 4 538 385 A1

# Fig.16

INTERFERENCE INTENSITY IMAGE ACQUISITION ~S61

NTERFERENCE INTENSITY IMAGE FOR
EACH LIGHT IRRADIATION DIRECTION

FIRST COMPLEX AMPLITUDE IMAGE GENERATION ~S62

COMPLEX AMPLITUDE IMAGE FOR
EACH LIGHT IRRADIATION DIRECTION

SECOND COMPLEX AMPLITUDE IMAGE GENERATION ~S63

COMPLEX AMPLITUDE IMAGE FOR
EACH POSITION AND EACH
LIGHT IRRADIATION DIRECTION

PHASE CONJUGATE OPERATION ~S64

COMPLEX AMPLITUDE IMAGE FOR
EACH POSITION AND EACH
LIGHT IRRADIATION DIRECTION

TWO-DIMENSIONAL PHASE IMAGE GENERATION ~S65

TWO-DIMENSIONAL PHASE IMAGE
FOR EACH POSITION

THREE-DIMENSIONAL PHASE IMAGE GENERATION ~S66

THREE -DIMENSIONAL PHASE IMAGE

REFRACTIVE INDEX DISTRIBUTION CALCULATION ~S67

THREE -DIMENSIONAL REFRACTIVE
INDEX DISTRIBUTION

51

# Fig.17

SECOND COMPLEX AMPLITUDE
IMAGE GENERATION STEP S63

TWO-DIMENSIONAL PHASE
IMAGE GENERATION STEP S65

$z = z_1$

$z = z_2$

$z = z_3$

COMPLEX
AMPLITUDE IMAGE

COMPLEX
AMPLITUDE IMAGE

COMPLEX DIFFERENTIAL
INTERFERENCE IMAGE

EP 4 538 385 A1

# Fig.18

SECOND COMPLEX AMPLITUDE IMAGE GENERATION STEP S63

PHASE CONJUGATE OPERATION STEP S64

TWO-DIMENSIONAL PHASE IMAGE GENERATION STEP S65

$z = z_0$

COMPLEX AMPLITUDE IMAGE

$z = z_1$

$z = z_2$

$z = z_3$

COMPLEX AMPLITUDE IMAGE

$z = z_1$

$z = z_2$

$z = z_3$

COMPLEX AMPLITUDE IMAGE

COMPLEX DIFFERENTIAL INTERFERENCE IMAGE

EP 4 538 385 A1

# Fig.19

SECOND COMPLEX AMPLITUDE
IMAGE GENERATION STEP S63

TWO-DIMENSIONAL PHASE
IMAGE GENERATION STEP S65

PHASE
CONJUGATE
OPERATION
STEP S64

$z = z_0$

COMPLEX
AMPLITUDE IMAGE

$z = z_0$

COMPLEX
AMPLITUDE IMAGE

$z = z_1$

$z = z_2$

$z = z_3$

COMPLEX
AMPLITUDE IMAGE

COMPLEX DIFFERENTIAL
INTERFERENCE IMAGE

EP 4 538 385 A1

Fig.20

# Fig.21

PHASE IMAGE GENERATED FROM
COMPLEX AMPLITUDE IMAGE AFTER
PHASE CONJUGATE OPERATION
IN TWO-DIMENSIONAL PHASE
IMAGE GENERATION STEP S65

THREE-DIMENSIONAL
PHASE IMAGE
GENERATION STEP S66

PHASE IMAGE GENERATED FROM
COMPLEX AMPLITUDE IMAGE BEFORE
PHASE CONJUGATE OPERATION
IN TWO-DIMENSIONAL PHASE
IMAGE GENERATION STEP S65

THREE -DIMENSIONAL
PHASE IMAGE

REFRACTIVE INDEX
DISTRIBUTION
CALCULATION
STEP S67

THREE -DIMENSIONAL
REFRACTIVE INDEX
DISTRIBUTION

# Fig.22

LIGHT
IRRADIATION $\quad U_{in}(k_{in})$

OBSERVATION OBJECT

$T(r_{out}, k_{in})$

$u_{out}(r_{out})$

IMAGING

IMAGING UNIT

# Fig.23

IMAGING UNIT

IMAGING

$u_{in} (r_{in})$

OBSERVATION OBJECT

$S (r_{in}, k_{out})$

LIGHT
IRRADIATION

$U_{out} (k_{out})$

*Fig.24*

COMPLEX AMPLITUDE IMAGE → IMAGE DIVIDING → DIVIDED PARTIAL IMAGE → PHASE CONJUGATE OPERATION → DIVIDED PARTIAL IMAGE → IMAGE COMBINING → COMPLEX AMPLITUDE IMAGE AFTER COMBINING

Fig.25

ANALYSIS UNIT — 70

INTERFERENCE INTENSITY IMAGE ACQUISITION UNIT — 71

FIRST COMPLEX AMPLITUDE IMAGE GENERATION UNIT — 72

SECOND COMPLEX AMPLITUDE IMAGE GENERATION UNIT — 73

TWO-DIMENSIONAL PHASE IMAGE GENERATION UNIT — 74

THREE-DIMENSIONAL PHASE IMAGE GENERATION UNIT — 75

REFRACTIVE INDEX DISTRIBUTION CALCULATION UNIT — 76

THIRD COMPLEX AMPLITUDE IMAGE GENERATION UNIT — 77

DISPLAY UNIT — 78

STORAGE UNIT — 79

RECORDING MEDIUM — 2

1G

Fig.26

ANALYSIS UNIT — 70

INTERFERENCE INTENSITY IMAGE ACQUISITION UNIT — 71

FIRST COMPLEX AMPLITUDE IMAGE GENERATION UNIT — 72

SECOND COMPLEX AMPLITUDE IMAGE GENERATION UNIT — 73

TWO-DIMENSIONAL PHASE IMAGE GENERATION UNIT — 74

THREE-DIMENSIONAL PHASE IMAGE GENERATION UNIT — 75

REFRACTIVE INDEX DISTRIBUTION CALCULATION UNIT — 76

THIRD COMPLEX AMPLITUDE IMAGE GENERATION UNIT — 77

DISPLAY UNIT — 78

STORAGE UNIT — 79

RECORDING MEDIUM — 2

EP 4 538 385 A1

**Fig.27**

EP 4 538 385 A1

# Fig.28

```
┌─────────────────────────────────────────────────┐
│  INTERFERENCE INTENSITY IMAGE ACQUISITION        │──S71
└─────────────────────────────────────────────────┘

      ┌─────────────────────────────────────┐
      │  INTERFERENCE INTENSITY IMAGE FOR   │
      │  EACH LIGHT IRRADIATION DIRECTION   │
      └─────────────────────────────────────┘

┌─────────────────────────────────────────────────┐
│  FIRST COMPLEX AMPLITUDE IMAGE GENERATION        │──S72
└─────────────────────────────────────────────────┘

      ┌─────────────────────────────────────┐
      │  COMPLEX AMPLITUDE IMAGE FOR        │
      │  EACH LIGHT IRRADIATION DIRECTION   │
      └─────────────────────────────────────┘

┌─────────────────────────────────────────────────┐
│                    j = 0                         │──S81
└─────────────────────────────────────────────────┘

┌─────────────────────────────────────────────────┐
│                  j ← j + 1                       │──S82
└─────────────────────────────────────────────────┘
                                                    ──S83
  ┌───────────────────────────────────────────────┐
  │  ┌─────────────────────────────────────────┐  │
  │  │  SECOND COMPLEX AMPLITUDE IMAGE          │  │──S73
  │  │  GENERATION                             │  │
  │  └─────────────────────────────────────────┘  │
  │  ┌─────────────────────────────────────────┐  │
  │  │  TWO-DIMENSIONAL PHASE IMAGE GENERATION  │  │──S74
  │  └─────────────────────────────────────────┘  │
  │  ┌─────────────────────────────────────────┐  │
  │  │  THREE-DIMENSIONAL PHASE IMAGE GENERATION│  │──S75
  │  └─────────────────────────────────────────┘  │
  │  ┌─────────────────────────────────────────┐  │
  │  │  REFRACTIVE INDEX DISTRIBUTION CALCULATION│  │──S76
  │  └─────────────────────────────────────────┘  │
  └───────────────────────────────────────────────┘

      ┌─────────────────────────────────────┐
      │  THREE -DIMENSIONAL REFRACTIVE       │
      │  INDEX DISTRIBUTION                  │
      └─────────────────────────────────────┘
                                          ──S84
            ◇ j < J ◇ ───false──► END
              true

┌─────────────────────────────────────────────────┐
│  THIRD COMPLEX AMPLITUDE IMAGE GENERATION        │──S77
└─────────────────────────────────────────────────┘

      ┌─────────────────────────────────────┐
      │  COMPLEX AMPLITUDE IMAGE FOR        │
      │  EACH LIGHT IRRADIATION DIRECTION   │
      └─────────────────────────────────────┘
```

# Fig.29

```
┌─────────────────────────────────────────┐
│  COMPLEX AMPLITUDE IMAGE FOR             │
│  EACH LIGHT IRRADIATION DIRECTION        │
└─────────────────────────────────────────┘
                    │
┌───────────────────────────────────────────────────────┐          ─S83
│  ┌─────────────────────────────────────────────────┐   │
│  │ SECOND COMPLEX AMPLITUDE IMAGE GENERATION       │   │  ─S73
│  └─────────────────────────────────────────────────┘   │
│              │                                          │
│  ┌─────────────────────────────────────┐               │
│  │ COMPLEX AMPLITUDE IMAGE FOR          │               │
│  │ EACH POSITION AND EACH               │               │
│  │ LIGHT IRRADIATION DIRECTION          │               │
│  └─────────────────────────────────────┘               │
│              │                                          │
│  ┌─────────────────────────────────────────────────┐   │
│  │ TWO-DIMENSIONAL PHASE IMAGE GENERATION          │   │  ─S74
│  └─────────────────────────────────────────────────┘   │
│              │                                          │
│  ┌─────────────────────────────────────┐               │
│  │ TWO-DIMENSIONAL PHASE                │               │
│  │ IMAGE FOR EACH POSITION              │               │
│  └─────────────────────────────────────┘               │
│              │                                          │
│  ┌─────────────────────────────────────────────────┐   │
│  │ THREE-DIMENSIONAL PHASE IMAGE GENERATION        │   │  ─S75
│  └─────────────────────────────────────────────────┘   │
│              │                                          │
│  ┌─────────────────────────────────────┐               │
│  │ THREE-DIMENSIONAL PHASE IMAGE        │               │
│  └─────────────────────────────────────┘               │
│              │                                          │
│  ┌─────────────────────────────────────────────────┐   │
│  │ REFRACTIVE INDEX DISTRIBUTION CALCULATION       │   │  ─S76
│  └─────────────────────────────────────────────────┘   │
└───────────────────────────────────────────────────────┘
                    │
┌─────────────────────────────────────────┐
│  THREE -DIMENSIONAL                      │
│  REFRACTIVE INDEX DISTRIBUTION           │
└─────────────────────────────────────────┘
```

Fig.30

EP 4 538 385 A1

THIRD BLOCK

$z = z_3$
$z = z_2$

SECOND BLOCK

$z = z_2$
$z = z_1$

FIRST BLOCK

$z = z_1$
$z = z_0$

OBSERVATION
OBJECT

$z = z_3$
$z = z_2$
$z = z_1$
$z = z_0$

# Fig.31

COMPLEX AMPLITUDE IMAGE IN THIRD BLOCK

THIRD COMPLEX AMPLITUDE IMAGE GENERATION STEP S77

COMPLEX AMPLITUDE IMAGE IN SECOND BLOCK

THIRD COMPLEX AMPLITUDE IMAGE GENERATION STEP S77

COMPLEX AMPLITUDE IMAGE IN FIRST BLOCK

STEP S83

$z = z_3$
$z = z_2$
REFRACTIVE INDEX DISTRIBUTION OF THIRD BLOCK

STEP S83

$z = z_2$
$z = z_1$
REFRACTIVE INDEX DISTRIBUTION OF SECOND BLOCK

STEP S83

$z = z_1$
$z = z_0$
z
y
x
REFRACTIVE INDEX DISTRIBUTION OF FIRST BLOCK

$z = z_3$
$z = z_2$
$z = z_1$
$z = z_0$
z
y
x
REFRACTIVE INDEX DISTRIBUTION OF OBSERVATION OBJECT

EP 4 538 385 A1

# Fig.32

$z = z_{j-1} + 7\Delta z = z_j$

$z = z_{j-1} + 6\Delta z$

$z = z_{j-1} + 5\Delta z$

$z = z_{j-1} + 4\Delta z$

$z = z_{j-1} + 3\Delta z$

$z = z_{j-1} + 2\Delta z$

$z = z_{j-1} + \Delta z$

$z = z_{j-1}$

j-TH BLOCK

EP 4 538 385 A1

# Fig.33

```
┌─────────────────────────────┐
│   COMPLEX AMPLITUDE IMAGE    │
│   AT FIRST POSITION z_{j-1}  │
└─────────────────────────────┘
              │
┌─────────────────────────────┐
│         z ← z_{j-1}          │──── S41
└─────────────────────────────┘
              │
              ▼
┌─────────────────────────────┐
│ INTERACTION BETWEEN THE COMPLEX │
│ AMPLITUDE AT POSITION z AND REFRACTIVE │──── S42
│ INDEX DISTRIBUTION AT POSITION z │
└─────────────────────────────┘
              │
┌─────────────────────────────┐
│   PROPAGATION OF DISTANCE Δz │──── S43
└─────────────────────────────┘
              │
┌─────────────────────────────┐
│   COMPLEX AMPLITUDE IMAGE    │
│   AT POSITION z+Δz           │
└─────────────────────────────┘
              │
┌─────────────────────────────┐
│         z ← z+Δz             │──── S44
└─────────────────────────────┘
              │
    true      ▼            S45
◁────────◇  z < z_j  ◇
              │
            false
              ▼
┌─────────────────────────────┐
│   COMPLEX AMPLITUDE IMAGE    │
│   AT SECOND POSITION z_j     │
└─────────────────────────────┘
```

# Fig.34

EP 4 538 385 A1

# Fig.35

| 1. ACQUIRE REFRACTIVE INDEX DISTRIBUTION DATA OF OBSERVATION OBJECT |

↓

| 2. DETERMINE JUDGMENT REGION FROM REFRACTIVE INDEX DISTRIBUTION DATA OF OBSERVATION OBJECT |

↓

| 3. ARE THERE FEATURES OF HAVING REGION EQUIVALENT TO NUCLEUS IN JUDGMENT REGION? | → No | OTHERS |

↓ Yes

| 4. SET THRESHOLD VALUE FOR AVERAGE VALUE OR MEDIAN VALUE OF REFRACTIVE INDEX |

↓

| 5. COMPARE THRESHOLD VALUE WITH REFRACTIVE INDEX DISTRIBUTION DATA OF JUDGMENT REGION |

↓

| 6. IS AVERAGE VALUE OR MEDIAN VALUE OF REFRACTIVE INDEX LOWER THAN THRESHOLD VALUE? |

↓ Yes          ↓ No

| 7. JUDGE TO BE REGION OF DEAD CELLS | | 7. JUDGE TO BE REGION OF LIVE CELLS |

# Fig.36

| 1. ACQUIRE REFRACTIVE INDEX DISTRIBUTION DATA OF OBSERVATION OBJECT |
|---|

↓

| 2. DETERMINE REGION OF CELLULAR NUCLEUS TO BE JUDGED FROM REFRACTIVE NDEX DISTRIBUTION DATA OF OBSERVATION OBJECT |
|---|

↓

| 3. SET THRESHOLD VALUE FOR STATISTICAL DISPERSION OF REFRACTIVE INDEX |
|---|

↓

| 4. COMPARE THRESHOLD VALUE WITH REFRACTIVE INDEX DISTRIBUTION DATA OF JUDGMENT REGION |
|---|

↓

| 5. IS STATISTICAL DISPERSION OF REFRACTIVE INDEX GREATER THAN THRESHOLD VALUE? |
|---|

↓ Yes          ↓ No

| 6. JUDGE TO BE REGION OF DEAD CELLS | 6. JUDGE TO BE REGION OF LIVE CELLS |
|---|---|

*Fig.37*

| 1. ACQUIRE REFRACTIVE INDEX DISTRIBUTION DATA AND FLUORESCENCE DATA OF REFERENCE OBSERVATION OBJECT |
|---|

↓

| 2. EXTRACT FEATURE QUANTITY (INCLUDING FEATURE QUANTITY CORRESPONDING TO FEATURES OF HAVING REGION EQUIVALENT TO NUCLEUS) FROM REFRACTIVE INDEX DISTRIBUTION DATA |
|---|

↓

| 3. EXTRACT REGIONS OF LIVE AND DEAD CELLS FROM FLUORESCENCE DATA |
|---|

↓

| 4. LEARN SO AS TO INFER REGIONS OF LIVE AND DEAD CELLS FROM FEATURE QUANTITY BY MACHINE LEARNING |
|---|

## Fig.38

1. ACQUIRE REFRACTIVE INDEX DISTRIBUTION DATA OF OBSERVATION OBJECT

2. EXTRACT FEATURE QUANTITY (INCLUDING FEATURE QUANTITY CORRESPONDING TO FEATURES OF HAVING REGION EQUIVALENT TO NUCLEUS) FROM REFRACTIVE INDEX DISTRIBUTION DATA

3. INFER USING LEARNING MODEL

4. EVALUATE TO BE REGION OF DEAD CELLS

4. EVALUATE TO BE REGION OF LIVE CELLS

# Fig.39

CELL CLUSTER A

CROSS-SECTION

LIVE CELL

CELL DEATH

CELL CLUSTER B

EP 4 538 385 A1

## Fig.40

(1)TWO-DIMENSIONAL CULTURE (2)FLOATING (3)COUNTING OF NUMBER OF CELLS (4)SEEDING (5)THREE-DIMENSIONAL CULTURE (6)SCREENING (7)EVALUATION

# Fig.41

CELL CLUSTER A

CELL CLUSTER B

LIVE CELL

CROSS-
SECTION

CELL DEATH

EP 4 538 385 A1

Fig.42

Fig.43

Fig.44

Fig.45

Fig.46

# Fig.47

1. ACQUIRE REFRACTIVE INDEX DISTRIBUTION DATA OF CELL CLUSTER

↓

2. DETERMINE JUDGMENT REGION FROM REFRACTIVE INDEX DISTRIBUTION DATA
OF CELL CLUSTER

↓

3. ARE THERE FEATURES OF HAVING REGION EQUIVALENT
TO NUCLEUS IN JUDGMENT REGION? ──No──▶ OTHERS

↓ Yes

4. SET THRESHOLD VALUE FOR AVERAGE VALUE OF REFRACTIVE INDEX
AVERAGE REFRACTIVE INDEX 1.350 OF WHOLE CELL WAS SET AS
THRESHOLD VALUE

↓

5. COMPARE THRESHOLD VALUE WITH REFRACTIVE INDEX DISTRIBUTION
DATA OF JUDGMENT REGION

↓

6. IS AVERAGE VALUE OF REFRACTIVE INDEX LOWER THAN THRESHOLD VALUE?

↓ Yes          ↓ No

7. JUDGE TO BE REGION          7. JUDGE TO BE REGION
OF DEAD CELLS          OF LIVE CELLS

# Fig.48

1. ACQUIRE REFRACTIVE INDEX DISTRIBUTION DATA OF CELL CLUSTER

2. DETERMINE REGION OF CELLULAR NUCLEUS TO BE JUDGED FROM
REFRACTIVE INDEX DISTRIBUTION DATA OF CELL CLUSTER

3. SET THRESHOLD VALUE FOR STATISTICAL DISPERSION OF REFRACTIVE INDEX
STANDARD DEVIATION 0.006 OF REFRACTIVE INDEX INSIDE NUCLEUS
WAS SET AS THRESHOLD VALUE

4. COMPARE THRESHOLD VALUE WITH REFRACTIVE INDEX DISTRIBUTION
DATA OF JUDGMENT REGION

5. IS STANDARD DEVIATION OF REFRACTIVE INDEX HIGHER THAN THRESHOLD VALUE?

↓ Yes ↓ No

| 6. JUDGE TO BE REGION OF DEAD CELLS | 6. JUDGE TO BE REGION OF LIVE CELLS |

## INTERNATIONAL SEARCH REPORT

| International application No. |
| --- |
| **PCT/JP2023/011554** |

### A. CLASSIFICATION OF SUBJECT MATTER

*C12Q 1/04*(2006.01)i; *G01N 21/45*(2006.01)i; *C12M 1/34*(2006.01)i
FI: C12Q1/04; C12M1/34 B; G01N21/45 A

According to International Patent Classification (IPC) or to both national classification and IPC

### B. FIELDS SEARCHED

Minimum documentation searched (classification system followed by classification symbols)

C12Q1/04; G01N21/45; C12M1/34

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | GORBENKO, D. A. et al. Quantification of changes in cellular morphology during cell necrosis obtained from 3D refractive index distributions. Journal of physics. 2019, pp. 1-5, doi:10.1088/1742-6596/1236/1/012015<br>entire text, in particular, pp. 2-4 | 1-6, 8-10 |
| Y | entire text, in particular, pp. 2-4 | 1-12 |
| Y | JP 6792616 B2 (HITACHI HIGH-TECHNOLOGIES CORPORATION) 10 November 2020 (2020-11-10)<br>paragraphs [0025]-[0027], examples | 1-12 |

☐ Further documents are listed in the continuation of Box C.    ☑ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **01 June 2023** | **13 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

| | International application No. |
|---|---|
| | **PCT/JP2023/011554** |

| Patent document cited in search report | Publication date (day/month/year) | Patent family member(s) | Publication date (day/month/year) |
|---|---|---|---|
| JP 6792616 B2 | 10 November 2020 | US 2019/0244349 A1 paragraphs [0054]-[0059], examples | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6792616 B

- JP 6122817 B

**Non-patent literature cited in the description**

- **SUNGSAM KANG et al.** Imaging deep within a scattering medium using collective accumulation of single-scattered waves. *NATURE PHOTONICS*, 2015, vol. 9, 253-258

- **SHIN MURAI**. *NATURE COMMUNICATIONS*, 2018, vol. 9, 4457